# EUROPEAN PATENT APPLICATION

(11) **EP 4 450 082 A1**
(43) Date of publication of application: **23.10.2024**
(21) Application number: 24170725.6
(22) Date of filing: 17.04.2024
(51) Int. Cl.: A61K 39/00, A61K 39/09

(54) **METHODS FOR IMPROVING THE ADSORPTION OF POLYSACCHARIDE-PROTEIN CONJUGATES AND MULTIVALENT VACCINE FORMULATION OBTAINED THEREOF**

(30) Priority: 17.04.2023 IN 202323028039
(71) Applicant: Serum Institute Of India Private Limited, Pune, Maharashtra 411 028 (IN)
(72) Inventor: POONAWALLA, Cyrus Soli, 411 028 Pune (IN); DHERE, Rajeev Mhalasakant, 411 028 Pune (IN); JANA, Swapan Kumar, 411 028 Pune (IN); JAIN, Shital Shantilal, 411 028 Pune (IN); MAHAJAN, Amol Dattatraya, 411 028 Pune (IN); PAUL, Gourab Shankar, 411 028 Pune (IN); MALVIYA, Hitesh Kumar, 411 028 Pune (IN); JOSHI, Chetan Vilas, 411 028 Pune (IN); GAUTAM, Manish Mahesh, 411 028 Pune (IN); KALE, Prathamesh Prakash, 411 028 Pune (IN); GAIROLA, Sunil Jagdish Prasad, 411 028 Pune (IN); MALLYA, Asha Dinesh, 411 028 Pune (IN); SONI, Dipen Jagdishbhai, 411 028 Pune (IN); PATNI, Sushil Vardhaman, 411 028 Pune (IN); GAVADE, Vinay Vijay, 411 028 Pune (IN)
(74) Representative: Studio Torta S.p.A.

(57) **Abstract**

The present disclosure relates to vaccine compositions comprising pneumococcal polysaccharide-carrier protein conjugates. The present disclosure particularly relates to improved, stable, immunogenic multivalent *Streptococcus pneumoniae* polysaccharide-protein conjugate vaccine compositions having at least three distinct carrier proteins, preparing the vaccine compositions and methods for prevention and/ or treatment of subjects with *Streptococcus pneumoniae.* These multivalent pneumococcal compositions will overcome carrier induced epitopic suppression, provide enhanced immune response for new serotypes (as compared to existing approved vaccines) and also help address the emergence of non-vaccine serotypes.

## Description

### FIELD

The present disclosure relates to methods for manufacturing the vaccine compositions and vaccine compositions obtained therefrom. Particularly, the present disclosure is related to methods for preparing the vaccine compositions, improved formulations, novel stable and immunogenic multivalent *Streptococcus pneumoniae* polysaccharide-protein conjugate vaccine compositions, and methods for prevention and/ or treatment of subjects with *Streptococcus pneumoniae.*

### BACKGROUND

All publications herein are incorporated by reference to the same extent as if each individual publication or patent application was specifically and individually indicated to be incorporated by reference. The following description includes information that may be useful in understanding the present invention. It is not an admission that any of the information provided herein is prior art or relevant to the presently claimed invention, or that any publication specifically or implicitly referenced is prior art.

*Streptococcus pneumoniae* is a major cause of pneumonia, meningitis, and pleural effusion in children and adults. There are more than 100 distinct serotypes of *Streptococcus pneumoniae* identified to date. *Streptococcus pneumoniae* is responsible for > 1 million deaths worldwide in children <5 years of age each year and is a leading cause of both invasive pneumococcal diseases (IPD) (e.g., bacteremia, bacteremic pneumonia, and meningitis) and non-invasive diseases (e.g., non-bacteremic pneumonia, otitis media, and sinusitis). Collectively, these conditions account for greater global morbidity and mortality than diseases caused by any other pathogen. Children below 5 years of age, older adults and adults with co-morbid conditions (such as HIV infection and other conditions associated with immune deficiency) tend to be more affected by pneumococcal disease. It is estimated that around 45 million episodes of lower respiratory infections and >300000 deaths occur annually in children below 5 years of age due to invasive pneumococcal disease (IPD).

Two classes of pneumococcal vaccines are currently available - one based on polysaccharides and the other based on polysaccharides conjugated to a carrier protein.

Pneumovax^{®} 23 (Merck Sharp & Dohme Corp.) is a polysaccharide-based vaccine containing purified capsular polysaccharides from the 23 serotypes (1, 2, 3, 4, 5, 6B, 7F, 8, 9N, 9V, 10A, 11A, 12F, 14, 15B, 17F, 18C, 19F, 19A, 20, 22F, 23F, and 33F) causing about 90% of invasive pneumococcal infection in industrialized countries. However, pneumococcal polysaccharides are T-cell independent antigens and thus are poorly immunogenic in those aged <2 years and fail to induce immune memory. Hence, infants and young children respond poorly to unconjugated pneumococcal polysaccharides.

Roughly 50% of the world's annual pneumococcal child deaths occur in 4 African and Asian countries: India (-68,700 deaths); Nigeria (-49,000); Democratic Republic of Congo (-14,500); and Pakistan (-14,400).

To address the problem of decreased immunogenicity associated with the pneumococcal polysaccharide vaccine in infants and children, improved polysaccharide-protein conjugate vaccines are required to be developed in which the epidemiologically most important pneumococcal serotypes are coupled to various protein carriers. This approach involves covalent linking of the polysaccharide to a protein to enhance immunogenicity and increase serum antibody levels. These protein carriers are T-cell-dependent antigens that stimulate a T-helper cell response that primes the vaccinated individual for an anamnestic or booster response. The coupling of a polysaccharide antigen to a protein carrier converts the polysaccharide from a T-cell-independent to a T-cell-dependent antigen to which young children can respond immunologically.

Conjugated vaccines consisting of polysaccharides conjugated to an immunogenic protein, elicits a T-cell dependent response with establishment of B-cell memory and long-term immunization. The advantages of the PCV include its activity in young children and its potential long-lasting activity. WHO prequalified pneumococcal conjugate vaccines (PCV) against *Streptococcus pneumoniae* include Prevnar^{®} (Pfizer), containing the 7 most frequently isolated serotypes (4, 6B, 9V, 14, 18C, 19F and 23F) causing invasive pneumococcal disease in young children and infants at the time, was first licensed in the United States in February 2000. Subsequently, Prevnar13^{®} (Pfizer) was approved, containing conjugates of polysaccharides from serotypes 6A, 6B, 19A, 19F in addition to 1, 3, 4, 5, 7F, 9V, 14, 18C and 23F. Synflorix^{®} (GSK) is another approved conjugate vaccine that provides protection against 1, 4, 5, 6B, 7F, 9V, 14, 18C, 19F, and 23F as well as cross protection against 19A and 6A. PNEUMOSIL^{®} (Serum Institute of India Pvt Ltd (SIIPL)), WHO pre-qualified 10 valent vaccine confers protection against pneumococcal serotypes 1, 5, 6A, 6B, 7F, 9V, 14, 19A, 19F and 23F (protects against the 10 most relevant serotypes most likely to cause serious disease in the world's highest-burden regions-Africa and Asia including many LATAM countries).

Use of pneumococcal conjugate vaccine (PCV) in pediatric National Immunizations Programs (NIPs) around the world has significantly reduced the mortality and morbidity associated with pneumococcal disease. Serum Institute' PNEUMOSIL^{®} has been introduced in at least 60 lower-income countries, and more than 225 million children have been vaccinated, and over 700,000 deaths have been prevented. Also, it is estimated that 175 million cases of pneumococcal disease and 625,000 deaths have been prevented by PCV13 in children under 5 years of age worldwide between 2010 and 2019. In addition to conferring protection to young children against pneumococcal disease, routine pediatric immunization has also provided indirect protection to non-vaccinated populations through a reduction in nasopharyngeal carriage of vaccine serotypes, thereby reducing transmission of disease. As more countries increase PCV uptake, PCV vaccination offers the potential to prevent around 54.6 million episodes and 399,000 deaths annually in children under 5 years of age due to pneumococcal disease globally.

However, one of the concerns with routine use of existing pneumococcal conjugate vaccines is serotype replacement. World Health Organization (WHO) has reported the emergence of non-vaccine serotype circulation and serotype replacement, leading to an increased proportion of pneumococcal disease of these serotypes in both children and adults (WHO Position Paper-February 2019. Wkly. Epidemiol. Rec. 2019; 94:85-104 & Weinbergera M.D et al; Lancet. 2012; 378:1962-1973). Each vaccine targets only some of all the pneumococcal serotypes, therefore the prevalence of non-vaccine serotypes (NVTs) are expected to increase leading to an increase in NVTs carriage and related clinical syndromes. Surveillance studies have demonstrated an increase in carriage of NVTs following the introduction of PCV-7 as well as an increase in NVT pneumococcal disease. Further, it is worth considering the biases in the disease surveillance systems that could underestimate the true amount of replacement.

The introduction of Prevnar13^{®} in 2010 in the immunization program was an effective measure in preventing IPDs in children. The disease burden of *S*. *pneumoniae* has remarkably declined since the application Prevnar13^{®}. Prevnar13^{®} has reduced the overall incidence of IPDs by more than 50%. However, there has been an apparent change in the serotype distribution of *S*. *pneumoniae* and the increased prevalence of NVTs may offset the benefit of the decreased prevalence of vaccine serotypes (VTs) after the introduction of PCV13. Therefore, higher-valent vaccines are needed that can adequately protect against maximum pneumococcal serotypes including serotypes 2, 8, 10A, 11A, 12F, 15B, 22F, 24F and 33F.

Further, individual serotype prevalence can change over time in different geographic regions or age groups. The increased prevalence and pathogenicity of NVTs pose new challenges to reduction of the burden of pneumococcal disease, which was a benefit from widespread PCV vaccination. Among NVTs, serotype 24F is considered one of the most prevalent causative agents of IPD in Europe and the Western Pacific Region. (Middle East and North Africa) Furthermore, serotype 24F and other NVT such as 8, 12F, and 33F were considered at the upper end of the invasiveness spectrum among children immunized with PCV. Therefore, geographic variations in serotype distribution and the evolving pneumococcal serotype prevalence should be considered while developing higher valent pneumococcal vaccines.

Recently two more multivalent pneumococcal vaccines have been approved for use in USA. Vaxneuvance^{®} (Merck Sharp & Dohme Corp) provides protection against 15 *Streptococcus pneumoniae* serotypes 1, 3, 4, 5, 6A, 6B, 7F, 9V, 14, 18C, 19A, 19F, 22F, 23F and 33F. Prevnar20^{®} (Pfizer) another approved conjugate vaccine provides protection against *Streptococcus pneumoniae* serotypes 1, 3, 4, 5, 6A, 6B, 7F, 8, 9V, 10A, 11A, 12F, 14, 15B, 18C, 19A, 19F, 22F, 23F, and 33F.

Pfizer 13 valent, Pfizer 20 valent, Merck's 15, Pfizer 7 valent are all vaccines consisting of single type of carrier protein i.e. CRM197. One of the most important factors that may influence the immunogenicity of conjugate vaccines is the selection of a carrier protein that avoids carrier-mediated suppression of the antibody response. Having a single type of carrier protein results in carrier induced epitopic suppression wherein for individual serotype, the desired anti-polysaccharide antibody response may not be achieved. Compounds such as polysaccharides and peptides are poorly immunogenic and cannot induce an immune response by themselves, they usually need to be covalently linked to a carrier, which usually are large proteins that are highly immunogenic. These carrier proteins bind to antigen presenting cell (APC) and provide T-cell epitopes via MHC Class II for presentation to T-helper cells and increases the immune response. Proteins such as tetanus toxoid (TT), diphtheria toxoid (DT), non-toxic mutant of diphtheria toxin (CRM197) are used as effective carriers to enhance immunogenicity. However, these carrier proteins induce an antibody response that competes with the anti-peptide/polysaccharide one; a problem that may be aggravated if the subject have been exposed to that carrier before. This situation becomes more aggravated for vaccines that use the same type of carrier protein in previous immunization and when the same carrier protein is used again in another vaccine will result in suppression of the immune response. Hence, the choice of carrier protein(s) is an important criterion during the manufacturing of vaccines. Therefore, alternative multivalent conjugate vaccines having multiple types of carrier proteins addressing the issue of carrier induced epitopic suppression are required.

Further aforementioned, Pfizer/ GSK/ Merck vaccines are as yet available only in the developed world and there still exists an urgent need for affordable pneumococcal vaccines that are available globally to low- and middle-income countries who are most vulnerable to the pneumococcal diseases and are capable of conferring protection against the most prevalent, emerging and pathogenic pneumococcal serotypes.

As mentioned above, for a polysaccharide to induce sufficient antibodies to be protective in an infant, the polysaccharide as an immunogen must elicit the help of T cells. Hence, the polysaccharides need to be covalently attached to a carrier protein to render them sufficiently immunogenic in young children. A mere mixture of polysaccharide and carrier protein does not render the polysaccharide more immunogenic in infants. Thus, a conjugate vaccine is generally composed of a bacterial polysaccharide, size reduced polysaccharide, or oligosaccharides derived there from, that is covalently attached to a T cell epitope containing polypeptide, generally a carrier protein. The immune response to a conjugate resembles closely that to a protein. This results in development of strong IgG response to a conjugated polysaccharide and generates immunologic memory in young infants.

Polysaccharides can also be fragmented/ sized before conjugation step to improve the conjugation efficiency/conjugate yield, increase the number of reactive groups resulting in enhanced immunogenicity of the conjugates. Fragmentation/ sizing of the polysaccharides may be carried out by different methods, such as thermal hydrolysis, chemical hydrolysis (acidic, alkaline, ozonolytic, and free radical), enzymatic sizing (lyases and hydrolases), sonication and homogenization, leading to a random size reduction often resulting in an increase in sample polydispersity, which can be subsequently sized (by chromatographic or ultrafiltration steps), to produce polysaccharides of more defined length for carrier protein conjugation. Size reduced polysaccharides are generally coupled to the carrier protein by using the end reducing sugar. Such selective approaches avoid chemical modification along the saccharide chain and produce better-defined structures, which are easier to manufacture with consistency. Enzymatic sizing, though simple is limited by the commercial availability of enzymes, their cost and their sensitivity to denaturation. Chemical hydrolysis (acidic and alkaline treatments or free radical mechanisms) results in the generation of large amounts of monosaccharides and by-products, which is not desirable during the large-scale production of vaccine.

Typically, the purified polysaccharide is chemically activated/ modified to generate reactive groups that can couple/ link to the carrier protein. An optimal degree of activation of the sugar chain needs to be identified to allow efficient conjugation, without altering saccharide structural integrity. Two of the most commonly used methods for polysaccharide activation are periodate oxidation and cyanylation. In periodate oxidation, terminal hydroxyl groups present on the polysaccharide are oxidized to an aldehyde using an oxidizing agent (periodate, such as sodium periodate, potassium periodate or periodic acid). The vicinal hydroxyl groups of the polysaccharide are cleaved by the oxidizing agent, resulting in the formation of the reactive aldehyde groups. An important potential problem with use of periodate to activate the polysaccharide is the alteration of the physical structure of the polysaccharide, with loss of important epitopes. It is seen that even low levels of activation can result is loss of the specific epitope and creation of new epitopes. Particularly, the periodate oxidation step before reductive amination could open the saccharide ring structure, which may lead to ring-opened conjugates, depending on the serotype. Such ring-opened polysaccharides may reclose after the conjugation process, in some cases, causing the polysaccharides to form additional new conformations, resulting in new epitopes (Poolman J. et al Clin. Vaccine Immunol. 2011; 18:327-336).

Depending on the chemistry chosen, polysaccharide and/or protein carriers might be derivatized before conjugation. Level of chemical derivatization can drive conjugation stoichiometry, impact on B-cell and T-cell epitopes preservation and aggregate formation. Level of chemical derivatization influences polysaccharide to protein ratio, and ultimately immunogenicity. Therefore, the choice of conjugation strategy can affect the efficiency of conjugation, saccharide to protein ratio and glycoconjugate structure and size, with consequent impact on immunogenicity.

The conjugation method can influence the immunogenicity and the functionality of the antibodies induced. Reductive amination conjugation method has been used in most of the licensed pneumococcal conjugate vaccine (PCVs) (Pfizer 7 valent 13 valent 20 valent/ Merck 15 valent PCVs) which consists of sugar-protein conjugation through Schiff base formation between sugar-derived aldehydes and amines in proteins followed by a reduction. The first step is the introduction of aldehyde groups along the polysaccharide chain by partial oxidation with sodium periodate. Cis-diols in the sugar ring and sialic acid residues are the most sensitives zones to generate aldehydes by periodate oxidation. The carrier protein is then added to the reaction. Aldehydes from sugars are typically reacted with the lysine residues of the carrier protein. This methodology consists of the initial formation of bonds by Schiff bases between a carbonyl group in the carbohydrate and an amino group in the protein. The Schiff base is then specifically reduced with the weak reductant sodium cyanoborohydride to a more stable amine. Reductive amination also utilizes aprotic solvents (DMSO). However, reductive amination has important drawbacks, including a low yield, low coupling efficiency, and the risk of the partial degradation of the carbohydrate structure. Further, DMSO is not practical due to the significantly higher boiling point of DMSO which may be detrimental to the polysaccharide and the carrier protein. DMSO is an organic solvent which may result in the denaturization of carrier proteins. Also, one of the major drawbacks of DMSO is the difficulty of removal after a reaction; its high boiling point (189 °C) makes removal by rotavapor very difficult.

Cyanylation chemistry initially utilized CNBr to create the reactive cyanoester groups on the polysaccharides used in the first Hib conjugate reported in 1980 by Dr. John Robbins. Cyanylation with CNBr is a commonly used method for preparing protein-polysaccharide conjugate vaccines. However, the efficiency of conjugates prepared by CNBr was low and necessitated the use of reactive linkers such as adipic acid dihydrazide (ADH). The activation yields are only 0.5-2%, resulting in the use of large amounts of CNBr, with all the concomitant health risks. Although CNBr is a low-cost reagent, it is very toxic and its use requires special attention. In addition, sensitive polysaccharides can be damaged by the high pH conditions required for CNBr activation. Another disadvantage of these processes is that the activated polysaccharide contains both cyanate ester and imido carbonate structures in different proportions. The cyanate ester groups are hydrolyzed at a high pH, while the imido carbonates form inactive carbonates on hydrolysis at a more acidic pH. (Victor Morais et al; Bioengineering (Basel). 2022 Dec; 9(12): 774).

Alternatively, CDAP has been utilized for protein-polysaccharide conjugate vaccines (Synflorix, PNEUMOSIL^{®}), using it as a cyanylating agent for the activation of soluble polysaccharides. CDAP, which is easier to use compared to CNBr, has the advantage that it can activate polysaccharides at a lower pH than CNBr and with fewer side reactions. Unlike CNBr, CDAP-activated polysaccharides can be directly conjugated to proteins, resulting in a simpler process. Moreover, CDAP-activated polysaccharides can be functionalized with a diamine or a dihydrazide to make amino- or hydrazide-derivatized polysaccharides.

Vaccine formulations must generally be stable and be of uniform consistency to accommodate the need for a long shelf-life and the use of multiple dose containers. Vaccines based on proteins, including polysaccharide-protein conjugates, are subject to protein aggregation and precipitation which can result in an effective lower total concentration of the vaccine due to the unavailability of the precipitated protein product. Polysaccharide-protein conjugate vaccines, in particular, appear to have a stronger tendency to aggregate than the carrier protein alone (See Berti et al, 2004, Biophys J 86:3-9). During manufacturing of polysaccharide-protein conjugate, formulate can comprise of aggregates of polysaccharide-polysaccharide type, protein-protein type or polysaccharide-protein type. Such aggregations are also observed in the finished product leading to rejection of 4% to 10% of filled vials of polysaccharide-protein conjugate(s) vaccine, thereby affecting the stability and efficacy of the conjugate vaccine.

Further, the level of unconjugated or "free" saccharide on conjugation and during storage is considered a critical stability attribute for polysaccharide-carrier protein vaccines, and has a major impact on vaccine efficacy. Unconjugated/ free polysaccharide may occur because of incomplete purification of the conjugate or instability of the final product. Free polysaccharide, free carrier protein, percent o-acetylation of polysaccharides and molecular size of polysaccharides are important stability indicating parameters and has an impact on the vaccine quality and immunogenicity and should be optimized.

All the pneumococcal serotypes are not equally prevalent, so an effective pneumococcal conjugate vaccine needs to contain those serotypes most commonly associated with pneumococcal disease. The increase in antibiotic resistance among serotype replacement strains and the incidence of serotype replacement in both carriage and infection isolates after implementation of conjugate vaccines imparts the need for pneumococcal vaccines with broader coverage against pneumococci.

The number of vaccine serotypes is generally limited because each serotype must be individually conjugated to the carrier protein, and there is concern about limiting the total dose of carrier protein to control carrier-induced tolerance. This may increase immune response to the carrier protein which can cause a systemic overload, resulting in the lowering of the immune response to the specific serotypes. Other limitations may include the volume of vaccine dose that would be required for administration if more conjugated polysaccharides are included, which may also have an impact on the affordability of the vaccine. Hence, it is important during the development of multivalent vaccines to extend the immune stimulus for existing and additional serotypes, and to work on all factors involved in the conventional established conjugation methods. In addition to offering suitable protection against increasing number of serotypes, there is also a need to develop methods to reduce carrier protein antibodies in spite of an increase in the number of serotypes.

Applicant hereby emphasizes that preparing a stable and immunogenic polysaccharide-protein conjugate is a challenge. Multivalent conjugate vaccines are very difficult to make reproducibly and to characterize, requiring many release tests before they can be used. Applicant has found that the physicochemical characteristics of the polysaccharide antigen, method of polysaccharide fragmentation/sizing, polysaccharide post sizing recovery, polysaccharide length/molecular weight, polysaccharide ratio to carrier protein and the type of conjugation chemistry used, conjugation efficiency, optimized adsorption for each serotype (particular combination of serotypes to be adsorbed together), aggregation free formulation (type of excipients, type/ concentration of polysorbate, type/concentration of alum adjuvant), stability (conjugate weight, percentage of free polysaccharide, percentage of free protein) on storage, all have an impact on both structure and the immune response of each polysaccharide-carrier protein conjugate vaccine, likewise each polysaccharide-protein conjugate manufacturing process, stability are different and cannot be extrapolated from one conjugate to another . The sites at which the polysaccharide antigen is attached can also have an impact on the immunogenicity of the vaccine. Further, in a multivalent conjugate vaccine, the dynamic interactions of different conjugates in a single formulation may result in either masking or enhancement of immune response of few serotypes. The preservation of integrity of polysaccharide structure across purification, sizing, conjugation and formulation stages is also a pre-requisite to ensure that immunogenic epitopes are preserved.

Hence, there still exists an urgent unmet need for multivalent pneumococcal conjugate vaccines prepared by employing optimum manufacturing processes and formulations that are capable of protection against maximum number of pneumococcal serotypes, has reduced carrier protein induced immune suppression and are highly stable, show improved immunogenicity and is also affordable for access/distribution globally.

### OBJECTS

Some of the objects of the present disclosure, which at least one embodiment herein satisfies, are as follows:
An object of the present disclosure is to ameliorate one or more problems of the prior art or to at least provide a useful alternative.

Another object of the present disclosure is to provide a method for preparing a multivalent *Streptococcus pneumoniae* polysaccharide-protein conjugate vaccine composition.

Still another object of the present disclosure is to provide a method for preparing a multivalent *Streptococcus pneumoniae* polysaccharide-protein conjugate vaccine composition having improved adsorption.

Yet another object of the present disclosure is to provide a method for preparing a multivalent *Streptococcus pneumoniae* polysaccharide-protein conjugate vaccine composition (preferably 20 valent or 21 valent) having conjugates prepared by utilizing CDAP comprising three different carrier proteins (DT, TT, CRM197), wherein polysaccharides from serotypes 4 and 24F are individually conjugated to DT; polysaccharides from serotypes 3, 6A, 9V, 15B, 19A, 22F are individually conjugated to TT, and polysaccharides from serotypes 1, 2, 5, 6B, 7F, 8, 10A, 11A, 12F, 14, 18C, 19F, 23F, 33F are individually conjugated to CRM197.

Still another object of the present disclosure is to provide a method for preparing a multivalent *Streptococcus pneumoniae* polysaccharide-protein conjugate vaccine composition wherein all CRM197 and DT based conjugates are prepared by activating with 1-cyano-4-Dimethyl amino pyridinium tetrafluoroborate (CDAP) using direct coupling approaches utilizing cyanylation chemistry.

Another object of the present disclosure is to provide a method for preparing a multivalent *Streptococcus pneumoniae* polysaccharide-protein conjugate vaccine composition wherein all TT based conjugates are obtained by first preparing ADH derivatized TT (in presence of EDAC) followed by conjugating it with CDAP activated polysaccharides.

Still another object of the present disclosure is to provide a method for preparing a multivalent *Streptococcus pneumoniae* polysaccharide-protein conjugate vaccine composition having high conjugation efficiency.

Another object of the present disclosure is to provide a method for preparing a multivalent *Streptococcus pneumoniae* polysaccharide-protein conjugate vaccine composition having high polysaccharide recovery.

Still another object of the present disclosure is to provide a method for preparing a multivalent *Streptococcus pneumoniae* polysaccharide-protein conjugate vaccine composition wherein the structural integrity of the polysaccharides is maintained by utilizing high pressure homogenization (as against chemical sizing).

Yet another object of the present disclosure is to provide a multivalent *Streptococcus pneumoniae* polysaccharide-protein conjugate vaccine composition comprising pneumococcal polysaccharide-carrier protein conjugates.

Another object of the present disclosure is to provide a multivalent *Streptococcus pneumoniae* polysaccharide-protein conjugate vaccine composition comprising more than one carrier protein conjugated pneumococcal polysaccharides.

Still another object of the present disclosure is to provide a multivalent *Streptococcus pneumoniae* polysaccharide-protein conjugate vaccine composition eliciting protection against the most prevalent pneumococcal serotypes.

Yet object of the present disclosure is to provide a multivalent *Streptococcus pneumoniae* polysaccharide-protein conjugate vaccine composition having improved immunogenicity.

Still another object of the present disclosure is to provide a multivalent *Streptococcus pneumoniae* polysaccharide-protein conjugate vaccine composition eliciting cross reactivity for pneumococcal serotypes.

Yet another object of the present disclosure is to provide a multivalent *Streptococcus pneumoniae* polysaccharide-protein conjugate vaccine composition that is devoid of aggregates and has long term stability across a wide temperature range.

Another object of the present disclosure is to provide a multivalent *Streptococcus pneumoniae* polysaccharide-protein conjugate vaccine composition having increased alum gel quantity resulting in optimum adsorption and thereby providing increased B cell mediated IgG antibody response.

Still another object of the present disclosure is to provide a multivalent *Streptococcus pneumoniae* polysaccharide-protein conjugate vaccine composition having higher quantity of polysorbate 20 (Tween 20) resulting in aggregation-free composition and improved flow properties.

Another object of the present disclosure is to provide a multivalent *Streptococcus pneumoniae* polysaccharide-protein conjugate vaccine composition having optimum molecular weight of sized polysaccharide of serotype 4 (100-200 kDa), serotype 24F (100-200 kDa), serotype 3 (100-200 kDa), serotype 6A (400-900 kDa), serotype 6B (100-200 kDa), serotype 9V (100-200 kDa), serotype 15B (100-200 kDa), serotype 19A (100-200 kDa), serotype 22F (100-200 kDa)} and respective polysaccharide-protein conjugates thereby ensuring improved immunogenicity (as compared to when these polysaccharides are conjugated to CRM197).

Still another object of the present disclosure is to provide a multivalent *Streptococcus pneumoniae* polysaccharide-protein conjugate vaccine composition having minimum free polysaccharide content.

Other objects and advantages of the present disclosure will be more apparent from the following description, which is not intended to limit the scope of the present disclosure.

### SUMMARY

Applicant has found that 1) lower adsorption on alum is expected to make the respective conjugate antigens to be at risk of degradation over time when the vaccine formulation is on shelf, therefore for *Streptococcus pneumoniae* serotypes 4, 6B, 7F, 14, 15B, 18C, 19F, 22F, increasing the alum gel quantity from 125 µg to 187.5 µg per dose resulted in improved adsorption (at least 30 % for all serotypes) (at least 80 % for serotypes 1, 2, 3, 5, 7F, 8, 9V, 10A, 12F, 14, 18C, 19A, 19F, 23F 33F) and thereby providing increased B cell mediated IgG antibody response (IgG at least 2 fold higher); 2) higher quantity of polysorbate 20 in the multivalent pneumococcal conjugate vaccines have resulted in aggregation-free formulation, improved flow properties (especially in the presence of increased gel content with higher number of antigens being added); 3) novel improved, stable and immunogenic multivalent polysaccharide-protein conjugate vaccine composition (preferably 20 valent or 21 valent) having conjugates prepared by utilizing CDAP comprising of three different carrier proteins (DT, TT, CRM197), wherein serotypes 4 and 24F are individually conjugated to DT; serotypes 3, 6A, 9V, 15B, 19A, 22F are individually conjugated to TT, rest of the polysaccharides from serotypes (1, 2, 5, 6B, 7F, 8, 10A, 11A, 12F, 14, 18C, 19F, 23F, 33F) are individually conjugated to CRM197; 4) all CRM197 and DT based conjugates are prepared by activating with 1-cyano-4-Dimethyl amino pyridinium tetrafluoroborate (CDAP) using direct coupling approaches utilizing cyanylation chemistry whereas all TT based conjugates are obtained by first preparing ADH derivatized TT (in presence of EDAC) followed by conjugating it with CDAP activated polysaccharides; 5) optimum molecular weight of sized Polysaccharide of serotype 4 is in the range of 130 kDa to 170 kDa (SEC-HPLC), serotype 24F is in the range of 100 kDa to 200 kDa (SEC-HPLC), serotype 3 is in the range of 140 kDa to 180 kDa (SEC-HPLC), serotype 6A is in the range of 400 kDa to 900 kDa (SEC-HPLC), serotype 6B is in the range of 100 kDa to 200 kDa (SEC-HPLC), serotype 9V is in the range of 120 kDa to 160 kDa (SEC-HPLC), serotype 15B is in the range of 120 kDa to 160 kDa (SEC-HPLC), serotype 19A-120 kDa to 160 kDa (SEC-HPLC), 22F is in the range of 100 kDa to 150 kDa (SEC-HPLC)) and optimum molecular weight of polysaccharide-protein conjugates of serotype 4 is in the range of 3000 kDa to 10000 kDa (SEC-MALS), serotype 24F is in the range of 1500 kDa to 10000 kDa (SEC-MALS), serotype 3 is in the range of 3000 kDa to 10000 kDa (SEC-MALS), serotype 6A is in the range of 7000 kDa to 10000 kDa (SEC-MALS), serotype 6B is in the range of 4000 kDa to 15000 kDa (SEC-MALS), serotype 9V is in the range of 15000 kDa to 30000 kDa (SEC-MALS), serotype 15B is in the range of 6000 kDa to 10000 kDa (SEC-MALS), serotype 19A is in the range of 5000 kDa to 8000 kDa (SEC-MALS), serotype 22F is in the range of 9000 kDa to 18000 kDa (SEC-MALS) thereby ensuring improved immunogenicity (as compared to when these polysaccharides are conjugated to CRM197); 6) high conjugation efficiency (40 % to 45 % for serotypes 4 and 24F, 30 % to 35 % for serotype 3, 40 % to 45 % for serotype 6A, 25 % to 30 % for serotype 9V, 45 % to 55 % for serotype 15B, 30 % to 35 % for serotype 19A, 40 % to 45 % for serotype 22F); 7) optimum adsorption for conjugates (at least 30 %); 8) free polysaccharide content <15 % for all conjugates and free protein (<10 %); and 9) high polysaccharide recovery and structural integrity maintained by utilizing high pressure homogenization (as against chemical sizing).

Further, the vaccine compositions have been prepared using a two-blend approach, wherein some of the serotypes are adsorbed individually as a first set and remaining serotypes are adsorbed together as a second set, followed by mixing of both sets to get the vaccine composition.

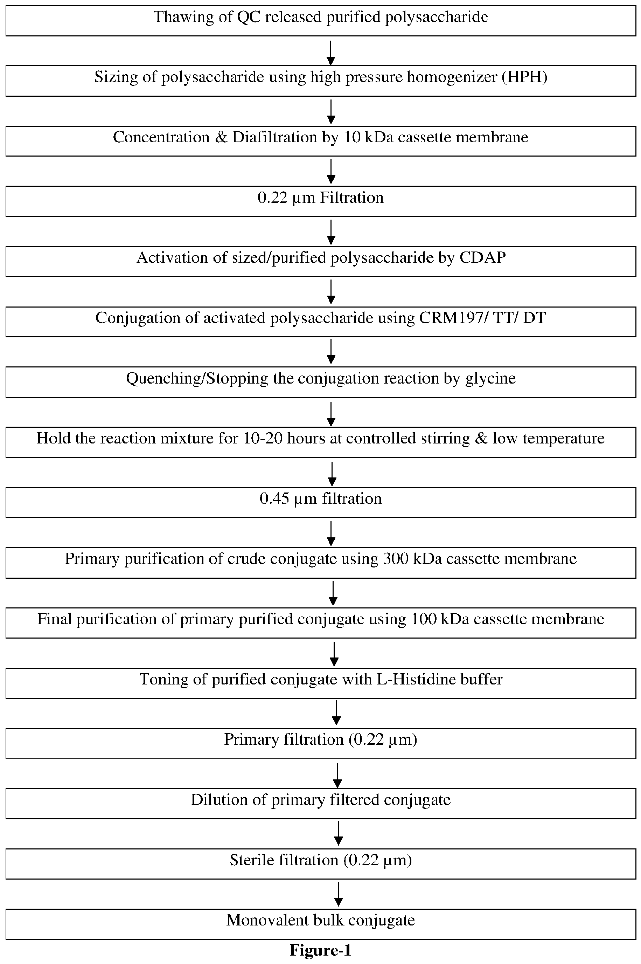

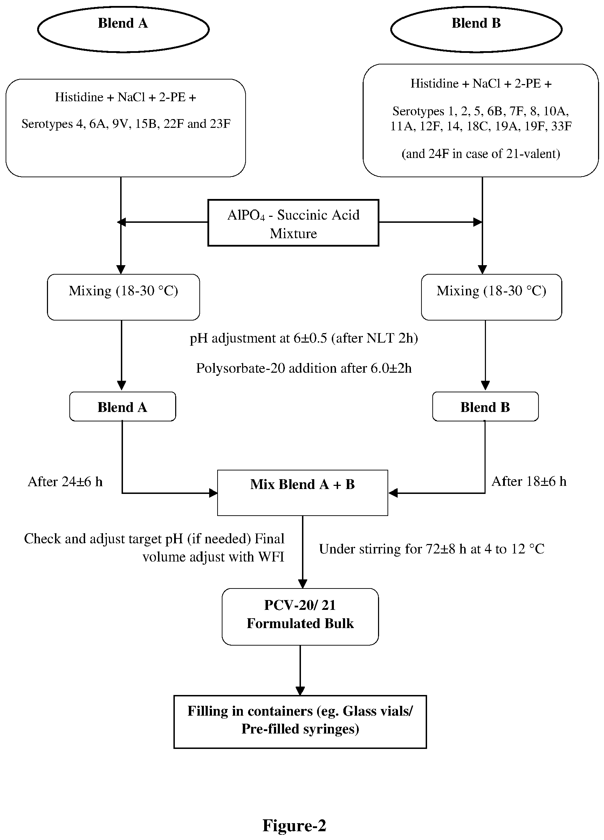

### BRIEF DESCRIPTION OF THE ACCOMPANYING DRAWING

The present disclosure will now be described with the help of the accompanying drawing, in which:
**Figure-1** illustrates the process flow for preparing pneumococcal polysaccharide-carrier protein conjugate in accordance with an embodiment of the present disclosure; and
**Figure-2** illustrates the process flow formulating the vaccine composition in accordance with an embodiment of the present disclosure.

### DETAILED DESCRIPTION

Although the present disclosure may be susceptible to different embodiments, certain embodiments are shown in the drawing and following detailed discussion, with the understanding that the present disclosure can be considered an exemplification of the principles of the disclosure and is not intended to limit the scope of disclosure to that which is illustrated and disclosed in this description.

Embodiments are provided so as to thoroughly and fully convey the scope of the present disclosure to the person skilled in the art. Numerous details are set forth, relating to specific components, and processes, to provide a complete understanding of embodiments of the present disclosure. It will be apparent to the person skilled in the art that the details provided in the embodiments should not be construed to limit the scope of the present disclosure. In some embodiments, well-known composition, well-known processes, and well-known techniques are not described in detail.

The terminology used, in the present disclosure, is only for the purpose of explaining a particular embodiment and such terminology shall not be considered to limit the scope of the present disclosure. As used in the present disclosure, the forms "a", "an", and "the" may be intended to include the plural forms as well, unless the context clearly suggests otherwise.

The terms "comprises", "comprising", "including", and "having" are open ended transitional phrases and therefore specify the presence of stated features, integers, steps, operations, elements, modules, units and/or components, but do not forbid the presence or addition of one or more other features, integers, steps, operations, elements, components, and/or groups thereof. The particular order of steps disclosed in the process of the present disclosure is not to be construed as necessarily requiring their performance as described or illustrated. It is also to be understood that additional or alternative steps may be employed.

The terms first, second, third, etc., should not be construed to limit the scope of the present disclosure as the aforementioned terms may be only used to distinguish one element, component, region, layer or section from another component, region, layer or section. Terms such as first, second, third etc., when used herein do not imply a specific sequence or order unless clearly suggested by the present disclosure.

It is understood that each feature or embodiment, or combination, described herein is a non-limiting, illustrative example of any of the aspects of the invention and, as such, is meant to be combinable with any other feature or embodiment, or combination, described herein. For example, where features are described with language such as "one embodiment", "some embodiments", "certain embodiments", "further embodiment", "specific exemplary embodiments", and/or "another embodiment", each of these types of embodiments is a non-limiting example of a feature that is intended to be combined with any other feature, or combination of features, described herein without having to list every possible combination. Such features or combinations of features apply to any of the aspects of the invention.

### Definitions:

In order for the present disclosure to be more readily understood, certain terms are first defined below. Additional definitions for the following terms and other terms may be set forth through the specification.

As used herein, 'serotype' as mentioned throughout the present disclosure refers to a *Streptococcus pneumoniae* serotype unless indicated otherwise.

As used herein, 'polysaccharide' refers to a polysaccharide/ saccharide/ sugar/ carbohydrate that is external to but contiguous with the cell wall of the *Streptococcus pneumoniae* serotypes: 1, 2, 3, 4, 5, 6, 6A, 6B, 6C, 6D, 6E, 6F, 6G, 6H, 7A, 7B, 7C, 7D, 7F, 8, 9A, 9L, 9F, 9N, 9V, 10A, 10B, 10C, 10D, 10F, 11, 11A, 11B, 11C, 11D, 11E, 11F, 12A, 12B, 12F, 13, 13F, 14, 15A, 15B, 15C, 15F, 16, 16A, 16F, 17A, 17F, 18, 18A, 18B, 18C, 18F, 19A, 19B, 19C, 19F, 20, 20A, 20B, 21, 22A, 22F, 23A, 23B, 23F, 24A, 24B, 24F, 25A, 25F, 27, 28A, 28F, 29, 31, 32A, 32F, 33A, 33B, 33C, 33D, 33E, 33F, 34, 35A, 35B, 35C, 35D, 35F, 36, 37, 38, 39, 40, 41A, 41F, 42, 43, 44, 45, 46, 47A, 47F, and 48.

As used herein, 'carrier protein' refers to any protein which is conjugated/ coupled/ attached conjugated to polysaccharide, typically for the purpose of enhancing or facilitating detection of the antigen by the immune system. The composition(s) of the present disclosure comprises more than one pneumococcal polysaccharide conjugate and each conjugate comprises a carrier protein conjugated to a polysaccharide from a different serotype of *Streptococcus pneumoniae.*

As used herein, `conjugate(s)' or 'polysaccharide-carrier protein conjugate(s)' refers to a *Streptococcus pneumoniae* polysaccharide conjugated to a carrier protein using CDAP conjugation chemistry.

As used herein, 'molecular weight' refers to the weight average molecular weight and is a measure of molecular size of polysaccharide as estimated either by HPLC-RI or HPLC-UV/RI or HPSEC-UV/RI/MALS. In case of pneumococcal polysaccharide(s), the molecular weight is calculated by size exclusion chromatography (SEC) in combination with high performance liquid chromatography (HPLC). The term SEC-HP-RI and SEC-HPLC is used interchangeably throughout the specification. In case of pneumococcal polysaccharide-carrier protein conjugate(s), the 'molecular weight' or 'molecular size' is calculated by High performance size exclusion chromatography (SEC) in combination with multi-angle laser light scattering (MALS).

As used herein, `vaccine composition' refers to a biological preparation that provides active acquired immunity against at least one *S. pneumoniae* serotype and can be interchangeably used with the term 'vaccine'.

As used herein, `effective amount' described in the present disclosure refers to an amount required to elicit an immune response in the subject to which the composition is administered. The immune response is characterized by the presence of one or more *Streptococcus pneumoniae* antigen-specific antibodies in the host that significantly reduce the likelihood or severity of infection of *Streptococcus pneumoniae* during a subsequent challenge.

As used herein, 'dose' refers to one/ single administration of the vaccine composition of the present disclosure and is typically 0.5 ml, unless otherwise mentioned.

As used herein, 'subject' refers to a mammal, including mice, rabbits, and humans. In certain embodiments the subject is an adult, an adolescent or an infant. In some embodiments, terms 'individual' or 'patient' are used and are intended to be interchangeable with 'subject'.

As used herein, the terms `polysorbate 20' and `tween 20' has been used interchangeably throughout the specification.

The present disclosure envisages methods for preparing the vaccine compositions using a two-blend approach. The *Streptococcus pneumoniae* polysaccharides are individually conjugated to carrier proteins and are manufactured using cyanylation conjugation chemistry to provide optimum immunogenicity and stability to the vaccine composition. More than one type of carrier protein is used in the vaccine composition. The present disclosure further envisages vaccine compositions comprising *Streptococcus pneumoniae* polysaccharides which are capable of prevention and/ or treatment of *Streptococcus pneumoniae* diseases.

In an aspect of the present disclosure, there is provided a method for preparing a stable and immunogenic multivalent pneumococcal polysaccharide-protein conjugate vaccine composition. The method comprises individually conjugating pneumococcal polysaccharides with specific carrier proteins. The obtained pneumococcal polysaccharide-carrier protein conjugates are mixed with histidine and NaCl, followed by adsorption onto an aluminium salt adjuvant in the presence of succinate as two separate blends comprising specific obtained pneumococcal polysaccharide-carrier protein conjugates. The two blends are then mixed to obtain the vaccine composition of the present disclosure. The method is described in detail in the following paragraphs.

In an embodiment of the present disclosure, the method for preparing the vaccine composition comprises the following steps:
i. individually conjugating pneumococcal polysaccharide derived from *Streptococcus pneumoniae* serotypes selected from the group comprising of 1, 2, 3, 4, 5, 6, 6A, 6B, 6C, 6D, 6E, 6F, 6G, 6H, 7A, 7B, 7C, 7D, 7F, 8, 9A, 9L, 9F, 9N, 9V, 10A, 10B, 10C, 10D, 10F, 11, 11A, 11B, 11C, 11D, 11E, 11F, 12A, 12B, 12F, 13, 13F, 14, 15A, 15B, 15C, 15F, 16, 16A, 16F, 17A, 17F, 18, 18A, 18B, 18C, 18F, 19A, 19B, 19C, 19F, 20, 20A, 20B, 21, 22A, 22F, 23A, 23B, 23F, 24A, 24B, 24F, 25A, 25F, 27, 28A, 28F, 29, 31, 32A, 32F, 33A, 33B, 33C, 33D, 33E, 33F, 34, 35A, 35B, 35C, 35D, 35F, 36, 37, 38, 39, 40, 41A, 41F, 42, 43, 44, 45, 46, 47A, 47F, and 48 to a carrier-protein in a ratio between 1: 0.5 and 1: 1.5, and at a pH in range of 8.0 to 10 to obtain polysaccharide-carrier protein conjugates;
ii. adsorbing a first set of pneumococcal polysaccharide-protein conjugates comprising polysaccharides derived from *Streptococcus pneumoniae* serotypes selected from the group consisting of 4, 6A, 9V, 15B, 22F and 23F obtained in step (i), on an aluminium salt adjuvant in presence of a salt, histidine and succinic acid at a temperature in the range of 18 °C to 30 °C, pH in the range of 5.0 to 7.0, for a time period of 18 hours to 30 hours;
iii. adsorbing a second set of pneumococcal polysaccharide-carrier protein conjugates comprising polysaccharides derived from *S. pneumoniae* serotypes selected from the group comprising 1, 2, 3, 4, 5, 6, 6A, 6B, 6C, 6D, 6E, 6F, 6G, 6H, 7A, 7B, 7C, 7D, 7F, 8, 9A, 9L, 9F, 9N, 9V, 10A, 10B, 10C, 10D, 10F, 11, 11A, 11B, 11C, 11D, 11E, 11F, 12A, 12B, 12F, 13, 13F, 14, 15A, 15B, 15C, 15F, 16, 16A, 16F, 17A, 17F, 18, 18A, 18B, 18C, 18F, 19A, 19B, 19C, 19F, 20, 20A, 20B, 21, 22A, 22F, 23A, 23B, 23F, 24A, 24B, 24F, 25A, 25F, 27, 28A, 28F, 29, 31, 32A, 32F, 33A, 33B, 33C, 33D, 33E, 33F, 34, 35A, 35B, 35C, 35D, 35F, 36, 37, 38, 39, 40, 41A, 41F, 42, 43, 44, 45, 46, 47A, 47F, and 48, 6A, 9V, 15B, 22F and 23F obtained in step (i), on an aluminium salt adjuvant in presence of a salt, histidine, and succinic acid at a temperature in the range of 18 °C to 30 °C, pH in the range of 5.0 to 7.0, for a time period of 12 hours to 24 hours; and
iv. blending the first set obtained in step (ii) with the second set obtained in step (iii) in presence of a surfactant in the range of 80 µg to 150 µg per 0.5 ml dose of the composition with the help of a Rushton turbine flat blade impeller for a time period of 50 hours to 80 hours, at a temperature in the range of 4°C to 12°C.

In an embodiment of the present disclosure, the pneumococcal polysaccharides are conjugated to carrier protein using cyanylation reaction, and the vaccine composition comprises at least three distinct carrier proteins.

In accordance with the embodiments of the present disclosure, the vaccine composition comprises i) total concentration of aluminium salt adjuvant in the range of 20-375 µg of Al³⁺ per 0.5 ml dose of the composition, ii) each polysaccharide-carrier protein has an adsorption percentage of at least 30 %, iii) preserves the desired physicochemical and immunogenic characteristics of the polysaccharide saccharide-carrier protein conjugate, and iv) the surfactant in the range of 80 µg to 150 µg per 0.5 ml dose of the composition results in minimal aggregate and particle formation and improved flow properties of the vaccine composition.

In another embodiment, the second set of pneumococcal polysaccharide-carrier protein conjugates comprises *S. pneumoniae* polysaccharides derived from serotypes selected from the group comprising 1, 2, 3, 4, 5, 6, 6A, 6B, 6C, 6D, 6E, 6F, 6G, 6H, 7A, 7B, 7C, 7D, 7F, 8, 9A, 9L, 9F, 9N, 9V, 10A, 10B, 10C, 10D, 10F, 11, 11A, 11B, 11C, 11D, 11E, 11F, 12A, 12B, 12F, 13, 13F, 14, 15A, 15B, 15C, 15F, 16, 16A, 16F, 17A, 17F, 18, 18A, 18B, 18C, 18F, 19A, 19B, 19C, 19F, 20, 20A, 20B, 21, 22A, 22F, 23A, 23B, 23F, 24A, 24B, 24F, 25A, 25F, 27, 28A, 28F, 29, 31, 32A, 32F, 33A, 33B, 33C, 33D, 33E, 33F, 34, 35A, 35B, 35C, 35D, 35F, 36, 37, 38, 39, 40, 41A, 41F, 42, 43, 44, 45, 46, 47A, 47F, and 48.

In a preferred embodiment, the second set of pneumococcal polysaccharide-carrier protein conjugates comprise *S. pneumoniae* polysaccharides derived from serotypes selected from the group consisting of 1, 2, 3, 5, 6B, 7F, 8, 10A, 11A, 12F, 14, 18C, 19A, 19F, 24F, and 33F.

In an embodiment, the second set of pneumococcal polysaccharide-carrier protein conjugates comprise *S. pneumoniae* polysaccharides derived from serotypes selected from the group consisting of 1, 2, 5, 6B, 7F, 8, 10A, 11A, 12F, 14, 18C, 19A, 19F and 33F.

In an embodiment, the second set of pneumococcal polysaccharide-carrier protein conjugates comprises *S. pneumoniae* polysaccharides derived from serotypes selected from the group consisting of 1, 2, 3, 5, 6B, 7F, 8, 10A, 11A, 12F, 14, 18C, 19A, 19F, 24F, and 33F.

In an embodiment, the second set of pneumococcal polysaccharide-carrier protein conjugates comprises *S. pneumoniae* polysaccharides derived from serotypes selected from the group consisting of 1, 2, 5, 6B, 7F, 8, 10A, 11A, 12F, 14, 18C, 19A, 19F, 24F, and 33F.

In another embodiment, the second set of pneumococcal polysaccharide-carrier protein conjugates comprise at least one *S. pneumoniae* polysaccharide derived from serotypes 9A, 9N, and 9L.

In still another embodiment, the second set of pneumococcal polysaccharide-carrier protein conjugates is devoid of *S*. *pneumoniae* polysaccharide derived from serotype 3.

The pneumococcal polysaccharides of the vaccine compositions of the present disclosure are extracted from *Streptococcus pneumoniae* using known methods. The fermentation, purification, sizing of *Streptococcus pneumoniae* serotypes may be carried out as per the methods disclosed in 786/MUM/2007, 1075/MUM/2009, 282/MUM/2012, WO2012127485, WO2013088448, 2889/MUM/2014, IN201623002962, WO2016199003, IN201921036006, or WO2021229604. Briefly, pneumococcal polysaccharides are obtained by growing the individual *S. pneumoniae* serotypes in a medium at a pH in the range of 7 to 8, a temperature in the range of 35-38°C under stirring at 50 to 150 RPM with an air flow rate of 0 to 0.5 vvm. The obtained harvest is concentrated and diafiltered and is typically followed by enzyme and chemical treatment to extract the polysaccharide. This is then followed by centrifugation and one or more chromatography steps for removal of impurities and further diafiltered to obtain purified native polysaccharides.

In an embodiment, the clarified fermentation broth of the pneumococcal serotypes obtained above are concentrated and diafiltered (100 KDa MWCO membrane; 25 mM sodium phosphate buffer at neutral pH followed by diafiltration with water for injection (WFI)). The concentrated and diafiltered broth containing polysaccharide is subjected to enzyme treatment (nuclease) at 37°C, for 10 ± 2 hours with stirring. The enzyme treated polysaccharide solution is treated with a chemical, such as ammonium sulphate and subjected to centrifugation, followed by diafiltration. The diafiltered solution containing polysaccharide is subjected to hydrophobic interaction chromatography (HIC) column, followed by concentration and diafiltration. This is followed by another chromatography step involving ion exchange chromatography column. The polysaccharides on to the column are eluted using a salt (NaCl) (various polysaccharides were eluted at different ionic strengths of salt). The polysaccharide solution is then concentrated using and then diafiltered. The diafiltered polysaccharide solution is further filtered and stored frozen until further use.

In an embodiment, the method of present disclosure is devoid of a multimodal chromatography step during purification of polysaccharide(s).

In another embodiment, the method of present disclosure is devoid of a flocculation step (multivalent cation selected from the group consisting of aluminium, iron, calcium and magnesium) during purification of the polysaccharide(s).

The bacterial strains used to prepare the polysaccharides may be obtained from established culture collection centers, such as, Centers for Disease Control and Prevention (CDC), Atlanta USA; CBER/FDA, USA; ATCC, USA; NIH, USA; NIAID, USA; PHE, UK and the like. Similarly, the carrier proteins used in the present disclosure may be procured from established sources.

In accordance with the embodiments of the present disclosure, (i) pneumococcal serotypes 1, 2, 3, 4, 5, 6A, 6B, 7F, 8, 9V, 10A, 11A, 12F, 14, 15B, 18C, 19A, 19F, 22F, 23F, and 33F are obtained from Centers for Disease Control and Prevention (CDC) Atlanta, USA; (ii) pneumococcal serotype 24F is obtained from Kempegowda Institute of Medical Sciences (KIMS), Bangalore, India; (iii) *Pseudomonas fluorescens* (for CRM197) is obtained from Pfenex Inc, USA; (iv) Corynebacterium diphtheriae (strain CN 2000) for Diphtheria Toxoid (DT) was obtained by the National Control Authority Central Research Institute (C.R.I.) Kasauli, HP, India from Wellcome Research Laboratory, London, UK and the strain was issued to Serum Institute of India Private Limited in 1973; and (v) *Clostridium tetani* (Harvard 49205) was obtained by the National Control Authority Central Research Institute (C.R.I.) Kasauli, HP, India from Netherlands Vaccine Institute (NVI) and the strain was issued to Serum Institute of India Private Limited in 1973.

In accordance with the embodiments of the present disclosure, the total nitrogen content (%) in the purified polysaccharide is determined/ measured using known methods. The total nitrogen content may be determined/ measured using TOC TNM technique, Kjeldahl method, CHNS method, NMR, HPAEC-PAD or any other method. In a preferred embodiment, the total nitrogen content may be determined/ measured using TOC TNM technique.

In an embodiment, the extracted and purified pneumococcal polysaccharides are used in the native form.

In another embodiment, the extracted and purified pneumococcal polysaccharides are sized to obtain one or more parts of the pneumococcal polysaccharides. The molecular weight of the sized polysaccharides is less than that of the extracted and purified pneumococcal polysaccharides in the native form.

In accordance with the embodiments of the present disclosure, the pneumococcal polysaccharide is sized prior to conjugation with the respective carrier protein using one or more of thermal treatment, sonic treatment, mechanical means, chemical hydrolysis, endolytic enzyme treatment, physical shear, Gaulin-homogenizer, and sonication to obtain sized pneumococcal polysaccharide having a molecular weight in the range of 50 kDa to 600 kDa (SEC-HPLC). In a preferred embodiment, the sized pneumococcal polysaccharide has a molecular weight in the range of 100 kDa to 200 kDa (SEC-HPLC). In an embodiment, the sizing is carried out using a chemical hydrolysis using one or more of FeCl₃, H₂O₂, sodium metaperiodate and sodium acetate treatment.

It is seen that the conjugation efficiency/conjugate yield, immunogenicity of conjugates prepared from using partially size reduced polysaccharides is higher as compared to conjugates prepared from full length (native) polysaccharides Also, size reduction of polysaccharides decreases the viscosity of the solution and increases the number of reactive end groups, contributing to an increased frequency of covalent bond formation.

In an embodiment, the sizing is carried out using mechanical means using high pressure homogenizer. In an embodiment, sizing is carried out at 20-30 KPSI (kilo-pounds per-square-inch), preferably 24-30 KPSI using at least 1 pass. In another embodiment, 1 to 3 passes are used to obtain the sized polysaccharide of desired molecular weight. In yet another embodiment, more than 3 passes may also be used to obtain the sized polysaccharide of desired molecular weight. The KPSI may vary depending on the *S. pneumoniae* serotype from which the polysaccharide is derived. In accordance with the embodiments of the present disclosure, high polysaccharide recovery and maintenance of structural integrity of polysaccharides is observed when high pressure homogenization is used for sizing the polysaccharides as compared to when chemical sizing method is used foe polysaccharide sizing.

In another embodiment, the pneumococcal polysaccharide is not subjected to sizing before conjugation with the carrier protein. In an embodiment, the polysaccharide derived from serotype 6A is not sized before conjugation with the carrier protein. In an embodiment, the polysaccharide derived from serotype 6A is used in the native form and has a molecular weight in the range of 400 kDa to 900 kDa (SEC-HPLC), preferably in the range of 700 kDa to 800 kDa (SEC-HPLC).

In an embodiment, the molecular weight of the sized polysaccharide derived from serotype 4 is in the range of 130 kDa to 170 kDa (SEC-HPLC), preferably in the range of 130 kDa to 165 kDa (SEC-HPLC).

In an embodiment, the molecular weight of the sized polysaccharide derived from serotype 3 is in the range of 140 kDa to 180 kDa (SEC-HPLC), preferably in the range of 150 kDa to 170 kDa (SEC-HPLC).

In an embodiment, the molecular weight of the sized polysaccharide derived from serotype 24F is in the range of 100 kDa to 200 kDa (SEC-HPLC), preferably in the range of 120 kDa to 170 kDa (SEC-HPLC), more preferably in the range of 130 kDa to 160 kDa (SEC-HPLC).

In an embodiment, the molecular weight of the sized polysaccharide derived from serotype 6B is in the range of 100 kDa to 200 kDa (SEC-HPLC), preferably in the range of 110 kDa to 175 kDa (SEC-HPLC), more preferably in the range of 130 kDa to 160 kDa.

In an embodiment, the molecular weight of the sized polysaccharide derived from serotype 9V is in the range of 120 kDa to 160 kDa (SEC-HPLC), preferably in the range of 130 kDa to 150 kDa (SEC-HPLC).

In an embodiment, the molecular weight of the sized polysaccharide derived from serotype 15B is in the range of 120 kDa to 160 kDa (SEC-HPLC), preferably in the range of 130 kDa to 150 kDa (SEC-HPLC).

In an embodiment, the molecular weight of the sized polysaccharide derived from serotype 19A is in the range of 120 kDa to 160 kDa (SEC-HPLC), preferably in the range of 130 kDa to 150 kDa (SEC-HPLC).

In an embodiment, the molecular weight of the sized polysaccharide derived from serotype 22F is in the range of 100 kDa to 150 kDa (SEC-HPLC), preferably in the range of 120 kDa to 140 kDa (SEC-HPLC).

In another embodiment, the polysaccharide derived from *S. pneumoniae* serotype 24F is sized using high pressure homogenizer. In an embodiment, the polysaccharide derived from *S*. *pneumoniae* serotype 24F is not sized using acid hydrolysis (acetic acid, HCl, phosphoric, citric or mixture).

In another embodiment, the native polysaccharide derived from *S. pneumoniae* serotype 6B has molecular weight >200 kDa (SEC-HPLC), which after size reduction using homogenizer is <200 kDa (SEC-HPLC). In still another embodiment, the vaccine composition of present disclosure is devoid of polysaccharide derived from *S. pneumoniae* serotype 6B having molecular weight 500-1800 kDa (SEC-HPLC).

In an embodiment of the present disclosure, the polysaccharide derived from *S. pneumoniae* serotype is sized using trifluoroacetic acid (TFA) to obtain sized polysaccharide having molecular weight in the range of 100 kDa to 200 kDa (SEC-HPLC).

In an embodiment, the polysaccharide derived from *S. pneumoniae* serotype 18C has >80% O-acetylation.

In accordance with the embodiments of the present disclosure, the salt in step (ii) and step (iii) is selected from the group comprising magnesium chloride, potassium chloride, sodium chloride and combinations thereof and the concentration of the salt in the vaccine composition is in the range of 2 mg to 10 mg per 0.5 ml dose of the vaccine composition. In an embodiment, the salt is sodium chloride. In a preferred embodiment, the concentration of the salt in the vaccine composition is 4.5 mg per 0.5 ml dose of the vaccine composition.

In another embodiment, histidine in step (ii) and step (iii) is in the range of 0.1 mg to 10 mg per 0.5 ml dose of the vaccine composition. In still another embodiment, succinic acid in step (ii) and step (iii) is in the range of 0.1 mg to 10 mg per 0.5 ml dose of the vaccine composition. Preferably the concentration of succinic acid in final formulation of multivalent conjugate vaccine is 20 mM (2.36 mg/mL).

Typically, the surfactant in step (iv) is selected from the group comprising polysorbate, polymer glycol, sorbitan ester, and combinations thereof; and the concentration of surfactant is in the range of 90 µg to 120 µg per 0.5 ml dose of the vaccine composition. In a preferred embodiment, the surfactant in step (iv) is polysorbate 20 and the concentration of polysorbate 20 is in the range of 80 µg to 120 µg per 0.5 ml dose of the vaccine composition.

In an embodiment, the pneumococcal polysaccharide is individually conjugated to carrier protein using a carbodiimide, reductive amination, or cyanylation conjugation reaction.

Polysaccharides can be conjugated to respective carrier proteins using different approaches. Amino acid residues that are most suitable for chemical linkage to polysaccharides are those which are exposed onto the carrier protein surface and whose side chains have reactive functional groups, such as primary amino groups of lysines and carboxylic groups of glutamic or aspartic acid residues. Carboxyl groups of carrier proteins can be conjugated to amino or hydrazide derivatives of polysaccharides by a carbodiimide mediated condensation; and lysine amino groups can be conjugated to polysaccharides by reaction with their derivatives containing active groups such as succinimido esters or cyano-esters, squarate, or by direct reductive amination.

In accordance with the embodiments of the present disclosure, the pneumococcal polysaccharides are conjugated to one or more carrier proteins selected from the group comprising CRM₁₉₇, diphtheria toxoid (DT), tetanus toxoid (TT), *Neisseria meningitidis* outer membrane complex, fragment C of tetanus toxoid, recombinant full-length tetanus toxin with eight individual amino acid mutations (8MTT), diphtheria toxin fragment B (DTFB) , pertussis toxoid, protein D of *H. influenzae, E. coli* LT, *E. coli* ST, exotoxin A from *Pseudomonas aeruginosa,* outer membrane complex c (OMPC), porins, fHbp, Por A, Por B, transferrin binding proteins, pneumolysin, pneumococcal surface protein A (PspA), pneumococcal surface adhesin A (PsaA), PhtA, PhtB, PhtE, pneumococcal PhtD, pneumococcal surface proteins BVH-3 and BVH-11, M. catarrhalis uspA, protective antigen (PA) of *Bacillus anthracis* and detoxified edema factor (EF) and lethal factor (LF) of *Bacillus anthracis,* ovalbumin, keyhole limpet hemocyanin (KLH), C5a peptidase group A or group B *Streptococcus,* human serum albumin, bovine serum albumin (BSA), purified protein derivative of tuberculin (PPD), Cholera toxin subunit B, synthetic peptides, heat shock proteins, pertussis proteins, cytokines, lymphokines, hormones, growth factors, artificial proteins comprising multiple human CD4+ T cell epitopes from various pathogen-derived antigens such as N 19, iron-uptake proteins, toxin A or B from *C*. *difficile* and *S*. *agalactiae* proteins and any equivalents thereof.

The carrier proteins should contain a sufficient number of exposed amino acids targeted for the selected conjugation process, should be stable and have good solubility in the buffers and concentrations at which the conjugation reactions take place.

In an embodiment, the method of the present disclosure makes use of three different carrier proteins, namely, diphtheria toxoid (DT), tetanus toxoid (TT) and CRM197. The pneumococcal polysaccharides individually are conjugated to the respective carrier protein.

In a preferred embodiment of the present disclosure, at least one pneumococcal polysaccharide derived from serotypes selected from the group comprising 3, 6A, 9V, 15B, 19A, and 22F is conjugated to tetanus toxoid as the carrier protein, at least one pneumococcal polysaccharide derived from serotypes 4 and 24F is conjugated to diphtheria toxoid as the carrier protein and at least one pneumococcal polysaccharide derived from serotype selected from the group comprising 1, 2, 3, 4, 5, 6, 6A, 6B, 6C, 6D, 6E, 6F, 6G, 6H, 7A, 7B, 7C,7D,7F, 8, 9A, 9L, 9F, 9N, 9V, 10A, 10B, 10C, 10D, 10F, 11, 11A, 11B, 11C, 11D, 11E, 11F, 12A, 12B, 12F, 13, 13F, 14, 15A, 15B, 15C, 15F, 16, 16A, 16F, 17A, 17F, 18, 18A, 18B, 18C, 18F, 19A, 19B, 19C, 19F, 20, 20A, 20B, 21, 22A, 22F, 23A, 23B, 23F, 24A, 24B, 24F, 25A, 25F, 27, 28A, 28F, 29, 31, 32A, 32F, 33A, 33B, 33C, 33D, 33E, 33F, 34, 35A, 35B, 35C, 35D, 35F, 36, 37, 38, 39, 40, 41A, 41F, 42, 43, 44, 45, 46, 47A, 47F, and 48 is conjugated to CRM197 as the carrier protein.

In an embodiment of the present disclosure, pneumococcal polysaccharide derived from serotypes 19A and 19F are individually conjugated to same type of carrier protein selected from tetanus toxoid, diphtheria toxoid and CRM197.

In an embodiment of the present disclosure, pneumococcal polysaccharide derived from serotypes 19A and 19F are individually conjugated to different types of carrier proteins selected from tetanus toxoid, diphtheria toxoid and CRM197.

There are two conjugation methods generally used for producing immunogenic polysaccharide-carrier protein conjugates: (1) direct conjugation of the polysaccharide and the carrier protein and (2) indirect conjugation of the polysaccharide and the carrier protein via a bifunctional linker or spacer reagent. Typically, both direct and indirect conjugation methods require chemical activation of the carbohydrate moiety prior to conjugation with the carrier protein.

Typically, the purified polysaccharide is chemically activated/ modified to generate reactive groups that can couple/ link to the carrier protein. Two of the most commonly used methods for polysaccharide activation are periodate oxidation and cyanylation. In periodate oxidation, terminal hydroxyl groups present on the polysaccharide are oxidized to an aldehyde using an oxidizing agent (periodate, such as sodium periodate, potassium periodate or periodic acid). The vicinal hydroxyl groups of the polysaccharide are cleaved by the oxidizing agent, resulting in the formation of the reactive aldehyde groups.

An important potential problem with use of periodate to activate the polysaccharide is the alteration of the physical structure of the polysaccharide, with loss of important epitopes. It is seen that even low levels of activation can result in loss of the specific epitope and creation of new epitopes.

Cyanylation chemistry involves creating reactive cyano ester groups on the polysaccharide. Cyanogen bromide (CNBr) was initially used as the cyanylating agent and has been mostly replaced by 1-cyano-4-dimethylaminopyridinium tetraflouroborate (CDAP) as the cyanylating agent. Cyanylation chemistry initially utilized CNBr to create the reactive cyanoester groups on the polysaccharides used in the first Hib conjugate reported in 1980 by Dr. John Robbins.

In accordance with the embodiments of the present disclosure, the pneumococcal polysaccharides may be conjugated to carrier protein using a conjugation reaction/ chemistry selected from the group consisting of cyanylation chemistry, carbodiimide chemistry, CNBr chemistry, and reductive amination chemistry.

In an embodiment of the present disclosure, the preferably conjugation reaction is carried out using a cyanylation agent selected from the group consisting of 1-cyano-4-dimethylaminopyridinium tetrafluoroborate (CDAP), CDAP-green, 1-cyano-4-pyrrolidinopyridinium tetrafluoroborate (CPPT), 1-cyano-imidazole (1-CI), 1-cyanobenzotriazole (1-CBT), 2-cyanopyridazine-3(2H)one (2-CPO), a functional derivative, and modifications thereof.

In accordance with the embodiments of the present disclosure, the pneumococcal polysaccharides are conjugated to carrier protein using a cyanylation reaction. The pneumococcal polysaccharides are reacted with 1-cyano-4-dimethylaminopyridinium tetrafluoroborate (CDAP) and mixed in a ratio of between 1:0.3 to 1:2 by weight of pneumococcal polysaccharides: CDAP at temperature in range of 22 °C to 25 °C for a time period of 4 minutes to 10 minutes, and contacting the CDAP activated polysaccharide with carrier protein mixed in a ratio of between 1: 0.5 and 1: 1.5 by weight of CDAP activated pneumococcal polysaccharides: carrier protein, reaction carried out at a pH in range of 8.0 to 10 for a time period of 3 hours to 5 hours followed by quenching with glycine to obtain the polysaccharide-carrier protein conjugates.

In a preferred embodiment of the present disclosure, the activation of the pneumococcal polysaccharides is carried out using 1-cyano-4-dimethylaminopyridinium tetraflouroborate (CDAP). CDAP reacts with the polysaccharide, exchanging a cyano group for a hydroxyl hydrogen, which are abundantly found on the polysaccharide, resulting in the creation of a cyanoester. This cyanoester is highly reactive. The activation is typically carried out at pH 9 to 10, and in fact there is a strong pH dependence on CDAP PS activation efficiency. The cyanoesters then react with the epsilon amines of lysine to form a stable O-alkyl-isourea linkage. The CDAP activation takes only a few minutes and conjugation is complete in a few hours. The reaction is then quenched using an amino-containing reagent such as glycine.

In accordance with the embodiments of the present disclosure, the polysaccharide to CDAP (Ps:CDAP) ratio (wt/ wt basis) for following serotypes are: serotype 4- 1: 0.8; serotype 24F-1:1±0.25; serotype 3-1:0.3; serotype 6A-1:1; serotype 9V-1:1; serotype 15B-1:1.2; serotype 19A-1:1; and serotype 22F-1:1.

In accordance with the embodiments of the present disclosure, the polysaccharide to carrier protein (Ps:Pr) ratio (wt/wt basis) for following serotypes are: serotype 4-1:0.9; serotype 24F- 1:1±0.25; serotype 3-1:1; serotype 6A-1:1.2; serotype 9V-1:1; serotype 15B-1:1; serotype 19A-1:1; and serotype 22F-1:1.

Further, sodium periodate oxidizes diols (2 adjacent carbons with hydroxyl groups) into aldehydes (RHC=O) and in this process breaks C-C bonds. Thus, depending upon the polysaccharide structure, periodate activation can fragment (downsize) a polysaccharide containing in-chain diols or open the ring structure of monosaccharide repeating units, which can alter the conformation, and potentially also the immunogenicity of the polysaccharide. The cyanylation chemistry using 1-cyano-4-dimethylaminopyridinium tetrafluoroborate (CDAP) allows a direct conjugation (without the use of a spacer) of polysaccharide antigens to proteins. CDAP reacts with the free OH groups of the polysaccharide with no chain breakage, creating cyanoester groups highly reactive with amino groups of proteins to form covalent linkages. This also allows better preservation of the native polysaccharide epitopes and has a positive impact on the immunogenicity of the vaccine compositions.

The present disclosure uses CDAP based activation of the pneumococcal polysaccharides prior to conjugation and therefore overcomes the drawbacks associated with reductive amination and CNBr based conjugation chemistry.

In an embodiment, the conjugation process of the present disclosure does not involve activation of polysaccharide using sodium acetate followed by conjugation with a carrier protein using reducing agent (sodium cyanoborohydride). In an embodiment, polysaccharide derived from serotype 23F with CRM197 as the carrier protein does not involve activation of polysaccharide using sodium acetate followed by conjugation using reducing agent (sodium cyanoborohydride).

In an embodiment, the method of present disclosure does not involve use of an eTEC spacer for conjugation of polysaccharide with a carrier protein. In an embodiment, prior to conjugation, the carrier protein(s), are derivatized to comprise, amino and/or carboxyl groups via a hetero or homo bifunctional linker selected from the group consisting of hydrazine, carbohydrazide, hydrazine chloride, a dihydrazide, ε-aminohexanoic acid, chlorohexanol dimethyl acetal, D-glucuronolactone, cystamine and p nitrophenylethyl amine, hexanediamine, ethylenediamine, 1,6-diaminooxyhexane or β-propinamido, nitrophenyl ethylamine, haloalkyl halide, 6-aminocaproic acid, and combinations thereof. The derivatization may be carried out using a carbodiimide, reductive amination or cyanylation reaction.

In a preferred embodiment, the hetero or homo-bifunctional linker is a dihydrazide. In an exemplary embodiment, the hetero or homo-bifunctional linker is adipic acid dihydrazide (ADH).

Hydrazide groups can be introduced into proteins through the carboxyl groups of aspartic acid and glutamic acid residues on the protein using a carbodiimide, reductive amination, cyanylation, reaction, for example, by reaction with hydrazine, carbohydrazide, succinyl dihydrazide, adipic acid dihydrazide, hydrazine chloride (e.g., hydrazine dihydrochloride) or any other dihydrazides in the presence of carbodiimide, such as 1-ethyl-3(3 dimethylaminopropyl) carbodiimide (EDC). EDC is employed as a catalyst to activate and modify the protein reactant with hydrazine or the dihydrazide.

In an embodiment, polysaccharides derived from *S. pneumoniae* serotypes 6A, 9V, 19A, 22F, 15B are conjugated independently conjugated to TT as the carrier protein. TT is first derivatized using ADH (in the presence of EDAC) and is then conjugated to the respective polysaccharides that have been activated using CDAP.

In another embodiment, polysaccharide derived from *S. pneumoniae* serotype 3 is independently conjugated to TT as the carrier protein.

In an embodiment, the pneumococcal polysaccharide is conjugated to the carrier protein in the absence of a linker.

In an embodiment, the pneumococcal polysaccharide-carrier protein conjugates of the vaccine composition of the present disclosure are not prepared using reductive amination chemistry. Further, the method for preparing the vaccine composition in accordance with the present disclosure is devoid of reagents/ chemicals/ enzymes such as protease, proteinase, silicon dioxide, acetic acid, DMSO, oxidizing agent (periodate, sodium periodate or meta periodate) and sodium borohydride. In another embodiment, the method of present disclosure does not use bicarbonate/ carbonate buffer for purification of polysaccharide derived from *S. pneumoniae* derived from serotype 1.

In an embodiment, the aluminium salt adjuvant in step (ii) and step (iii) is selected from the group comprising aluminium hydroxide, aluminium phosphate, aluminum hydroxyphosphate, and potassium aluminum sulfate and mixtures thereof. The concentration of aluminium salt adjuvant is in the range of 20 µg to 375 µg of Al³⁺ per 0.5 ml dose of the vaccine composition. In a preferred embodiment, the total concentration of aluminium salt adjuvant is in the range of 170 µg to 200 µg of Al³⁺ per 0.5 ml dose of the vaccine composition.

In accordance with the embodiments of the present disclosure, the pneumococcal polysaccharide-carrier protein conjugates have free polysaccharide less than 15 %, and free protein less than 10 %.

In an embodiment, the adsorption in step (ii) and step (iii) is carried out in the presence of a preservative. The amount of preservative is in the range of 1 mg to 8 mg per 0.5 ml dose of the vaccine composition. The preservative is selected from the group comprising 2-phenoxyethanol, benzethonium chloride (Phemerol), phenol, m-cresol, thiomersal, formaldehyde, methyl paraben, propyl paraben, benzalkonium chloride, benzyl alcohol, chlorobutanol, p-chlor-m-cresol, benzyl alcohol and combinations thereof. In a preferred embodiment, the preservative is 2-phenoxyethanol having concentration in the range of 2.5 mg to 5 mg per 0.5 ml dose of the vaccine composition, preferably NMT 5 mg/dose of 0.5 ml. In an exemplary embodiment of the present disclosure, the concentration of 2-phenoxyethanol is 4 mg per 0.5 ml dose of the vaccine composition.

In another aspect of the present disclosure, there is provided a stable and immunogenic multivalent pneumococcal polysaccharide-protein conjugate vaccine composition comprising at least one pneumococcal polysaccharide-carrier protein conjugate.

In accordance with the embodiments of the present disclosure, the pneumococcal polysaccharide is derived from the *S. pneumoniae* serotypes selected from the group comprising 1, 2, 3, 4, 5, 6, 6A, 6B, 6C, 6D, 6E, 6F, 6G, 6H, 7A, 7B, 7C, 7D, 7F, 8, 9A, 9L, 9F, 9N, 9V, 10A, 10B, 10C, 10D, 10F, 11, 11A, 11B, 11C, 11D, 11E, 11F, 12A, 12B, 12F, 13, 13F, 14, 15A, 15B, 15C, 15F, 16, 16A, 16F, 17A, 17F, 18, 18A, 18B, 18C, 18F, 19A, 19B, 19C, 19F, 20, 20A, 20B, 21, 22A, 22F, 23A, 23B, 23F, 24A, 24B, 24F, 25A, 25F, 27, 28A, 28F, 29, 31, 32A, 32F, 33A, 33B, 33C, 33D, 33E, 33F, 34, 35A, 35B, 35C, 35D, 35F, 36, 37, 38, 39, 40, 41A, 41F, 42, 43, 44, 45, 46, 47A, 47F, and 48.

In accordance with the embodiments of the present disclosure, the composition is a 15, 16, 17, 18, 19, 20 21, 22, 23, 24, 25, 26, 27, 28, 29, or 30-valent pneumococcal polysaccharide-carrier protein conjugate composition.

In an embodiment of the present disclosure, the molecular weight of the pneumococcal polysaccharides except for the polysaccharide derived from serotype 6A is in the range of 50 kDa to 600 kDa (SEC-HPLC), preferably in the range of 100 kDa to 200 kDa (SEC-HPLC).

In another embodiment, the molecular weight of the pneumococcal polysaccharide derived from serotype 6A is in the range of 400 kDa to 900 kDa (SEC-HPLC).

In another embodiment of the present disclosure, each pneumococcal polysaccharide present in the vaccine composition is individually conjugated to a carrier protein.

In accordance with the embodiments of the present disclosure, the pneumococcal polysaccharides are conjugated to one or more carrier proteins selected from the group comprising CRM₁₉₇, diphtheria toxoid (DT), tetanus toxoid (TT), *Neisseria meningitidis* outer membrane complex, fragment C of tetanus toxoid, recombinant full-length tetanus toxin with eight individual amino acid mutations (8MTT), diphtheria toxin fragment B (DTFB), pertussis toxoid, protein D of *H. influenzae, E. coli* LT, *E. coli* ST, exotoxin A from *Pseudomonas aeruginosa,* outer membrane complex c (OMPC), porins, fHbp, Por A, Por B, transferrin binding proteins, pneumolysin, pneumococcal surface protein A (PspA), pneumococcal surface adhesin A (PsaA), PhtA, PhtB, PhtE, pneumococcal PhtD, pneumococcal surface proteins BVH-3 and BVH-11, M. catarrhalis uspA, protective antigen (PA) of *Bacillus anthracis* and detoxified edema factor (EF) and lethal factor (LF) of *Bacillus anthracis,* ovalbumin, keyhole limpet hemocyanin (KLH), C5a peptidase group A or group B *Streptococcus,* human serum albumin, bovine serum albumin (BSA), purified protein derivative of tuberculin (PPD), Cholera toxin subunit B, synthetic peptides, heat shock proteins, pertussis proteins, cytokines, lymphokines, hormones, growth factors, artificial proteins comprising multiple human CD4+ T cell epitopes from various pathogen-derived antigens such as N 19, iron-uptake proteins, toxin A or B from *C*. *difficile* and *S*. agalactiae proteins and any equivalents thereof.

In accordance with the embodiments of the present disclosure, each pneumococcal polysaccharide-carrier protein conjugate has a molecular weight in the range of 1500 kDa to 30000 kDa (SEC-MALS).

In an embodiment, the molecular weight of 6A-TT conjugate in the vaccine composition is in the range of 7000 kDa to 10000 kDa (SEC-MALS), preferably in the range of 8000 kDa to 9000 kDa (SEC-MALS).

In an embodiment, the molecular weight of 4-DT conjugate in the vaccine composition is in the range of 3000 kDa to 10000 kDa (SEC-MALS), preferably in the range of 3000 kDa to 9000 kDa (SEC-MALS).

In an embodiment, the molecular weight of 3-TT conjugate in the vaccine composition is in the range of 3000 kDa to 10000 kDa (SEC-MALS), preferably in the range of 4000 kDa to 10000 kDa (SEC-MALS).

In an embodiment, the molecular weight of 24F-DT conjugate in the vaccine composition is in the range of 1500 kDa to 10000 kDa (SEC-MALS), preferably in the range of 2000 kDa to 6000 kDa (SEC-MALS).

In an embodiment, the molecular weight of 6B-CRM197 conjugate in the vaccine composition is in the range of 4000 kDa to 15000 kDa (SEC-MALS), preferably in the range of 5000 kDa to 12000 kDa (SEC-MALS).

In an embodiment, the molecular weight of 9V-TT conjugate in the vaccine composition is in the range of 15000 kDa to 30000 kDa (SEC-MALS), preferably in the range of 20000 kDa to 30000 kDa (SEC-MALS).

In an embodiment, the molecular weight of 15B-TT conjugate in the vaccine composition is in the range of 6000 kDa to 10000 kDa (SEC-MALS), preferably in the range of 7500 kDa to 9000 kDa (SEC-MALS).

In an embodiment, the molecular weight of 19A-TT conjugate in the vaccine composition is in the range of 5000 kDa to 8000 kDa (SEC-MALS), preferably in the range of 6000 kDa to 7000 kDa (SEC-MALS).

Typically, the molecular weight of 22F-TT conjugate in the vaccine composition of the present disclosure is > 12500 kDa (SEC-MALS). In an embodiment, the molecular weight of 22F-TT conjugate in the vaccine composition is in the range of 9000 kDa to 18000 kDa (SEC-MALS), preferably in the range of 10000 kDa to 17000 kDa (SEC-MALS).

The vaccine composition of the present disclosure comprises use of more than one type of carrier protein, individually conjugated to specific pneumococcal polysaccharides.

Conjugate vaccines containing same carrier protein are known to be associated with suppression of the immune response to polysaccharides. This may be due to (i) competition for antigen capture and presentation between B cells with surface immunoglobulins specific for epitopes on the carrier protein and B cells specific for the polysaccharide, (ii) prevention of the binding of the conjugate vaccines to polysaccharide-specific B cells by the free protein carrier, and (iii) suppression of the response to polysaccharides by expansion of the number of carrier-specific B cells induced by previous injection of the carrier protein which prevents the polysaccharide binding with the B cells.

The vaccine composition of the present disclosure uses multiple carrier proteins, which overcomes the hereinabove mentioned drawbacks associated with use of single type of carrier proteins in conjugate vaccine composition.

In an embodiment of the present disclosure, the pneumococcal polysaccharides are conjugated to three different carrier proteins. In an exemplary embodiment of the present disclosure, the three different carrier proteins are diphtheria toxoid (DT), tetanus toxoid (TT) and CRM197.

In accordance with the embodiments of the present disclosure, at least one pneumococcal polysaccharide is conjugated to CRM197 as the carrier protein, at least one pneumococcal polysaccharide selected from serotypes 3, 6A, 9V, 15B, 19A, 22F is conjugated to TT as the carrier protein and at least one pneumococcal polysaccharide is conjugated to DT as the carrier protein.

In accordance with the embodiments of the present disclosure, at least one pneumococcal polysaccharide is conjugated to CRM197 as the carrier protein, at least one pneumococcal polysaccharide selected from serotypes 3, 6A, 9V, 15B, 19A, 22F is conjugated to TT as the carrier protein and at least one pneumococcal polysaccharide selected from serotypes 4 and 24F is conjugated to DT as the carrier protein.

In accordance with the embodiments of the present disclosure, at least one pneumococcal polysaccharide is conjugated to CRM197 as the carrier protein, at least two pneumococcal polysaccharides selected from serotypes 3, 6A, 9V, 15B, 19A, 22F is conjugated to TT as the carrier protein and at least one pneumococcal polysaccharide is conjugated to DT as the carrier protein.

In another embodiment of the present disclosure, at least three pneumococcal polysaccharides selected from serotypes 3, 6A, 9V, 15B, 19A, 22F are conjugated to TT, one pneumococcal polysaccharide is conjugated to DT and remaining pneumococcal polysaccharides are conjugated to CRM197.

In still another embodiment of the present disclosure, at least four pneumococcal polysaccharides selected from serotypes 3, 6A, 9V, 15B, 19A, 22F are conjugated to TT, one pneumococcal polysaccharide is conjugated to DT, and remaining pneumococcal polysaccharides are conjugated to CRM197.

In an embodiment of the present disclosure, at least five pneumococcal polysaccharides selected from serotypes 3, 6A, 9V, 15B, 19A, 22F are conjugated to TT, one pneumococcal polysaccharide is conjugated to DT, and remaining pneumococcal polysaccharides are conjugated to CRM197.

In another embodiment of the present disclosure, six pneumococcal polysaccharides derived from serotypes 3, 6A, 9V, 15B, 19A, 22F are conjugated to TT, one pneumococcal polysaccharide is conjugated to DT, and remaining pneumococcal polysaccharides are conjugated to CRM197.

In still another embodiment of the present disclosure, six pneumococcal polysaccharides are conjugated to TT, two pneumococcal polysaccharides are conjugated to DT, and remaining pneumococcal polysaccharides are conjugated to CRM197.

In an embodiment of the present disclosure, five pneumococcal polysaccharides are independently conjugated to TT, two pneumococcal polysaccharides are independently conjugated to DT, and remaining pneumococcal polysaccharides are independently conjugated to CRM197.

In accordance with the embodiments of the present disclosure, the pneumococcal polysaccharide conjugated to CRM197 is selected from the group comprising 1, 2, 3, 4, 5, 6, 6A, 6B, 6C, 6D, 6E, 6F, 6G, 6H, 7A, 7B, 7C, 7D, 7F, 8, 9A, 9L, 9F, 9N, 9V, 10A, 10B, 10C, 10D, 10F, 11, 11A, 11B, 11C, 11D, 11E, 11F, 12A, 12B, 12F, 13, 13F, 14, 15A, 15B, 15C, 15F, 16, 16A, 16F, 17A, 17F, 18, 18A, 18B, 18C, 18F, 19A, 19B, 19C, 19F, 20, 20A, 20B, 21, 22A, 22F, 23A, 23B, 23F, 24A, 24B, 24F, 25A, 25F, 27, 28A, 28F, 29, 31, 32A, 32F, 33A, 33B, 33C, 33D, 33E, 33F, 34, 35A, 35B, 35C, 35D, 35F, 36, 37, 38, 39, 40, 41A, 41F, 42, 43, 44, 45, 46, 47A, 47F, and 48.

In an embodiment of the present disclosure, the pneumococcal polysaccharides conjugated to TT are selected from the group comprising 1, 3, 5, 6A, 9V, 15B, 19A, and 22F.

In another embodiment of the present disclosure, the pneumococcal polysaccharides conjugated to DT are selected from the group comprising 3, 4, 15B, and 24F.

In an embodiment, pneumococcal polysaccharide derived from serotype 1 is conjugated to CRM197 as the carrier protein.

In an embodiment, pneumococcal polysaccharide derived from serotype 3 is conjugated to TT as the carrier protein.

In an embodiment, pneumococcal polysaccharide derived from serotype 9V is conjugated to TT as the carrier protein.

In an embodiment, the pneumococcal polysaccharide derived from serotype 9A may be conjugated to a carrier protein selected from DT, TT, and CRM197.

In an embodiment, the pneumococcal polysaccharide derived from serotype 9L may be conjugated to a carrier protein selected from DT, TT, and CRM197.

In an embodiment, the pneumococcal polysaccharide derived from serotype 9N may be conjugated to a carrier protein selected from DT, TT, and CRM197.In an embodiment of the present disclosure, the pneumococcal polysaccharide derived from serotype 4 is conjugated to DT as the carrier protein. In an embodiment, pneumococcal polysaccharide derived from serotype 5 is conjugated to CRM197 as the carrier protein.

In another embodiment, pneumococcal polysaccharides derived from serotypes 1 and 5 are independently conjugated to CRM197 as the carrier protein; and pneumococcal polysaccharide derived from serotype 3 is conjugated to TT as the carrier protein.

In yet another embodiment, the vaccine composition comprises pneumococcal polysaccharides derived from serotypes 1 and 5 independently conjugated to CRM197 as the carrier protein and is devoid of *S*. *pneumoniae* polysaccharide derived from serotype 3. In an embodiment, pneumococcal polysaccharide derived from serotype 6A is conjugated to TT as the carrier protein. In an embodiment, pneumococcal polysaccharide derived from serotype 9V is conjugated to TT as the carrier protein. In an embodiment, pneumococcal polysaccharide derived from serotype 15B is conjugated to TT as the carrier protein. In an embodiment, pneumococcal polysaccharide derived from serotype 18C is conjugated to CRM197 as the carrier protein. In an embodiment, pneumococcal polysaccharide derived from serotype 19A is conjugated to TT as the carrier protein. In an embodiment, pneumococcal polysaccharide derived from serotype 19F is conjugated to CRM197 as the carrier protein. In an embodiment, pneumococcal polysaccharide derived from serotype 24F is conjugated to DT as the carrier protein. In an embodiment of the present disclosure, at least one pneumococcal polysaccharide derived from serotypes selected from the group comprising 3, 6A, 9V, 15B, 19A, and 22F is conjugated to tetanus toxoid as the carrier protein, at least one pneumococcal polysaccharide derived from serotypes 4 and 24F is conjugated to diphtheria toxoid as the carrier protein and at least one pneumococcal polysaccharide derived from serotype selected from the group comprising 1, 2, 3, 4, 5, 6, 6A, 6B, 6C, 6D, 6E, 6F, 6G, 6H, 7A, 7B, 7C,7D,7F, 8, 9A, 9L, 9F, 9N, 9V, 10A, 10B, 10C, 10D, 10F, 11, 11A, 11B, 11C, 11D, 11E, 11F, 12A, 12B, 12F, 13, 13F, 14, 15A, 15B, 15C, 15F, 16, 16A, 16F, 17A, 17F, 18, 18A, 18B, 18C, 18F, 19A, 19B, 19C, 19F, 20, 20A, 20B, 21, 22A, 22F, 23A, 23B, 23F, 24A, 24B, 24F, 25A, 25F, 27, 28A, 28F, 29, 31, 32A, 32F, 33A, 33B, 33C, 33D, 33E, 33F, 34, 35A, 35B, 35C, 35D, 35F, 36, 37, 38, 39, 40, 41A, 41F, 42, 43, 44, 45, 46, 47A, 47F, and 48 is conjugated to CRM197 as the carrier protein.

The vaccine composition of the present disclosure is devoid of Protein D, PsaA, pneumococcal surface protein A and Diphtheria toxin fragment as carrier protein(s) for the pneumococcal polysaccharides.

In an embodiment of the present disclosure, the vaccine composition comprises pneumococcal polysaccharide derived from serotype 2; and remaining pneumococcal polysaccharides are derived from serotypes selected from the group consisting of 1, 3, 4, 5, 6, 6A, 6B, 6C, 6D, 6E, 6F, 6G, 6H, 7A, 7B, 7C, 7D, 7F, 8, 9A, 9L, 9F, 9N, 9V, 10A, 10B, 10C, 10D, 10F, 11, 11A, 11B, 11C, 11D, 11E, 11F, 12A, 12B, 12F, 13, 13F, 14, 15A, 15B, 15C, 15F, 16, 16A, 16F, 17A, 17F, 18, 18A, 18B, 18C, 18F, 19A, 19B, 19C, 19F, 20, 20A, 20B, 21, 22A, 22F, 23A, 23B, 23F, 24A, 24B, 24F, 25A, 25F, 27, 28A, 28F, 29, 31, 32A, 32F, 33A, 33B, 33C, 33D, 33E, 33F, 34, 35A, 35B, 35C, 35D, 35F, 36, 37, 38, 39, 40, 41A, 41F, 42, 43, 44, 45, 46, 47A, 47F, and 48.

In an embodiment of the present disclosure, the vaccine composition comprises pneumococcal polysaccharides derived from serotypes 6A and 6B; and remaining pneumococcal polysaccharides are derived from serotypes selected from the group consisting of 1, 2, 3, 4, 5, 6, 6C, 6D, 6E, 6F, 6G, 6H, 7A, 7B, 7C, 7D, 7F, 8, 9A, 9L, 9F, 9N, 9V, 10A, 10B, 10C, 10D, 10F, 11, 11A, 11B, 11C, 11D, 11E, 11F, 12A, 12B, 12F, 13, 13F, 14, 15A, 15B, 15C, 15F, 16, 16A, 16F, 17A, 17F, 18, 18A, 18B, 18C, 18F, 19A, 19B, 19C, 19F, 20, 20A, 20B, 21, 22A, 22F, 23A, 23B, 23F, 24A, 24B, 24F, 25A, 25F, 27, 28A, 28F, 29, 31, 32A, 32F, 33A, 33B, 33C, 33D, 33E, 33F, 34, 35A, 35B, 35C, 35D, 35F, 36, 37, 38, 39, 40, 41A, 41F, 42, 43, 44, 45, 46, 47A, 47F, and 48.

In an embodiment of the present disclosure, pneumococcal polysaccharides derived from serotypes 6A and 6B are both conjugated to CRM197 as the carrier protein. In another embodiment of the present disclosure, pneumococcal polysaccharide derived from serotype 6A is conjugated to TT and pneumococcal polysaccharide derived from serotype 6B is conjugated to CRM197 as the carrier protein.

In accordance with the embodiments of the present disclosure, the vaccine compositions of the present invention may further comprise pneumococcal polysaccharides derived from serotypes 6C, 20B, 15A, 15C, 16F, 20A, 20B, 31, 9A, 9N, 9L, 35B, 23A, 23B, 17F, 29, 34, 39.

In accordance with the embodiments of the present disclosure, the vaccine composition is a 15, 16, 17, 18, 19, 20 21, 22, 23, 24, 25, 26, 27, 28, 29, or 30-valent pneumococcal polysaccharide-carrier protein conjugate composition.

In an embodiment, the vaccine composition is a 20-valent composition comprising 20 distinct *Streptococcus pneumoniae* polysaccharide-carrier protein conjugates having polysaccharides derived from serotypes 1, 2, 4, 5, 6A, 6B, 7F, 8, 9V, 10A, 11A, 12F, 14, 15B, 18C, 19A, 19F, 22F, 23F, and 33F.

In an embodiment, the vaccine composition is a 20-valent composition comprising 20 distinct *Streptococcus pneumoniae* polysaccharide-carrier protein conjugates having polysaccharides derived from serotypes 1, 2, 3, 4, 5, 6A, 6B, 7F, 8, 9V, 10A, 12F, 14, 15B, 18C, 19A, 19F, 22F, 23F, and 33F.

In an embodiment, the vaccine composition is a 20-valent composition comprising 20 distinct *Streptococcus pneumoniae* polysaccharide-carrier protein conjugates having polysaccharides derived from serotypes 1, 3, 4, 5, 6A, 6B, 7F, 8, 9V, 10A, 11A, 12F, 14, 15B, 18C, 19A, 19F, 22F, 23F, and 33F.

In an embodiment, the vaccine composition is a 21-valent composition comprising 21 distinct *Streptococcus pneumoniae* polysaccharide-carrier protein conjugates having polysaccharides derived from serotypes 1, 2, 4, 5, 6A, 6B, 7F, 8, 9V, 10A, 11A, 12F, 14, 15B, 18C, 19A, 19F, 22F, 23F, 24F and 33F.

In an embodiment, the vaccine composition is a 21-valent composition comprising 21 distinct *Streptococcus pneumoniae* polysaccharide-carrier protein conjugates having polysaccharides derived from serotypes 1, 2, 3, 4, 5, 6A, 6B, 7F, 8, 9V, 10A, 11A, 12F, 14, 15B, 18C, 19A, 19F, 22F, 23F and 33F.

In an embodiment, the vaccine composition is a 22-valent composition comprising 22 distinct *Streptococcus pneumoniae* polysaccharide-carrier protein conjugates having polysaccharides derived from serotypes 1, 2, 3, 4, 5, 6A, 6B, 7F, 8, 9V, 10A, 11A, 12F, 14, 15B, 18C, 19A, 19F, 22F, 23F, 24F and 33F.

In accordance with the embodiments of the present disclosure, the vaccine composition comprises 1.0 µg to 10 µg of polysaccharide of each pneumococcal serotype, preferably in the range of 2.2 µg to 3.3 µg. In another embodiment, the vaccine composition comprises 4.4 µg to 6.6 µg of the polysaccharide derived from serotype 6B.

The vaccine composition of the present disclosure comprises pharmaceutically acceptable excipients selected from surfactants, stabilizers, buffers, diluents, adjuvants, preservatives, salts and solvents.

In an embodiment, the adjuvant is an aluminium salt adjuvant selected from the group consisting of aluminum hydroxide, aluminum phosphate, aluminum hydroxyphosphate, and potassium aluminum sulfate. Typically, the concentration of the adjuvant is not more than 1.15 mg per 0.5 ml dose of the vaccine composition. In an embodiment, the concentration of the adjuvant is in the range of 0.5 mg to 1 mg per 0.5 ml dose of the vaccine composition. In accordance with the embodiments of the present disclosure, the concentration of adjuvant is not more than 1.13 mg per 0.5 ml dose of the vaccine composition. In a preferred embodiment, the concentration of the adjuvant is 0.847 mg per 0.5 ml dose of the vaccine composition.

In another embodiment, the adjuvant comprises 20 to 375 µg of Al³⁺ per 0.5 ml dose of the vaccine composition.

In an embodiment, the adjuvant is aluminum phosphate containing 170 to 200 µg of Al³⁺ per 0.5 ml dose of the vaccine composition.

In a preferred embodiment, the adjuvant comprises 187.5 µg of Al³⁺ per 0.5 ml dose of the vaccine composition.

The buffer is selected from the group comprising histidine, succinate, citrate, phosphate, tris-acetate- ethylenediaminetetraacetic acid (TAE), and 4-(2-hydroxyethyl)- 1 piperazineethanesulfonic acid (HEPES). The concentration of the buffer is in the range of 0.1 mg to 10 mg.

In an embodiment, the vaccine composition comprises histidine as the buffer.

In another embodiment, the vaccine composition comprises succinate as the buffer.

In still another embodiment, the vaccine composition comprises 0.1 mg to 10 mg histidine and 0.1 mg to 10 mg succinate as the buffer. In a preferred embodiment, the vaccine composition comprises 1.55 mg histidine and 1.18 mg succinate as the buffer.

The surfactant is selected from the group comprising polysorbate, polymer glycol, sorbitan ester, and combinations thereof and the concentration of the surfactant in the vaccine composition is in the range of 80 µg to 120 µg. In an embodiment of the present disclosure, the surfactant is polysorbate 20 (and not polysorbate 80). In a preferred embodiment, the concentration of the surfactant in the vaccine composition is 100 µg.

The HLB (hydrophilic-lipophilic balance) value for Polysorbate 20 is 16.7 and Polysorbate 80 has 15 HLB values. Polysorbate 20 is more hydrophilic in nature. The HLB value of 16.7 of Polysorbate 20 confers relatively stronger hydrophilic nature. This ensures its property as a good emulsifier and a stabilizer for aqueous suspension and emulsion systems by improving the wetting properties of particles in the formulation. Hence it is likely to be more suitable to stabilize alum suspension-based vaccine formulations especially pneumococcal vaccine. This aspect is more relevant when 22 monovalent conjugates are mixed in the formulation with the presence of alum gel and other excipients like histidine. Polysorbate 20 was found to be advantageous for multivalent pneumococcal vaccine formulations (PCV20 and PCV21) to ensure its stability. Polysorbate 20 protects vaccine formulation against aggregation caused by interface induced stresses. Such stresses (air /liquid or liquid to surfaces/container) occurred during the stages of production which may lead to undesired adsorption, aggregation or precipitation.

Polysorbate 80 is known to be used as a surfactant in vaccine composition. However, the vaccine composition of the present disclosure uses polysorbate 20 as the surfactant, which is different from polysorbate 80 as illustrated below.
(i) Polysorbate 20 used in the present disclosure is structurally different from polysorbate 80.

| |
|---|
| 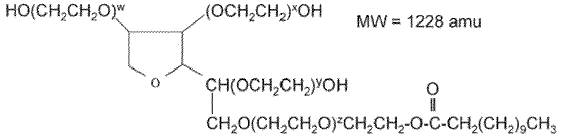 |
| Polysorbate 20 (Tween 20): polyoxyethylene sorbitan monolaurate |
| 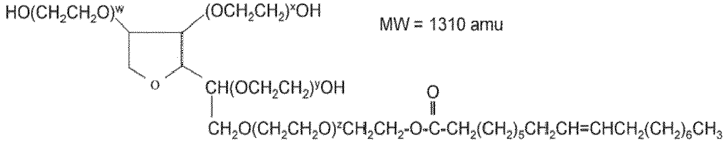 |
| Polysorbate 80 (Tween 80): polyoxyethylene sorbitan monooleate |
| *(w* + *x* + y + *z)* = *20* |

Chemical structure of polysorbates 20 and 80. w þ x þ y þ z refers to the total number of oxyethylene subunits on each surfactant molecule and may not exceed 20.
(ii) The fatty acid content of polysorbate 20 differs from that of polysorbate 80

### Fatty acid contents of polysorbate 20 and 80

| | EU Specifications | | |
|---|---|---|---|
| Acid | PS-20 (%)*^{a}* | PS-80 (%)*^{b}* | Structure |
| Caproic | ≤1 | | CH₃(CH₂)₄COOH |
| Caprylic | ≤10 | | CH₃(CH₂)₆COOH |
| Capric | ≤10 | | CH₃(CH₂)₈COOH |
| Lauric | 40-60 | | CH₃(CH₂)₁₀COOH |
| Myristic | 14-25 | ≤5 | CH₃(CH₂)₁₂COOH |
| Palmitic | 7-15 | ≤16 | CH₃(CH₂)₁₄COOH |
| Palmitoleic | | ≤8 | CH₃(CH₂)₅CH=CH(CH₂)₇COOH |
| Stearic | ≤7 | ≤6 | CH₃(CH₂)₁₆COOH |
| Oleic | ≤11 | ≥58 | CH₃(CH₂)₇CH=CH(CH₂)₇COOH |
| Linoleic | ≤3 | ≤18 | CH₃(CH₂)₃CH=CH(CH₂)₇COOH |
| Linolenic | | ≤4 | CH₃(CH₂)₄CH=CHCH₂CH=CH(CH₂)₇COOH |
| *^{a}*European Directorate for the Quality of Medicines and Healthcare. European Pharmacopoeia On-line. 5th Edition 2007, 01/ 2005:0426. | | | |
| *^{b}*European Directorate for the Quality of Medicines and Healthcare. European Pharmacopoeia On-line. 5th Edition 2007, 04/ 2006:0428. | | | |

Further, Polysorbate 20 is more hydrophilic as compared to Polysorbate 80 (PS-20 has HLB value 16.71, whereas PS-80 has HLB value 15; The higher surfactant HLB value, the more hydrophilic it is. The lower surfactant HLB value, the more lipophilic it is). The monolaurate fraction of polysorbate 20 makes up only 40-60% of the mixture. Whereas, the monooleate fraction of polysorbate 80 makes up >58% of the mixture. Polysorbate 20 has a molecular weight of 1,225 daltons. Polysorbate 80 has molecular weight of 1.31 kDa. Polysorbate 80 is more prone to generation of oxidative species compared with polysorbate 20 as a result of the greater content of unsaturated alkyl side chains in polysorbate 80.

The higher quantity of surfactant (polysorbate 20) in the vaccine composition of the present disclosure aids in obtaining an aggregation-free composition having improved flow properties even in the presence of increased alum gel content with higher number of antigens (pneumococcal polysaccharides) being included in the composition.

The salt is selected from the group comprising magnesium chloride, potassium chloride, sodium chloride and combinations thereof and the concentration of the salt in the vaccine composition is in the range of 2 mg to 10 mg per 0.5 ml dose of the vaccine composition. In an embodiment, the salt is sodium chloride. In a preferred embodiment, the concentration of the salt in the vaccine composition is 4.5 mg per 0.5 ml dose of the vaccine composition.

In an embodiment, the vaccine composition is in the form of a single-dose composition and is free of preservative.

In another embodiment, the vaccine composition is in the form of a multi-dose composition and comprises a preservative selected from the group comprising 2-phenoxyethanol, benzethonium chloride (Phemerol), phenol, m-cresol, thiomersal, formaldehyde, methyl paraben, propyl paraben, benzalkonium chloride, benzyl alcohol, chlorobutanol, p-chlor-m-cresol, benzyl alcohol, and combinations thereof.

In an embodiment, the concentration of preservative is in the range of 1 mg to 8 mg per 0.5 ml dose of the vaccine composition.

In another embodiment, the concentration of the preservative is in the range of 2.5 mg to 5 mg per 0.5 ml dose of the vaccine composition.

In still another embodiment, the preservative is 2-phenoxyethanol in a concentration in the range of 2.5 mg to 5 mg per 0.5 ml dose of the vaccine composition.

Typically, the pH of the vaccine composition is in the range of 5 to 7, preferably in the range of 5 to 6.

In an embodiment, the vaccine composition is a 20-valent composition and is formulated as a 0.5 ml dose comprising:
a. 20 distinct *S*. *pneumoniae* polysaccharides derived from serotypes 1, 2, 4, 5, 6A, 6B, 7F, 8, 9V, 10A, 11A, 12F, 14, 15B, 18C, 19A, 19F, 22F, 23F, and 33F;
b. aluminium phosphate as adjuvant containing 170 µg to 200 µg of Al³⁺;
c. histidine at a concentration of 1 mg to 2 mg;
d. succinate at a concentration of 1 mg to 2 mg;
e. polysorbate 20 at a concentration of 80 µg to 120 µg;
f. sodium chloride at a concentration of 2 mg to 10 mg; and
g. pH in the range of 5 to 7;
   wherein
   i. *S*. *pneumoniae* polysaccharide derived from serotype 4 is conjugated to DT as carrier protein; *S. pneumoniae* polysaccharides derived from serotypes 15B, 6A, 9V, 19A, and 22F are conjugated to TT as carrier protein; and *S*. *pneumoniae* polysaccharides derived from serotypes 1, 2, 5, 6B, 7F, 8, 10A, 11A, 12F, 14, 18C, 19F, 23F and 33F are conjugated to CRM197 as the carrier protein;
   ii. 2.2 µg to 3.3 µg of each *S. pneumoniae* polysaccharide except for serotype 6B;
   iii. 4.4 µg to 6.6 µg *S*. *pneumoniae* polysaccharide from serotype 6B;
   iv. 10 µg to 200 µg of CRM197
   v. 5 µg to 70 µg of tetanus toxoid; and
   vi. 1 µg to 25 µg of diphtheria toxoid.

In an embodiment, the vaccine composition is a 21 valent composition and is formulated as a 0.5 ml dose comprising:
a. 21 distinct *S. pneumoniae* polysaccharides derived from serotypes 1, 2, 3, 4, 5, 6A, 6B, 7F, 8, 9V, 10A, 11A, 12F, 14, 15B, 18C, 19A, 19F, 22F, 23F, and 33F;
b. aluminium phosphate as adjuvant containing 170 - 200 of Al³⁺;
c. histidine at a concentration of 1 to 2 mg;
d. succinate at a concentration of 1 to 2 mg;
e. polysorbate 20 at a concentration 80 to 120 µg;
f. sodium chloride at a concentration 2 mg to 10 mg; and
g. pH in the range of 5 to 7;
wherein
i. the *S. pneumoniae* polysaccharide derived from serotype 4 is conjugated to DT as carrier protein; *S. pneumoniae* polysaccharides derived from serotypes 6A, 9V, 15B, 19A, and 22F are conjugated to TT as carrier protein; and remaining *S. pneumoniae* polysaccharides are conjugated to CRM197 as the carrier protein; The vaccine composition comprises
ii. 2.2 to 3.3 µg of each S. pneumoniae polysaccharide except for serotype 6B;
iii. 4.4 to 6.6 µg S. pneumoniae polysaccharide from serotype 6B;
iv. 10 µg to 200 µg of CRM197
v. 5 µg to 70 µg of tetanus toxoid; and
vi. 1 µg to 25 µg of diphtheria toxoid.

In an embodiment, the vaccine composition is a 21 valent composition and is formulated as a 0.5 ml dose comprising:
a. 21 distinct *S*. *pneumoniae* polysaccharides derived from serotypes 1, 2, 4, 5, 6A, 6B, 7F, 8, 9V, 10A, 11A, 12F, 14, 15B, 18C, 19A, 19F, 22F, 23F, 24F and 33F;
a. aluminium phosphate as adjuvant containing 170 µg to 200 µg of Al³⁺;
b. histidine at a concentration of 1 mg to 2 mg;
c. succinate at a concentration of 1 mg to 2mg;
d. polysorbate 20 at a concentration of 80 µg to 120 µg;
e. sodium chloride at a concentration of 2 mg to 10 mg; and
f. pH in the range of 5 to 7;
   wherein
   i. *S*. *pneumoniae* polysaccharide derived from serotypes 4, and 24F are conjugated to DT as carrier protein; *S. pneumoniae* polysaccharides derived from serotypes 15B, 6A, 9V, 19A, and 22F are conjugated to TT as carrier protein; and *S. pneumoniae* polysaccharides derived from serotypes 1, 2, 5, 6B, 7F, 8, 10A, 11A, 12F, 14, 18C, 19F, 23F and 33F are conjugated to CRM197 as the carrier protein;
   ii. 2.2µg to 3.3 µg of each *S. pneumoniae* polysaccharide except for serotype 6B;
   iii. 4.4 µg to 6.6 µg *S*. *pneumoniae* polysaccharide from serotype 6B;
   iv. 10 µg to 200 µg of CRM197
   v. 5 µg to 70 µg of tetanus toxoid; and
   vi. 1 µg to 25 µg of diphtheria toxoid.

In an embodiment, the vaccine composition is a 22 valent composition and is formulated as a 0.5 ml dose comprising:
a. 22 distinct *S. pneumoniae* polysaccharides derived from serotypes 1, 2, 3, 4, 5, 6A, 6B, 7F, 8, 9V, 10A, 11A, 12F, 14, 15B, 18C, 19A, 19F, 22F, 23F, 24F and 33F;
a. aluminium phosphate as adjuvant containing 170 µg to 200 µg of Al³⁺;
b. histidine at a concentration of 1 mg to 2 mg;
c. succinate at a concentration of 1 mg to 2mg;
d. polysorbate 20 at a concentration of 80 µg to 120 µg;
e. sodium chloride at a concentration of 2 mg to 10 mg; and
f. pH in the range of 5 to 7;
   wherein
   i. *S*. *pneumoniae* polysaccharide derived from serotypes 4, and 24F are conjugated to DT as carrier protein; *S. pneumoniae* polysaccharides derived from serotypes 3, 15B, 6A, 9V, 19A, and 22F are conjugated to TT as carrier protein; and *S. pneumoniae* polysaccharides derived from serotypes 1, 2, 5, 6B, 7F, 8, 10A, 11A, 12F, 14, 18C, 19F, 23F and 33F are conjugated to CRM197 as the carrier protein;
   ii. 2.2µg to 3.3 µg of each *S. pneumoniae* polysaccharide except for serotype 6B;
   iii. 4.4 µg to 6.6 µg *S*. *pneumoniae* polysaccharide from serotype 6B;
   iv. 10 µg to 200 µg of CRM197
   v. 5 µg to 70 µg of tetanus toxoid; and
   vi. 1 µg to 25 µg of diphtheria toxoid.

In an embodiment, the *S*. *pneumoniae* polysaccharide derived from serotype 15B is conjugated to DT as carrier protein. In another embodiment, the *S. pneumoniae* polysaccharide derived from serotype 15B may be conjugated to CRM197 as carrier protein.

In an embodiment, the vaccine composition may comprise at least one *S. pneumoniae* polysaccharide derived from serotypes selected from 9A, 9N, and 9L.

In an embodiment, the vaccine composition may comprise *S. pneumoniae* polysaccharide derived from serotype 3.

In another embodiment, the vaccine composition is devoid of *S*. *pneumoniae* polysaccharide derived from serotype 3.

In another embodiment, the 21 valent vaccine composition comprises 8 µg to 45 µg of tetanus toxoid per 0.5 ml dose of the composition. In still another embodiment, the vaccine composition comprises 1 µg to 15 µg of diphtheria toxoid per 0.5 ml dose of the composition. In an embodiment the vaccine composition comprises 1 µg to 8 µg of diphtheria toxoid per 0.5 ml of the composition.

The vaccine composition of the present disclosure as described above when formulated as a multi-dose composition, comprises 2-phenoxyethanol in a concentration in the range of 2.5 mg to 5 mg per 0.5 ml dose of the vaccine composition.

The vaccine composition of the present disclosure vaccine composition is stable at 2-8 °C, 25 °C and 40°C. In accordance with the embodiments of the present disclosure, the vaccine composition is stable at 2-8 °C, and 25 °C for a period of at least 3 months.

In accordance with the embodiments of the present disclosure, vaccine composition the pneumococcal polysaccharide-carrier protein conjugates have free polysaccharide less than 15 %, and free protein less than 10 %.

In an embodiment, the vaccine composition may optionally comprise of excipients, such as mannitol, glycine, lactose, trehalose, sucrose, raffinose, polymers (carboxy methyl cellulose, poloxamer, PEG, and the like).

The vaccine composition of the present disclosure is formulated for administration to a human subject for prophylaxis, reducing the onset of or preventing infections caused by *Streptococcus pneumoniae* by administration of an effective amount of the vaccine composition.

In accordance with the embodiments of the present disclosure, the human subject is selected from an infant (< 1 year of age), a toddler (around 12 months to 24 months of age), a young child (around 2 years to 5 years of age), an older child (around 5 years to 13 years of age), an adolescent (around 13 years to 18 years of age), an adult (around 18 years to 65 years of age), or an elder (>65 years of age).

In an embodiment, the amount of pneumococcal polysaccharide-carrier protein conjugate in a dose of the vaccine composition of the present disclosure is present in an amount sufficient to induce an immunoprotective response, without significant, adverse effects. Though, the amount of each pneumococcal polysaccharide-carrier protein conjugate may vary depending upon the pneumococcal serotype, each dose of the vaccine compositions comprises 1.0 µg to 10 µg of each pneumococcal polysaccharide conjugated to each carrier protein.

In an embodiment, there is provided a method for inducing an immune response in a human subject to a *Streptococcus pneumoniae* polysaccharide-carrier protein conjugate, by administering an immunogenically effective amount of the vaccine composition of the present disclosure. The vaccine composition described hereinabove may be administered to a subject in need by any of the conventional routes used in the field of vaccines including, but not limited to, oral route, mucosal route, nasal route, intranasal route, and through needle injections (intramuscular, intradermal, subcutaneous, intravenous, etc).

As used herein, an "effective amount" of the compositions described in the present disclosure refers to an amount required to elicit an immune response in the subject to which the vaccine composition is administered. The immune response is characterized by the presence of one or more *Streptococcus pneumoniae* antigen-specific antibodies in the subject that significantly reduce the likelihood or severity of infection of *Streptococcus pneumoniae* during a subsequent challenge.

In an embodiment of the present disclosure, there is at least 2-fold increase in total IgG titer and functional IgG titer when serotypes 3, 6A, 9V, 19A, and 15B are conjugated to TT as carrier protein as compared to when serotypes 3, 6A, 9V, 19A, and 15B are conjugated to CRM197 as carrier protein.

In accordance with the embodiments of the present disclosure, the pneumococcal polysaccharides derived from serotypes 3, 6A, 9V and 19A show enhanced IgG titers when they are conjugated to tetanus toxoid (TT) as carrier protein as compared to when pneumococcal polysaccharides derived from serotypes 3, 6A, 9V and 19A are conjugated to CRM197 as the carrier protein.

Further, the pneumococcal polysaccharides derived from serotypes 6A, 6B and 19A, 19F elicit cross reactivity (possibly from 6A and 19A, respectively) when the pneumococcal polysaccharides derived from serotypes 4, 24F are conjugated to DT as carrier protein; pneumococcal polysaccharides derived from serotypes 3, 15B, 6A, 9V, 19A, and 22F are conjugated to TT as carrier protein; and pneumococcal polysaccharides derived from serotypes 1, 2, 5, 6B, 7F, 8, 10A, 11A, 12F, 14, 18C, 19F, 23F and 33F are conjugated to CRM197 as the carrier protein.

The immunogenicity studies of the vaccine composition of the present disclosure in mice and rabbit shows an improvement in immune response for serotype 6B, which is likely due to cross-reactivity from 6A-TT. Further, vaccine compositions having serotypes 3, 6A, 9V, 15B and 19A conjugated to TT as carrier protein instead of CRM197 as carrier protein, elicited better immune response (IgG titers). Similar trend is also observed in opsonization assay (OPA) response also.

Vaccine compositions having serotype 4 conjugated to DT as carrier protein (as compared to CRM197 as carrier protein), elicited better immune response (IgG titers).

Similar results are also expected, when the vaccine composition of the present disclosure is devoid of *S*. *pneumoniae* polysaccharide derived from serotype 3.

The present disclosure provides vaccine compositions and methods for preparing the same. The vaccine compositions of the present disclosure comprise one or more pneumococcal polysaccharide-carrier protein conjugates, wherein each of the conjugate contains a polysaccharide from a different serotype of *Streptococcus pneumoniae* conjugated to a carrier protein. The vaccine compositions comprise more than one type of carrier protein and are individually conjugated to the different polysaccharides. The vaccine compositions further comprise optimum concentration of histidine and succinate as buffer, aluminium salt adjuvant, polysorbate 20 and sodium chloride as excipient. A preservative is added to the composition in case of multi-dose formulation. Further, the vaccine compositions have been prepared using a two-blend approach, wherein some of the serotypes are adsorbed together as a first set and remaining serotypes are adsorbed together as second set, followed by mixing of both sets to get the vaccine composition. The vaccine compositions of the present disclosure are devoid of aggregation; show long-term stability across wide temperature ranges thus preserving the desired characteristics of the multiple pneumococcal conjugates and provide protection against the most prevalent pneumococcal serotypes.

Present disclosure 1) uses increased alum gel quantity from 125 µg to 187.5 µg per dose resulting in improved adsorption (at least 30 % for all serotypes) (at least 80 % for serotypes 1, 2, 3, 5, 7F, 8, 9V, 10A, 12F, 14, 18C, 19A, 19F, 23F, 33F) and thereby providing increased B cell mediated IgG antibody response (IgG titers of at least 2 fold higher were obtained for serotypes 1, 10A, 15B and 22F); 2) higher quantity of polysorbate 20 in the multivalent pneumococcal conjugate vaccines have resulted in aggregation-free formulation, improved flow properties (especially in the presence of increased gel content with higher number of antigens being added); 3) novel improved, stable and immunogenic multivalent polysaccharide-protein conjugate vaccine composition (preferably 20 valent or 21 valent) having conjugates prepared by utilizing CDAP comprising of three different carrier proteins (DT, TT, CRM197), wherein serotypes 4 and 24F are individually conjugated to DT; serotypes 6A, 9V, 15B, 19A, 22F are individually conjugated to TT, rest of the polysaccharides from serotypes (1, 2, 5, 6B, 7F, 8, 10A, 11A, 12F, 14, 18C, 19F, 23F, 33F) are individually conjugated to CRM197; 4) all CRM197 and DT based conjugates are prepared by activating with 1-cyano-4-Dimethyl amino pyridinium tetrafluoroborate (CDAP) using direct coupling approaches utilizing cyanylation chemistry whereas all TT based conjugates are obtained by first preparing ADH derivatized TT (in presence of EDAC) followed by conjugating it with CDAP activated polysaccharides; 5) optimum molecular weight of sized Polysaccharide of serotype 4 is in the range of 130 kDa to 170 kDa (SEC-HPLC), serotype 24F is in the range of 100 kDa to 200 kDa (SEC-HPLC), serotype 3 is in the range of 140 kDa to 180 kDa (SEC-HPLC), serotype 6A is in the range of 400 kDa to 900 kDa (SEC-HPLC), serotype 6B is in the range of 100 kDa to 200 kDa (SEC-HPLC), serotype 9V is in the range of 120 kDa to 160 kDa (SEC-HPLC), serotype 15B is in the range of 120 kDa to 160 kDa (SEC-HPLC), serotype 19A-120 kDa to 160 kDa (SEC-HPLC), 22F is in the range of 100 kDa to 150 kDa (SEC-HPLC)) and respectively optimum molecular weight of polysaccharide-protein conjugates of serotype 4 is in the range of 3000 kDa to 10000 kDa (SEC-MALS), serotype 24F is in the range of 1500 kDa to 10000 kDa (SEC-MALS), serotype 3 is in the range of 3000 kDa to 10000 kDa (SEC-MALS), serotype 6A is in the range of 7000 kDa to 10000 kDa (SEC-MALS), serotype 6B is in the range of 4000 kDa to 15000 kDa (SEC-MALS), serotype 9V is in the range of 15000 kDa to 30000 kDa (SEC-MALS), serotype 15B is in the range of 6000 kDa to 10000 kDa (SEC-MALS), serotype 19A is in the range of 5000 kDa to 8000 kDa (SEC-MALS), serotype 22F is in the range of 9000 kDa to 18000 kDa (SEC-MALS) thereby ensuring improved immunogenicity (as compared to when these polysaccharides are conjugated to CRM197); 6) high conjugation efficiency (40 % to 45 % for serotypes 4 and 24F, 30 % to 35 % for serotype 3, 40 % to 45 % for serotype 6A, 25 % to 30 % for serotype 9V, 45 % to 55 % for serotype 15B, 30 % to 35 % for serotype 19A, 40 % to 45 % for serotype 22F); 7) optimum adsorption for conjugates (at least 30 %); 8) free polysaccharide content <15 % for all conjugates and free protein (<10 %); and 9) high polysaccharide recovery and structural integrity maintained by utilizing high pressure homogenization (as against chemical sizing).

Further, the vaccine compositions have been prepared using a two-blend approach, wherein some of the serotypes are adsorbed individually as a first set and remaining serotypes are adsorbed together as a second set, followed by mixing of both sets to get the vaccine composition.

Further the formulation of the present invention can be filled into siliconized or non-siliconized containers.

### EXAMPLES

The foregoing description of the embodiments has been provided for purposes of illustration and not intended to limit the scope of the present disclosure. Individual components of a particular embodiment are generally not limited to that particular embodiment, but, are interchangeable.

Such variations are not to be regarded as a departure from the present disclosure, and all such modifications are considered to be within the scope of the present disclosure.

The present disclosure is further described in light of the following examples which are set forth for illustration purpose only and not to be construed for limiting the scope of the disclosure.

### Source of biological resources used in the present disclosure

*Streptococcus pneumoniae* serotypes, namely, 1, 2, 3, 4, 5, 6A, 6B, 7F, 8, 9V, 10A, 11A, 12F, 14, 15B, 18C, 19A, 19F, 22F, 23F, and 33F, were sourced from Centers for Disease Control and Prevention (CDC), Atlanta, USA. *Streptococcus pneumoniae* serotype 24F was obtained from Kempegowda Institute of Medical Sciences (KIMS), Bangalore, India.

### Carrier proteins:

Diphtheria Toxoid (DT): The strain Corynebacterium diphtheriae PW8 CN2000 was obtained from the Wellcome Research Laboratory, London, United Kingdom by the National Control Authority Central Research Institute (C.R.I.) Kasauli, Himachal Pradesh, India in lyophilized form in the year 1973.The strain was revived and further lyophilized under Master Seed Lot-C. diphtheriae CN2000 A1 at C.R.I. Kasauli.

### Tetanus Toxoid (TT): The strain Clostridium tetani Harvard Strain No.49205 was obtained from The Rijks Institute Voor de Volksgezondheid (Netherlands) by the National Control Authority C.R.I. Kasauli, in Lyophilized form.

CRM197: CRM197 is obtained from Recombinant Strain CS463-003 (MB101) of *Pseudomonas fluorescens* procured from Pfenex USA.

### Example-1: Preparation of multivalent vaccine composition using three different carrier proteins

### (i) Fermentation:

Inoculum of the pneumococcal serotypes (for 21-valent vaccine composition, following pneumococcal serotypes were used: 1, 2, 4, 5, 6A, 6B, 7F, 8, 9V, 10A, 11A, 12F, 14, 15B, 18C, 19A, 19F, 22F, 23F, 24F and 33F) were grown at pH 7.1 ± 0.5, at 35-38°C under stirring at 40-100 RPM with an air flow rate of 0-0.5 vvm. The rate of feed medium addition was equivalent to the rate of alkali mixture addition for maintaining a preset pH (7.1). Manganese sulphate was used for viscosity reduction. (When serotype 3 was used, feed medium was not used).

### (ii) Purification:

The clarified fermentation broth of the pneumococcal serotypes obtained in the above step were concentrated and diafiltered using a 100 KDa MWCO membrane. Diafiltration was accomplished using 154 mM NaCl followed by diafiltration with water for injection (WFI).

The concentrated and diafiltered broth containing polysaccharide was subjected to enzyme treatment (nuclease) at 37 °C, for 10 ± 2 hours with stirring.

Ammonium sulphate was added to the nuclease treated polysaccharide solution to 50% saturation and incubated at 2 to 8 °C for 12±2 hours (except serotype 5). The mixture was subjected to depth filtration to remove the precipitate, which was discarded. The solution was subjected to 100 kDa diafiltration using NaCl followed by chilled WFI. This diafiltered solution containing polysaccharide with a buffer and high salt concentration was loaded on hydrophobic interaction chromatography (HIC) column.

The hydrophobic interaction chromatography column (300 ml) was equilibrated with 5 M NaCl in phosphate buffer at pH 6.8 and the polysaccharide solution (500 ml) was then loaded onto the column in pH range 6 to 8, preferably at pH 6 to 7. The column was further washed with 5 M NaCl in phosphate buffer solution at pH 6.8. Under these conditions, the polysaccharide was recovered in the flow-through and equilibration wash from the column. The polysaccharide solution was then concentrated using a 100 kDa MWCO filter and then diafiltered with NaCl and Water for Injection (WFI).

The ion exchange chromatography column (300 ml) (strong anion exchanger) was equilibrated with 50 mM sodium phosphate buffer and the polysaccharide solution (500 ml) was then loaded onto the column in pH range 6 to 8, preferably at pH 6.5 to 7.5 pH. The column was further washed with same buffer. The adsorbed polysaccharides were eluted with step gradient elution using 1.0 M NaCl (various polysaccharides were eluted at different ionic strengths of NaCl).

The polysaccharide solution was then concentrated using a 100 kDa MWCO filter and then diafiltered with Water for Injection (WFI).

The diafiltered polysaccharide solution was filtered through a 0.22 µ membrane filter into polypropylene bottles. The purified polysaccharide was stored frozen. This process was followed for serotypes 1, 6A, 6B, 7F, 9V, 14, 19A, 19F and 23F.

For serotypes 2, 4, 8, 10A, 11A, 12F, 15B, 18C, 22F, 24F and 33F, only HIC method was used. IEC chromatography was not used for these serotypes. Instead of ammonium sulphate precipitation, pH precipitation was used and thereafter diafiltration with WFI method was used for all these serotypes. Only HIC method was used, if serotype 3 was used in the vaccine composition.

Process condition for pH precipitation for these serotypes is:
pH: 5.8±0.2 pH using 20 % HCl, Time: 6±2 hours, temperature: 7±2 °C.

### (iii) Sizing:

A high-pressure homogenizer (Microfluidics Inc., Model M-110EH-30) apparatus was used to reduce the molecular weight of the polysaccharides before the activation step except for polysaccharide from serotype 6A. The size reduction was done at 20-30 KPSI (kilo-pounds per-square-inch) and 3 to 30 passes were used. The KPSI and number of passes (Table-1) varied depending on the serotype from which the polysaccharide was derived. Table-1 provides the pressure used and number of passes for each serotype used in the composition of the present disclosure.

| **Table-1: Pressure and passes for high pressure homogenization** | | | |
|---|---|---|---|
| **S. No.** | **Serotype** | **Pressure (KPSI)** | **Number of passes** |
| 1 | 1 | 26-30 | 7-10 |
| 2 | 5 | 26-30 | 20-25 |
| 3 | 6A | 3 - 8 | 1 * |
| 4 | 6B | 23-27 | 1-3 |
| 5 | 7F | 25-29 | 1-3 |
| 6 | 9V | 23-27 | 2-4 |
| 7 | 14 | 20-26 | 1-3 |
| 8 | 19A | 26-30 | 1-3 |
| 9 | 19F | 21-25 | 1-3 |
| 10 | 23F | 26-30 | 3-6 |
| 11 | 2 | 28 - 30 | 5-8 |
| 12 | 3 | 28 - 30 | 9-12 |
| 13 | 4 | 28 - 30 | 10-14 |
| 14 | 8 | 28 - 30 | 9-12 |
| 15 | 10A | 25 - 29 | 2-5 |
| 16 | 11A | 25-29 | 3-6 |
| 17 | 12F | 28-30 | 25-30 |
| 18 | 15B | 27-30 | 2-4 |
| 19 | 18C | 28-30 | 5-8 |
| 20 | 22F | 25-30 | 3-6 |
| 21 | 24F | 28-30 | 15-25 |
| 22 | 33F | 20-25 | 3-6 |
| * If Molecular Weight (SEC-HP-RI) is above 900 kDa | | | |

### (iv) Conjugation:

General conjugation process for all serotypes: The conjugation parameters in accordance with an embodiment of the present disclosure are provided in Table-2 and the general process flow is provided in Figure-1.

Purified and homogenized polysaccharides having molecular weight of 100 kDa to 200 kDa (except for purified serotype 6A polysaccharide which is used in native form having molecular weight of 400 kDa to 900 kDa) was taken for conjugation. The starting polysaccharide concentration was adjusted before concentration. Different polysaccharide concentration is used for different serotypes as shown in Table-2. For polysaccharide activation by 1-cyano-4-dimethylaminopyridine tetrafluoroborate (CDAP), freshly prepared CDAP chemical solution (-100 mg/ml in acetonitrile) was added as per the polysaccharide: CDAP ratio mentioned in Table-1 with mixing by magnetic stirrer (110±25 RPM at 22±3 °C). After complete mixing of CDAP under stirring, pH was adjusted to 9.5±0.3 using sodium hydroxide/ acetic acid, except for serotype 19A, wherein pH is in the range of 9.0 ± 0.3. The time duration for activation of the polysaccharide is 3 to 10 minutes depending on the serotype, and the activation time starts from pH adjustment.

After completion of activation, using the same agitation and temperature conditions, the specific carrier protein for each polysaccharide was added to the activated polysaccharide (polysaccharide: carrier protein ratio = 1: 0.5 to 1: 1.5) to initiate the conjugation reaction. The carrier protein was added after 3 to 10 minutes of activation depending on the specific serotype. The pH for conjugation was maintained at 9.5± 0.3 except for serotype 19A, wherein pH was in the range of 9.0±0.3. The conjugation duration varies for different serotypes and was typically carried out for 20 minutes to 9 hours depending upon the serotype for which conjugation reaction is being carried out. For serotype 6A, conjugation was continued up to 4±1.5 hours. After complete conjugation, the reaction was quenched using NLT 10 parts of 2M glycine with pH adjustment to 8.1±0.3. The reaction is to be continued for NLT 1 hour at 22±3 °C followed by 10 to 20 hours at 12±3 °C with stirring.

The conjugates were purified using 0.45 µm filtration, followed by tangential flow filtration (TFF). Unwanted reaction residues (free polysaccharides, protein, CN-, ACN, DMAP) and low molecular weight conjugates were primarily removed by TFF of 300 kDa (primary purification) membrane with NLT 20 CVD in 0.9% NaCl solution followed by 100 kDa diafiltration as final purification step using similar process steps as that of primary purification.

The molecular weight of purified individual conjugates was in the range of 1500 kDa to 30000 kDa (SEC-MALS).

The purified conjugate was diluted/ stabilized by adding stock solution of 200 mM L-histidine in 0.9 % w/v NaCl in order to make its final concentration of 20 mM L- histidine. A final 0.22 µ filtration was carried out to obtain Monovalent Bulk Conjugate (MBC), which was stored at 2 to 8 °C till further use.

| **Table-2: Critical conjugation parameters** | | | | | | | | | | | |
|---|---|---|---|---|---|---|---|---|---|---|---|
| **Serotypes** | **1** | **2** | **3** | **4** | **5** | **6A** | **6B** | **7F** | **8** | **9V** | **10A** |
| Tentative PnPs Mw | 100 - 200 kDa (except type 6A: purified / 400-900 kDa) | | | | | | | | | | |
| PnPs conc. (mg/ml) before activation | 3-7 | 4-6 | 3-5 | 5-7 | 5-8 | 5-8 | 10-12 | 9-12 | 3-5 | 7-9 | 7-9 |
| Electrolyte | Sodium chloride to 2±0.2 M final concentration | | | | | | | | | | |
| CDAP solution | ∼100 mg/ml in acetonitrile. Freshly prepared, CDAP parts are ±0.25 parts w.r.t. PnPs (w/w) activation and conjugation conditions are 110±25 RPM at 22±3 °C | | | | | | | | | | |
| Ps:CDAP (ratios); (wt/wt) | 1:1.1 | 1:0.8 | 1:0.3 | 1:0.8 | 1:1.1 | 1:1 | 1:1.5 | 1:1.5 | 1:0.5 | 1:1 | 1:1 |
| pH adjust after | ∼ 1 min | | | | | | | | | | |
| Activation pH (± 0.3) | 9.5 | | | | | | | | | | |
| Activation duration (min) | 4 ± 1 | | | | | | | | | | |
| **Protein** | CRM 197 | CRM 197 | TT | DT | CRM 197 | TT | CRM 197 | CRM 197 | CRM 197 | TT | CRM 197 |
| Protein conc. (mg/ml) | 20 mg/ml carrier protein+ sodium chloride to 2±0.2 M final concentration, Carrier protein parts are ±0.25 parts | | | | | | | | | | |
| Ps:Protein parts (wt/wt) | 1:1 | 1:1 | 1:1 | 1:0.9 | 1:1 | 1:1.2 | 1:1.1 | 1:1 | 1:1 | 1:1 | 1:1 |
| Protein addition After | 3-10 minutes of activation depending on serotypes | | | | | | | | | | |
| Conjugation pH (± 0.3) | 9.5 | | | | | | | | | | |
| Conjugation duration | 20 min - 9 hours depending on the serotypes (For 6A, continue reaction up to 4±1.5 hours) | | | | | | | | | | |
| Quenching with | PnPs : glycine = 1 : NLT10 parts (w/w ) | | | | | | | | | | |
| Quenching pH | 8.1±0.3 | | | | | | | | | | |
| Quenching duration | NLT 1 hour at 22±3 °C followed by 10-20 hours at 12±3 °C with stirring | | | | | | | | | | |

| **Table-2 (Continued)** | | | | | | | | | | | |
|---|---|---|---|---|---|---|---|---|---|---|---|
| **Serotypes** | **11A** | **12F** | **14** | **15B** | **18C** | **19A** | **19F** | **22F** | **23F** | **33F** | **24F** |
| Tentative PnPs Mw | 100 - 200kDa | | | | | | | | | | |
| PnPs conc. (mg/ml) before activation | 8-10 | 4-6 | 9-11 | 10-13 | 4-6 | 9-12 | 9-11 | 9-12 | 7-10 | 4-6 | 3-7 |
| Electrolyte | Sodium chloride to 2±0.2 M final concentration | | | | | | | | | | |
| CDAP solution | ∼100 mg/ml in acetonitrile. Freshly prepared, CDAP parts are ± 0.25 parts w.r.t. PnPs (w/w) activation and conjugation conditions are 110 ±25 RPM at 22 ± 3 °C | | | | | | | | | | |
| CDAP (parts); w/w | 1:1 | 1:1.1 | 1:1 | 1:1.2 | 1:1.2 | 1:1 | 1:0.8 | 1:1 | 1:1.5 | 1:0.9 | 1:1 |
| pH adjust after | - 1 min | | | | | | | | | | |
| Activation pH (± 0.3) | 9.5 | 9.5 | 9.5 | 9.5 | 9.5 | 9.0 | 9.5 | 9.5 | 9.5 | 9.5 | 9.5 |
| Activation duration (min) | 4 ± 1 | 4 ± 1 | 4 ± 1 | 4 ± 1 | 4 ± 1 | 6 ± 3 | 7 ± 3 | 4 ± 1 | 4 ± 1 | 4 ± 1 | 4 ± 1 |

| **Protein** | CRM 197 | CRM 197 | CRM 197 | TT | CRM 197 | TT | CRM 197 | TT | CRM 197 | CRM 197 | DT |
|---|---|---|---|---|---|---|---|---|---|---|---|
| Protein conc. (mg/ml) | 20 mg/ml carrier protein+ sodium chloride to 2 ± 0.2 M final concentration, Carrier protein parts are ± 0.25 parts | | | | | | | | | | |
| Protein parts (w/w) | 1:1 | 1:1.1 | 1:1.1 | 1:1 | 1:1 | 1:1 | 1:1 | 1:1.25 | 1:1 | 1:1 | 1:1 |
| Protein addition after | 3-10 min of activation depending on serotypes | | | | | | | | | | |
| Conjugation pH (± 0.3) | 9.5 | 9.5 | 9.5 | 9.5 | 9.5 | 9.0 | 9.5 | 9.5 | 9.5 | 9.5 | 9.5 |
| Conjugation duration | 20 min - 9 hours depending on the serotypes | | | | | | | | | | |
| Quenching with | PnPs : glycine = 1 : NLT10 parts (w/w ) | | | | | | | | | | |
| Quenching pH | 8.1±0.3 | | | | | | | | | | |
| Quenching duration | NLT 1 hour at 22±3 °C followed by 10-20 hours at 12±3 °C with stirring | | | | | | | | | | |

Table-3 illustrates the characteristics of the pneumococcal polysaccharides and the pneumococcal polysaccharide-carrier protein conjugates present in the vaccine composition of the present disclosure.

| **Table-3: Characteristics of the pneumococcal polysaccharides and the pneumococcal polysaccharide-carrier protein conjugates** | | | | | |
|---|---|---|---|---|---|
| **Sero type** | **Carrier protein** | **Mw of polysaccharide (kDa) (SEC-HPLC)** | **Polysaccharide: carrier protein ratio** | **Free polysaccharide (%)** | **Molecular weight of Conjugate (kDa) (SEC-MALS)** |
| 2 | CRM | 157 | 1.63 | 2.55 | 6998 |
| 2 | CRM | 142 | 0.66 | <1 | 12840 |
| 3 | CRM | 168 | 0.81 | 3.9 | 4818 |
| 3 | CRM | 168 | 0.79 | 1.25 | 9630 |
| 3 | CRM | 168 | 0.77 | 2.81 | 6253 |
| 4 | DT | 160 | 1.27 | 9 | 8030 |
| 4 | DT | 144 | 1 | ND | 4316 |
| 4 | DT | 144 | 0.9 | 1.74 | 3327 |
| 4 | CRM | 144 | 0.73 | 2.41 | 4999 |
| 12F | CRM | 160 | 1.04 | 18.5 | 6592 |
| 12F | CRM | 151 | 0.8 | 3.03 | 12686 |
| 12F | CRM | 151 | 0.93 | 10.1 | 3353 |
| 15B | TT | 141 | 0.7 | 1.54 | 8852 |
| 15B | CRM | 141 | 0.7 | <1 | 8361 |
| 15B | TT | 141 | 0.8 | 7.05 | 8171 |
| 18C | CRM | 155 | 0.97 | >10 | 4803 |
| 18C | CRM | 152 | 0.84 | 2.71 | 6549 |
| 18C | CRM | 220 | 0.89 | 3.39 | 8198 |

| | | | | | |
|---|---|---|---|---|---|
| 22F | TT | 123 | 0.6 | <1 | 15370 |
| 22F | TT | 123 | 0.8 | <2 | 10850 |
| 22F | TT | 132 | 0.75 | <1 | 16750 |
| 22F | TT | 132 | 0.56 | <2 | Not available |
| 22F | CRM | 132 | 0.63 | <2 | 10770 |
| 8 | CRM | 135 | 0.81 | 3.65 | 2973 |
| 10A | CRM | 155 | 0.77 | 1.3 | 8417 |
| 11A | CRM | 146 | 0.85 | <1 | 7854 |
| 11A | CRM | 146 | 0.6 | <2 | 9652 |
| 33F | CRM | 135 | 0.9 | <1 | 23340 |
| 6A | TT | 763 | 1.07 | 1.91 | 8431 |
| 9V | TT | 148 | 0.48 | ND | 24050 |
| 19A | TT | 137 | 0.76 | <2.69 | 6769 |
| 19A | DT | 137 | 0.63 | <1 | 4734 |
| 24F | DT | 157 | 1.08 | 3.77 | 2370 |
| 1 | CRM | 139 | 0.7 | 2.3 | 6154 |
| 5 | CRM | 147 | 0.6 | 13.2 | 4289 |
| 6B | CRM | 146 | 0.6 | <1.5 | 8837 |
| 7F | CRM | 145 | 0.8 | 3.5 | 12260 |
| 14 | CRM | 136 | 0.7 | <1.5 | 13840 |
| 19F | CRM | 154 | 0.6 | <1.5 | 6222 |
| 23F | CRM | 144 | 0.6 | 3.7 | 8534 |

### Estimation of free polysaccharide and free protein:

1. Free protein estimation:
   a. Protocol for free TT/DT estimation by BBCIA/ Sandwich ELISA

### Two step protocol

### Step 1: Isolation of free TT/DT

Monoclonal antibody specific to respective serotypes are incubated with Protein A resin. Protein A resin has affinity towards Fc region of Ab, allowing Fab region of Ab to interact with specific antigen. Conjugated Ps when incubated with Ab tagged protein A resin, binds to conjugated Ps and free Ps present in conjugate. Ab tagged resin along with conjugate is centrifuged to pellet down resin. Supernatant that contains free TT/DT is collected in separate tube. Free TT is estimated using bead based competitive inhibition assay (BBCIA). Free DT is estimated using Sandwich ELISA.

### Step 2: Estimation of TT by BBCIA

Microsphere beads are coupled with TT for using them in BBCIA. Supernatant i.e. expected to have free TT, is pre-neutralized using anti TT monoclonal antibody in microtiter plate. Pre-neutralized sample is transferred to microsphere beads coupled with TT. Free Ab available post pre-neutralization step interacts with TT coupled beads. Ab-Ag coupled bead reaction is detected using anti mouse IgG (fc specific)-Phycoerythrin conjugate. The plate is read using BioPlex -200 protein suspension array system. MFI (Median fluorescent intensity) is inversely proportional to the concentration present in sample.

### Step 2: Estimation of DT by Sandwich ELISA

ELISA plates are coated with anti DT equine polyclonal sera. Supernatant i.e. expected to have free DT is added to the wells. Anti DT murine monoclonal antibody (in-house) is conjugated with HRP using HRP conjugation kit. DT antigen is sandwiched between polyclonal antibody specific to DT and anti DT murine monoclonal antibody-HRP conjugate. TMB along with substrate is added to detect the reaction. The reaction is quenched using 1N HCl solution. Absorbance is measured at 450 nm using microplate spectrophotometer. Absorbance is directly proportional to the concentration of DT present in sample.

### b. Alternative method for free protein estimation by CZE

Micellar electrokinetic chromatography (MEKC) is one of methods of electrokinetic chromatography and is based on the use of ionic surfactant in running buffer at a concentration sufficiently higher than its critical micellar concentration. Sodium dodecyl sulphate (SDS) is one of the most popular surfactants and is used extensively for MEKC. The SDS micelle migrates towards positive electrode by electrophoresis. The electroosmotic flow is in the direction of negative electrode and it is stronger than the electrophoretic migration of the SDS micelle at a pH above 5. Thus, SDS micelle also migrates towards the negative electrode at a velocity equal to the difference between the electroosmotic and electrophoretic velocities. A neutral analyte migrates in the micellar solution at the electroosmotic velocity when it is free from the micelle and at the velocity of the micelle when it is incorporated into the micelle. Thus, separation of solutes will be a result of relative distribution coefficients in micellar phase (micelles) and aqueous phase.

Free CRM197 is separated from the conjugate by capillary electrophoresis in micellar electrokinetic chromatography (MEKC) mode. The absorbance at 200 nm is monitored and free CRM197, DT or TT is quantified using respective reference standard. The specific polysaccharide-carrier protein combinations in accordance with the present disclosure for estimating free protein is given in Table-4.

| **Table-4: Free protein estimation by MEKC method: pneumococcal polysaccharides conjugated to the specific carrier proteins** | | |
|---|---|---|
| **Serotypes** | **Conjugated with carrier protein** | **Method followed** |
| 2, 8, 10A, 11A, 12F, 18C, 33F | CRM 197 | MEKC (Micellar electrokinetic chromatography) |
| 4, 24F | DT | |
| 6, 9V, 19A, 3, 15B, 22F | TT | |

### 2. Free polysaccharide estimation

### Principle

Sodium deoxycholate (DOC) is an ionic detergent and is used for selective precipitation of protein in complex solution. This property of DOC is used here for estimation of free saccharide in polysaccharide conjugate samples.

In conjugate samples, DOC precipitate the free as well as conjugated protein. The precipitate can be removed by filtration and filtrate can be used for estimation of saccharide content.

### Experimental procedure

Method A: For serotypes 1, 3, 6B, 7F, 8, 10A, 11A, 12F, 14, 15B, 18C, 22F, 23F, 33F
   Blank preparation: Take 600 µL of 20 mM Histidine buffer in place of sample and follow the same procedure as given below.
   Take 600 µL of sample into each Eppendorf tube in duplicate.
   Add 204 µL of 10% DOC solution in to each sample and vortex gently.
   Incubate the samples at room temperature for 10 minutes.
   Add 77 µL of 1N Hydrochloric acid in to each sample and vortex gently.
   Incubate the samples at room temperature for 10 minutes.
   Vortex gently and filter the entire volume with 0.22µ syringe filter into a new Eppendorf tube or transfer into a spin filter and centrifuge at 5000 RPM for 2 minutes.
   Transfer immediately 500 µL of the filtrate in a new Eppendorf tube containing 30 µL of 0.5M sodium bicarbonate in to the sample and vortex gently.
Method B: For serotypes 2, 4, 6A, 19A,19F
   Blank preparation: Take 650 µL of 20 mM Histidine buffer in place of sample and follow the same procedure as given below.
   Take 650 µL of sample in to each Eppendorf tube in duplicate.
   Add 221 µL of 10% DOC solution in to each sample and vortex gently.
   Incubate samples in Ice-bath for 2 minutes.
   Add 62 µL of 1N Hydrochloric acid in to each sample.
   Vortex gently and filter the entire volume with 0.22µ syringe filter into a new Eppendorf tube or transfer into a spin filter and centrifuge at 5000 RPM for 2 minutes.
   Transfer immediately 500µL of the filtrate in to a new Eppendorf tube containing 20µL of 0.5M sodium bicarbonate and vortex gently.

### (v) Formulation:

The general process for preparing the 20/ 21-valent vaccine composition in accordance with the present disclosure is provided in Figure-2.

Blend A: pneumococcal polysaccharide-carrier protein conjugates 4-DT, 6A-TT, 9V-TT, 15B-TT, 22F-TT and 23F-CRM; histidine; NaCl; and 2-PE (in case of multi-dose formulation) was mixed with aluminium phosphate; and succinate at a temperature of 18 °C to 30 °C, followed by pH adjustment at 6±0.5 (after NLT 2h), polysorbate 20 was added after 6.0±2h.

Blend B: pneumococcal polysaccharide-carrier protein conjugates 1-CRM, 2-CRM, 5-CRM, 6B-CRM, 7F-CRM, 8-CRM, 10A-CRM, 11A-CRM,12F-CRM,14-CRM, 18C-CRM,19A-TT, 19F-CRM, 33F-CRM (24F-DT in case of 21-valent composition); histidine; NaCl; and 2-PE (in case of multi-dose formulation) was mixed with aluminium phosphate; and succinate at a temperature of 18 °C to 30 °C, followed by pH adjustment at 6±0.5 (after NLT 2h), polysorbate 20 was added after 6.0±2h. Pneumococcal polysaccharide-carrier protein conjugate 3-TT if used was present in Blend-B.

Blend A (after 24±6 h) and Blend B (after 18±6 h) was mixed under stirring for 72 h at 4 to 12 °C. pH was adjusted, if required, to be 6±0.5 and final volume was adjusted with water for injection (WFI) to obtain the vaccine composition (PCV-20/ 21 Formulated Bulk). This was filled in suitable containers (such as glass vials, siliconized vials, pre-filled syringes) as required. The siliconized vials were supplied by (i) Muller + Muller - Joh, Germany; (ii) Nuova Ompi, Italy; and (iii) Schott Kaisha, India. The prefilled syringe was supplied by BD Medical Pharmaceuticals.

Different formulations were prepared using the method of the present disclosure as provided in Table-5.

| **Table-5: Vaccine compositions in accordance with the present disclosure (All the values are per 0.5 ml dose)** | | | | | | | | | | | |
|---|---|---|---|---|---|---|---|---|---|---|---|
| **Serotype*** | **Expt-106** | **Expt-111** | **Expt-124** | **Expt-119** | **Expt-121** | **Expt-123** | **Expt-131** | **Expt-131a** | **Expt-131b** | **Expt-125** | **Expt-135** |
| | **21-valent** | | | | | | | | | | **22-valent** |
| **1** | CRM | CRM | CRM | CRM | CRM | CRM | CRM | CRM | CRM | CRM | CRM |
| **2** | CRM | CRM | CRM | CRM | CRM | CRM | CRM | CRM | CRM | CRM | CRM |
| **3** | CRM | CRM | CRM | CRM | CRM | CRM | TT | TT | TT | CRM | TT |
| **4** | DT | DT | DT | DT | CRM | DT | DT | DT | DT | DT | DT |
| **5** | CRM | CRM | CRM | CRM | CRM | CRM | CRM | CRM | CRM | CRM | CRM |
| **6A** | CRM | CRM | CRM | TT | CRM | TT | TT | TT | TT | CRM | TT |
| **6B** | CRM | CRM | CRM | CRM | CRM | CRM | CRM | CRM | CRM | CRM | CRM |
| **7F** | CRM | CRM | CRM | CRM | CRM | CRM | CRM | CRM | CRM | CRM | CRM |
| **8** | CRM | CRM | CRM | CRM | CRM | CRM | CRM | CRM | CRM | CRM | CRM |
| **9V** | CRM | CRM | CRM | TT | CRM | TT | TT | TT | TT | CRM | TT |
| **10A** | CRM | CRM | CRM | CRM | CRM | CRM | CRM | CRM | CRM | CRM | CRM |
| **11A** | CRM | CRM | CRM | CRM | CRM | CRM | CRM | CRM | CRM | CRM | CRM |
| **12F** | CRM | CRM | CRM | CRM | CRM | CRM | CRM | CRM | CRM | CRM | CRM |
| **14** | CRM | CRM | CRM | CRM | CRM | CRM | CRM | CRM | CRM | CRM | CRM |
| **15B** | TT | TT | TT | CRM | CRM | TT | TT | DT | CRM | TT | TT |
| **18C** | CRM | CRM | CRM | CRM | CRM | CRM | CRM | CRM | CRM | CRM | CRM |
| **19A** | CRM | CRM | CRM | TT | CRM | TT | TT | TT | TT | CRM | TT |
| **19F** | CRM | CRM | CRM | CRM | CRM | CRM | CRM | CRM | CRM | CRM | CRM |
| **22F** | TT | TT | TT | TT | CRM | TT | TT | TT | TT | TT | TT |
| **23F** | CRM | CRM | CRM | CRM | CRM | CRM | CRM | CRM | CRM | CRM | CRM |
| **33F** | CRM | CRM | CRM | CRM | CRM | CRM | CRM | CRM | CRM | CRM | CRM |
| **24F** | - | - | - | - | - | - | - | - | - | - | DT |
| **AlPO4** | NMT 1.13 mg (0.847 mg Aluminium phosphate, i.e., 187.5 µg as Al³⁺) | | | | | | | | | | |
| **Histidine** | 1.55 mg | | | | | | | | | | |
| **PS -20** | 100 µg | | | | | | | | | | |
| **Succinate** | 1.18 mg | | | | | | | | | | |
| **NaCl** | 4.5 mg | | | | | | | | | | |
| **2-PE** | 4 mg | | | | | | | | | | |
| *The vaccine composition may include at least one *S. pneumoniae* polysaccharide derived from serotypes selected from 9A, 9N, and 9L. | | | | | | | | | | | |

Increasing the number of serotypes conjugated to tetanus toxoid (TT) may result in immunosuppression and hence formulations of 131c (refer Table-5a) and 135a (refer Table-5b), comprising 20 valent and 21 valent compositions, respectively and devoid of serotype 3 (serotype 3 conjugated to TT as the carrier protein) were also studied.

| **Table-Sa: Vaccine compositions in accordance with the present disclosure (All the values are per 0.5 ml dose)** | |
|---|---|
| **Serotype** | **Expt-131c (20 valent)** |
| **1** | CRM |
| **2** | CRM |
| **4** | DT |
| **5** | CRM |
| **6A** | TT |
| **6B** | CRM |
| **7F** | CRM |
| **8** | CRM |
| **9V** | TT |
| **10A** | CRM |
| **11A** | CRM |
| **12F** | CRM |
| **14** | CRM |
| **15B** | TT |
| **18C** | CRM |
| **19A** | TT |
| **19F** | CRM |
| **22F** | TT |
| **23F** | CRM |
| **33F** | CRM |
| **24F** | - |
| **AlPO4** | NMT 1.13 mg (0.847 mg Aluminium phosphate, i.e., 187.5 µg as Al³⁺) |
| **Histidine** | 1.55 mg |
| **PS -20** | 100 µg |
| **Succinate** | 1.18 mg |
| **NaCl** | 4.5 mg |
| **2-PE** | 4 mg |

| **Table-Sb: Vaccine compositions in accordance with the present disclosure (All the values are per 0.5 ml dose)** | |
|---|---|
| **Serotype** | **Expt-135a (21 valent)** |
| **1** | CRM |
| **2** | CRM |
| **4** | DT |
| **5** | CRM |
| **6A** | TT |
| **6B** | CRM |
| **7F** | CRM |
| **8** | CRM |
| **9V** | TT |
| **10A** | CRM |
| **11A** | CRM |
| **12F** | CRM |
| **14** | CRM |
| **15B** | TT |
| **18C** | CRM |
| **19A** | TT |
| **19F** | CRM |
| **22F** | TT |
| **23F** | CRM |
| **33F** | CRM |
| **24F** | DT |
| **AlPO4** | NMT 1.13 mg (0.847 mg Aluminium phosphate, i.e., 187.5 µg as Al³⁺) |
| **Histidine** | 1.55 mg |
| **PS -20** | 100 µg |
| **Succinate** | 1.18 mg |
| **NaCl** | 4.5 mg |
| **2-PE** | 4 mg |

Table-6 shows the percent adsorption of 20-valent composition (Expt-131c) prepared in accordance with the present disclosure. The 20-valent composition (Expt-131c) comprises three different carrier proteins that are individually conjugated to the polysaccharides derived from 20 pneumococcal serotypes.

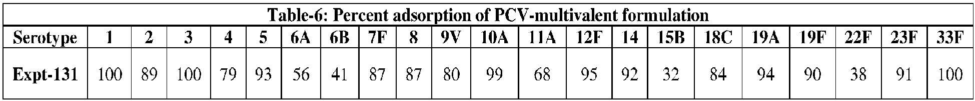

It is seen from Table-6 that the 20-valent composition (Expt-131c) prepared in accordance with the present disclosure has % adsorption of at least 20% for all the serotypes. Further, ≥ 90% adsorption is seen for serotypes 1, 3, 5, 10A, 12F, 14, 19A, 19F, 23F and 33F. Similar adsorption is also expected, when the composition is devoid of serotype 3 conjugate.

No aggregation/ white particles or any type of floccules was observed in any of the PCV formulations indicating that formulation is quite stable and consistent. The absence of aggregation can be attributed to the use of a sized polysaccharide in the range of 100 - 200 kDa, and polysaccharide to CDAP (Cyanylation agent) ratio in the range of (1):(0.3 - 1.5).

The 21-valent composition (Expt-135 and Expt-135a) contains an additional conjugate (24-DT), rest of the components are same as Expt-131 and Expt-135c. As seen from Table-6, most of the polysaccharides conjugated to TT/ CRM show good adsorption (>50%), similarly, Serotype 24 conjugated to DT is expected to give good adsorption when included in the 21-valent formulation.

### Example-2: Determination of Total Nitrogen Content of purified polysaccharides

### Serotypes: 2, 3, 4, 8, 10A, 11A, 12F, 15B, 18C, 22F, 24F and 33F

| **Table-7: Nitrogen Content limits** | | | |
|---|---|---|---|
| **Serotype** | **Specifications (% of dry weight)** | **Reference** | **Results (%)** |
| 2 | 0 - 1.0 | EP/BP/WHO | 0.5 |
| 3 | 0 - 1.0 | EP/BP/WHO | 0.7 |
| 4 | 4.0 - 6.0 | EP/BP/WHO | 4.8 |
| 8 | 0 - 1.0 | EP/BP/WHO | 0.3 |
| 10A | 0.5 - 3.5 | EP/BP/WHO | 1.5 |
| 11A | 0 - 2.5 | EP/BP/WHO | 0.3 |
| 12F | 3.0 - 5.0 | EP/BP/WHO | 3.7 |
| 15B | 1.0 - 3.0 | EP/BP/WHO | 1.7 |
| 18C | 0 - 1.0 | EP/BP/WHO | 0.5 |
| 22F | 0 - 2.0 | EP/BP/WHO | 0.3 |
| 24F | Should be present | In-house | 1.6 |
| 33F | 0 - 2.0 | EP/BP/WHO | 0.5 |

| | | | |
|---|---|---|---|
| It is seen from the Table-7 that the total nitrogen content of the polysaccharides used in the vaccine composition(s) of the present disclosure are well within the specified range. | | | |

TNM method is based on detection and quantification of total nitrogen using chemiluminescence detector. The detection offered by TNM is superior to existing methods such as Kjeldahl method in terms of following advantages:
a) High throughput
b) Does not involve use of concentrated acids which is the case for Kjeldahl based methods.
c) Capable of detection of all kinds of nitrogen linkages such as peptides, amino acids, nucleic acids, amino-sugars, nitrates and nitrites. The Kjeldahl based methods have limitations that it cannot determine nitrogen forms such as nitrate and nitrite.
d) Very sensitive. It can detect nitrogen up to ppb level which is not the case with Kjeldahl (sensitivity in ppm).
e) Requires minimal quantity of samples as compared to Kjeldahl or CHNS methods.

TNM method for estimating nitrogen content as used in the present disclosure offers following advantages to CHNS method as described in Table-8.

| **Table-8: Comparison of TOC TNM and CHNS elemental analyzer** | | |
|---|---|---|
| **S. No** | **TOC TNM** | **CHNS elemental analyzer** |
| 1 | Measurement range is 0 to 10, 000 mg/Liter. | <100 ppm |
| 2 | Detection limit is ≥5ppb. | Detection limit is ≥300 ppm. |
| 3 | Duplicate CV is 3.0% max. | CVs can go up to 10 % due to differences of thermal conductivity rate of different compounds. |
| 4 | Chemiluminescence detector | Thermal conductivity detector |
| 5 | Highly specific and selective-No interference of metallic ions or bromine in test results. | Interference of metallic ions or bromine in test results. |

### Example-3: Optimization of aluminium phosphate gel and polysorbate 20

### Aluminium phosphate gel:

Aluminum salt is regularly used in vaccine and its safety profile, ease of preparation, stability and potent immunostimulatory ability is well established.

The vaccine compositions of the present disclosure were evaluated with different concentrations of aluminium phosphate gel (alum gel) and its impact on adsorption of individual serotypes was studied. Composition details and the result obtained is provided in Table-9.

| **Table-9: % Adsorption of Serotypes (conjugates)** | | |
|---|---|---|
| **Serotype** | **Expt-98 (AlPO₄ - 125 µg/0.5ml)** | **Expt-131 (AlPO₄ - 187.5 µg/0.5ml)** |
| **1** | 100 | 100 |
| **2** | 38 | 89 |
| **3** | 100 | 100 |
| **4** | 54 | 79 |
| **5** | 73 | 93 |
| **6A** | 83 | 56 |
| **6B** | 10 | 41 |
| **7F** | 28 | 87 |
| **8** | 50 | 87 |
| **9V** | 88 | 80 |
| **10A** | 86 | 99 |
| **11A** | 0 | 68 |
| **12F** | 91 | 95 |
| **14** | 90 | 92 |
| **15B** | 60 | 32 |
| **18C** | 65 | 84 |
| **19A** | 91 | 94 |
| **19F** | 0 | 90 |
| **22F** | 0 | 38 |
| **23F** | 82 | 91 |
| **33F** | 96 | 100 |

It is seen from Table-9 that increasing the alum gel resulted in a positive impact on the adsorption of a large number of the serotypes of 21-valent vaccine composition. Increasing the alum gel from 125 µg to 187.5 µg (Al³⁺) per dose significantly improved the adsorption for most of the serotypes. More than 70% of the serotype conjugates showed improvement in adsorption when alum content was increased. Further, ≥ 90% adsorption is seen for serotypes 1, 3, 5, 10A, 12F, 14, 19A, 19F, 23F and 33F in the formulation having more alum content (187.5 of Al³⁺ µg per dose). Similar results are also expected when the vaccine composition of the present disclosure is devoid of polysaccharide from *S. pneumoniae* serotype 3.

Conjugate serotype 4 which has DT as Carrier protein showed lower adsorption (54%) with 125 µg/ dose (Al³⁺) alum gel. The adsorption (79%) was considerably improved when alum gel content was increased to 187.5 µg/ dose (Al³⁺). Similar trend is observed for majority of the remaining serotypes also. It was seen that the additional serotypes that were not present in Applicant's 10-valent pneumococcal vaccine (PNEUMOSIL^{®}), showed lower adsorption when lower amount of alum gel was used. However, a considerable improvement in adsorption was seen when higher amount of alum gel (187.5 µg (Al³⁺)) per dose was used.

### Comparative adsorption data for multivalent vaccine composition of the present disclosure:

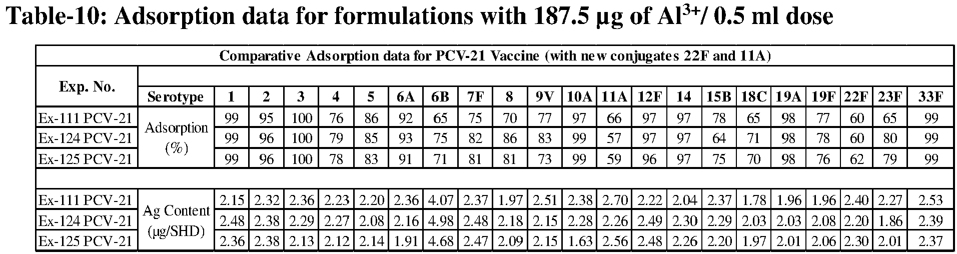

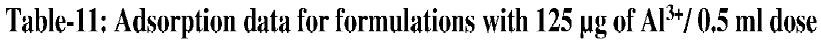

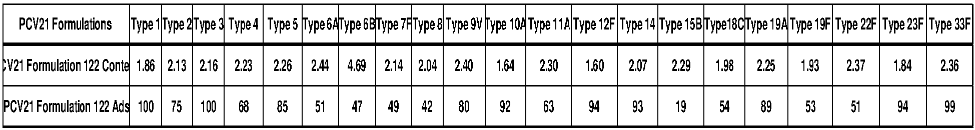

It is seen from Table-10 and Table-11 adsorption was relatively lower with low alum content for many serotypes including 6B, 7F and 15B, whereas higher adsorption was observed when the alum content was increased. Hence, 187.5 µg of Al³⁺/ 0.5 ml dose was used in further studies.

### Comparative studies on effect of aluminium content in formulation on adsorption of serotypes

Table-12 shows the formulation detail and the effect of different aluminium content on adsorption and the antigen content.

| **Table-12: Effect of aluminium content on adsorption of serotypes** | | | | |
|---|---|---|---|---|
| **Serotype** | **Exp-98 PCV-21** (with Al³⁺ 125µg/SHD) | | **Exp-97 PCV-21** (with Al³⁺ 187.5µg/SHD) | |
| | Antigen content (µg/SHD) | % Adsorption | Antigen content (µg/SHD) | % Adsorption |
| **1** | 1.94 | 100 | 1.96 | 100 |
| **2** | 2.47 | 38 | 2.52 | 98 |
| **3** | 2.47 | 100 | 2.30 | 100 |
| **4** | 2.33 | 54 | 2.26 | 83 |
| **5** | 2.19 | 73 | 1.85 | 81 |
| **6A** | 2.48 | 83 | 2.27 | 94 |
| **6B** | 4.75 | 10 | 3.23 | 75 |
| **7F** | 2.12 | 28 | 1.93 | 76 |
| **8** | 2.25 | 50 | 2.32 | 85 |
| **9V** | 1.87 | 88 | 1.86 | 92 |
| **10A** | 2.52 | 86 | 1.88 | 100 |
| **11A** | 2.17 | 0 | 1.79 | 39 |
| **12F** | 2.17 | 91 | 2.26 | 96 |
| **14** | 1.98 | 90 | 1.88 | 97 |
| **15B** | 2.37 | 60 | 2.47 | 84 |
| **18C** | 2.00 | 65 | 1.90 | 77 |
| **19A** | 2.10 | 91 | 1.71 | 97 |
| **19F** | 2.22 | 0 | 1.80 | 68 |
| **22F** | 2.77 | 0 | 2.51 | 69 |
| **23F** | 1.93 | 82 | 2.06 | 94 |
| **33F** | 2.24 | 96 | 2.17 | 99 |

Table-12a illustrates the effect of aluminium content in the multivalent composition on IgG titer.

| **Table-12a: Effect of aluminium content in PCV 21 formulation on IgG (Rabbit, 42 days) Titre** | | |
|---|---|---|
| **Serotype** | **Exp 97 PCV21 with Al³⁺ 187.5µg/ SHD** | **Exp 98 PCV21 with Al³⁺ 125µg/ SHD** |
| 1 | 16600 | 5869 |
| 2 | 4525 | 2934 |
| 3 | 4150 | 2263 |
| 4 | 27917 | 19740 |
| 5 | 21527 | 15222 |
| 6A | 21527 | 15222 |
| 6B | 7611 | 6979 |
| 7F | 11738 | 6400 |
| 8 | 2934 | 2691 |
| 9V | 18102 | 9870 |
| 10A | 4525 | 1600 |
| 11 | 33199 | 21527 |
| 12F | 6979 | 3805 |
| 14 | 39481 | 33199 |
| 15B | 15222 | 4150 |
| 18C | 25600 | 21527 |
| 19A | 3200 | 2468 |
| 19F | 23475 | 18102 |
| 22F | 102400 | 78961 |
| 23F | 19740 | 13958 |
| 33F | 16600 | 15222 |
| Similar results are also expected, when the vaccine composition of the present disclosure is devoid of S. pneumoniae polysaccharide derived from serotype 3. | | |

Further, it is seen that increasing the amount of alum gel did not negatively impact the antigenicity of the vaccine compositions. It is found that higher alum gel in the immunogenic formulation is also beneficial in increasing B cell mediated IgG antibody response as evidenced by rabbit immunogenicity study data for almost all serotypes as shown in Table-12a. IgG antibody response in terms of numerical superiority for all the serotypes was observed. Further, IgG titers of at least 2-fold higher were obtained for serotypes 1, 10A, 15B and 22F.

Higher adsorption of individual conjugate serotypes is considered as a positive quality attribute for the vaccine compositions and is a stability indicating parameter. The increase in adsorption of conjugates will further improve the stability of individual antigen and lot to lot consistency of formulation batches in clinical material and commercial material manufacturing. This is particularly more important for few serotypes e.g. 4, 6B, 7F, 15B, 18C. Lower adsorption is expected to make the respective antigens to be at risk of degradation over time when the vaccine compositions are on shelf.

The individual adsorption and animal immunogenicity data has confirmed the advantage of use of higher alum gel in multivalent vaccine composition of the present disclosure. These compositions with higher immune response are likely to provide more consistent and potent results, useful at large scale vaccine manufacturing operations.

### Polysorbate 20:

The vaccine compositions of the present disclosure comprise aluminium phosphate particles, which are suspended in aqueous medium and polysorbate 20 is used as non-ionic surfactant as stabilizer. Surfactants play an important role in stabilizing formulation by reducing liquid-air, solid-liquid interfacial tension and improving the stability and flow properties of suspension-based formulations.

Applicant had previously used 50 µg of polysorbate 20 in their marketed 10-valent pneumococcal vaccine composition (PNEUMOSIL^{®}). However, the amount of polysorbate 20 was increased (100 µg) due to increased number of antigens (pneumococcal polysaccharides) and increased quantity of alum gel used in the vaccine compositions of the present disclosure. The higher quantity of polysorbate 20 in the present vaccine compositions show improved flow properties especially in the presence of increased alum gel content with higher number of antigens (pneumococcal polysaccharides) being added. Further, aggregates or floccules of product were not observed with increased polysorbate 20 concentrations in the vaccine compositions, when stored at 2 °C to 8 °C. Also, no adverse effect was observed when higher polysorbate 20 content was used, with respect to individual adsorption of the conjugate serotypes, individual antigen content and the immune response observed in animal studies (mice and rabbits).

The higher quantity of surfactant (polysorbate 20) in the vaccine composition of the present disclosure aids in obtaining an aggregation-free composition having improved flow properties even in the presence of increased alum gel content with higher number of antigens (pneumococcal polysaccharides) being include in the composition.

### Example-4: Immunogenicity studies

### (a) Comparison of Immunogenicity Data in NZW Rabbits for Serum Institute's/ SIIPLs multivalent pneumococcal formulation Expt. 131 with Expt. 106, Expt. 119 and commercial comparator (Prevenar13)

**Study Objective:** The objective of this study was to check immunogenicity of SIIPL's developmental multivalent pneumococcal formulation of Expt. 131 and compare with Expt. 106, Expt. 119 along with commercial comparator (Prevenar13) by assessing total IgG titres by multiplexed bead-based assay and functional IgG titres either by OPA or Multiplexed Opsonophagocytic assay (MOPA).

**Study Details:** Applicant's developmental PCV21 formulation of Expt. # 131 was injected with aluminium phosphate adjuvant in group of 8 New Zealand White rabbits (4 male and 4 female animals) following three intramuscular immunizations at day 0, day 14 and day 28 from the date of study initiation. The serum was separated from blood collected at days 0, 28 and 42 of the study to check for total IgG by bead-based assay and for functional IgG by Multiplexed Opsonophagocytic assay.

The titres at day 42 were compared with data of day 42 of previously tested PCV21 Expt. 106, Expt. 119 along with Prevenar13. The details of the formulations used in this study is described in Table-13 and the result is given in Table-14.

| **Table-13: Formulation Details** | |
|---|---|
| **Formulation** | **Details** |
| **PCV21 Expt. 106** | Each serotype at 2.2 µg/dose, except, type 6B at 4.4µg/dose; All serotypes as CRM197 conjugates except Type4-DT, Type 15B-TT and Type22F-TT; Aluminium phosphate gel at 187.5 µg/dose |
| **PCV21 Expt. 119** | Each serotype at 2.2 µg/dose, except, type 6B at 4.4µg/dose; All serotypes as CRM197 conjugates except Type4-DT, Type 6A, 9V, 19A, 22F-TT; Aluminium phosphate gel at 187.5 µg/dose |
| **PCV21 Expt. 131** | Each serotype at 2.2 µg/dose, except, type 6B at 4.4µg/dose; All serotypes as CRM197 conjugates except Type4-DT, Type 3, 6A, 9V, 15B, 19A, 22F-TT; Aluminium phosphate gel at 187.5 µg/dose |

### Results:

**Table-14-: Combined Total IgG and Functional IgG results for Expt. 106, Expt.119 and 131 along with Prevenar13 at Day 42**

| **Description** | **Assay** | **1** | **5** | **6A** | **68** | **7F** | **9V** | **14** | **19A** | **19F** | **23F** | |
|---|---|---|---|---|---|---|---|---|---|---|---|---|
| **SS5F1 (SIIPL, PCV21, Expt. 106)** | **Total IgG** | 6400 | 11738 | 8300 | 10763 | 6979 | 12800 | 30444 | 3200 | 15222 | 15222 | |
| | **OPA** | **23** | **247** | **95** | **142** | **135** | **52** | **175** | **32** | **41** | **235** | |
| | | | | | | | | | | | | |
| **S85F3 (SIIPL, PCV21, Expt. 119)** | **Total IgG** | 10763 | 25600 | 102400 | 13958 | 9870 | 51200 | 36204 | 9051 | 23475 | 19740 | |
| | **OPA** | **21** | **307** | **1839** | **387** | **181** | **512** | **269** | **431** | **596** | **260** | |
| | | | | | | | | | | | | |
| **S90F4 (SIIPL, PCV21, Expt. 131)** | **Total IgG** | 1104 | 12800 | 91228 | 28735 | 18102 | 64508 | 51200 | 8063 | 20319 | 22807 | |
| | **MOPA** | **32** | **81** | **1024** | **1024** | **456** | **2048** | **575** | **256** | **181** | **203** | |
| | | | | | | | | | | | | |
| **S85F4 (Prevenar 13)** | **Total IgG** | 4150 | 5382 | 6979 | 23475 | 3490 | 6400 | 2015 | 4150 | 11738 | 9870 | |
| | **OPA** | **13** | **67** | **283** | **596** | **108** | **119** | **70** | **211** | **233** | **279** | |

| **Description** | **Assay** | **2** | **3** | **4** | **12F** | **15B** | **18C** | **22F** | **8** | **10A** | **11A** | **33F** |
|---|---|---|---|---|---|---|---|---|---|---|---|---|
| **S85F1 (SIIPL, PCV21, Expt. 106)** | **Total IgG** | 4525 | 2691 | 16600 | 4935 | 3200 | 25600 | 223336 | 3200 | 2691 | 30444 | 18102 |
| | **OPA** | **197** | **5** | **953** | **762** | **32** | **160** | **2896** | **283** | **304** | **320** | **10** |
| | | | | | | | | | | | | |
| **S85F3 (SIIPL, PCV21, Expt. 119)** | **Total IgG** | 4935 | 3805 | 23475 | 7611 | 9051 | 36204 | 204800 | 2468 | 3805 | 60887 | 13958 |
| | **OPA** | **218** | **4** | **437** | **269** | **87** | **190** | **1133** | **309** | **260** | **664** | **19** |
| | | | | | | | | | | | | |
| **S90F4 (SIIPL, PCV21, Expt. 131)** | **Total IgG** | 9051 | 20319 | 40637 | 4525 | 45614 | 36204 | 144815 | 20319 | 7184 | 51200 | 5702 |
| | **MOPA** | **228** | **203** | **912** | **45** | **114** | **456** | **2048** | **128** | **456** | **512** | **323** |
| | | | | | | | | | | | | |
| **S85F4 (Prevenar 13)** | **Total IgG** | | 13958 | 16600 | | | 23475 | | | | | |
| | **OPA** | | **4** | **342** | | | **151** | | | | | |

### Overall Inference for the comparison:

Effect of conjugation with TT- As shown in Table-14, titers for both Total IgG (By BBA) and Functional IgG (By MOPA) have increased significantly for serotype 3, 6A, 9V and 19A wherein TT conjugate are used in Expt.#131 as compared to CRM conjugates in Expt.#106.

Additionally, cross reactivity for similar serotypes (i.e. 6A-6B and 19A-19F) is also observed for Expt.#131 as compared to Expt.#106. An antibody reacts with antigens other than the antigen that stimulated it. This phenomenon is called cross-reaction or cross-reactivity. Cross-reaction occurs due to the shared epitope on an antigen or conformational similarity of epitopes. In the present study it was seen from the data that there was an increase in the immune response (due to change in carrier protein) of 6A and 19A, immune response towards 6B and 19F also increased possibly due to cross-reactivity.

Titers (both, total IgG and Functional IgG) for Expt.#131 are comparable with titers obtained for commercial comparator (Prevnar13).

Immunogenicity studies of vaccine compositions devoid of pneumococcal polysaccharide derived from serotype 3 are ongoing and similar results are expected.

### Total IgG by Bead Based Assay:

The multiplexed bead-based assays on Luminex flow-based systems are set up on the basis of xMAP (multi-analyte profiling) technology. The unique multiplexing capabilities (up to 100-plex) of Luminex assays are based upon the use of microspheres that have been internally dyed with red and infrared fluorophores of differing intensities. Each bead is given a unique number, or 'bead region', allowing differentiation of one bead from another.

The multiplexed bead-based assay proved to be an effective alternative to widely used ELISA for estimating antibody response. The assay has many advantages over ELISA as this assay is more sensitive, specific and robust. Capacity of ELISA and cell-based assay as OPA to evaluate response for single antigen at a time makes it more laborious and more time consuming to generate data for multivalent vaccines. The multiplexed bead-based assay with its high throughput application requires much less time to estimate total IgG titre. Very few laboratories have resources and setup to implement complex cell-based assay as against bead-based assay is much simple and easy to replicate anywhere. When it comes to test multivalent vaccine candidate, volume of serum sample availability is a major issue for ELISA and OPA, but bead-based assay requires relatively lower volume of samples and generating data as precise and accurate as monovalent assay. Care has been taken to avoid cross reacting antibodies to bind with antigen of interest by using pneumococcal cell wall polysaccharide for serum dilutions. Various reports and published articles have shown that the data generated by multiplexed assay correlates well with traditional OPA titres.

In this assay, individual bead sets are coupled with each pneumococcal capsular polysaccharide (PnPS) antigen of 21-valent vaccine group using in-house developed coupling method. The antibodies in various serial dilutions of serum samples are allowed to react with polysaccharide antigen coupled on to beads and detected using fluorescent dye (R-Phycoerythrin) tagged antibody conjugate. The fluorescence generated by bead type of each antigen is measured on to Luminex system (Bio-Plex200, Bio-Rad).

The results were analysed in the form of antibody titres. The cut-off to calculate the total IgG titre was set as the reciprocal of the highest dilution of the serum sample yielding ≥ 100 MFI after deducting the background value for a particular antigen. If the blank subtracted MFI in 1:100 dilutions is <100, then two-fold less / previous dilution of 1:100 i.e. 50 is considered as the titre.

Similarly, if last dilution has ≥200 MFI after blank value deduction, the next respective two-fold dilution is considered as the titre.

### Functional Antibody estimation by Opsonophagocytic assay (OPA) or Multiplexed Opsonophagocytic assay (MOPA):

Opsonophagocytic killing assay (OPA) and Enzyme-linked immunoassays (EIA) are the most popular methods to assess vaccine efficacy. Although, EIAs provide useful information about the number of antibodies generated because of pneumococcal vaccines, it never really provides the information regarding the functional IgGs generated during the immune response. Since host protection against pneumococci is mediated via phagocytes causing opsonization of the bacteria, Opsonophagocytic killing assay (OPA) proves to be the preferred bioanalytical assay for the assessment of anti-pneumococcal antibodies that are functionally involved in the process of opsonization. In vitro opsonophagocytic assays (OPAs) with antibody and complement to mediate opsonophagocytic killing of bacteria have been designed and developed as an adjunct to the standardized serum immunoglobulin G anti-pneumococcal capsular polysaccharide enzyme immunoassay to assess the effectiveness of pneumococcal vaccines. OPA acts as a cornerstone during the development of a pneumococcal vaccine. It is vital to perform an OPA before the vaccine's licensure since any modulations or changes in the vaccine formulation can be thoroughly assessed followed by subsequent improvements in the formulation during the vaccine's development.

MOPA is a Multiplexed OPA, wherein OPA titers against four bacteria can be analyzed in a single well. Since monoplex OPA are time and labor intensive, performing MOPA has multiple advantages over OPA like requirement of less sera volume for testing extensive number of serotypes included in the vaccine formulation and high throughput nature of the assay allows fast and accurate analysis.

MOPA strains were procured from UAB (University of Alabama- Prof Moon Nahm's Lab) which is also the reference lab for Pneumococcal immunogenicity analysis. All leading pneumococcal vaccine manufactures are currently using the same MOPA procedure and strains supplied by UAB for their research and clinical evaluation of the vaccine.

MOPA uses modified pneumococcal clinical strains which have antibiotic resistant for a particular antibiotic (antibiotics include: Streptomycin, Spectinomycin, Trimethoprim and Optochin) and are used in sets of bacteria (4 bacteria per set). Various dilutions of sera samples are analysed and highest dilution of the sera yielding ≥50% killing of the bacteria as compared to the complement control wells is considered as the MOPA titer.

**Modified Amine Coupling for estimating antigen content estimation** Luminex xMAP is new technology which utilizes carboxylated beads (microspheres) that are labelled internally with two different fluorophores, designating each bead set a unique ID called bead region. This process imparts an important application to this method that it can be employed in assessing multiple (up to 100) different analytes at the same time in a given sample. For this, there is need of analytes to be coupled / conjugated onto beads using COOH group present on them. Many coupling methods are reported for coupling of bacterial polysaccharides, which are mostly two-day long protocol. When previously developed methods as DMTMM-COOH, DMTMM-NH₂, PLL were applied for coupling of SIIPL type 6B, 9V and 19A polysaccharides, the resulting coupled beads were found to not yielding a linear curve in competitive inhibition assay for antigen content estimation, though they were suitable for IgG titration assays. The dynamic range of the beads was so low that they could generate mostly 4-6 standard concentrations in linear range out of total eight standard points that were needed for developed bead based competitive inhibition assay (BBCIA). As assay includes antibodies from SSI rabbit sera, many times beads found to be not suitable due to the large extent of cross reacting antibodies in the polyclonal sera. As the BBCIA is a multiplexed assay (10-Plex), each serotype specific antibodies are being used in a mixture. The variable cross reacting patterns of the SSI sera made it difficult to optimize each serotype specific antibody dilution. This has forced to bring in place a new coupling method which can enhance polysaccharide coupling thereby increasing the chances of easy optimization of antibody dilution and having a linear curve over eight different standard concentrations of BBCIA.

The newly developed coupling method was constructed in a unique way that it utilizes magnetic ELISA washer for bead washing steps instead of centrifuge when using magnetic beads for coupling of polysaccharides as well as protein analytes.

Protocol for Modified Amine Coupling:
1. For 1X scale coupling reaction, the bead pellet from properly mixed, monodispersed 100 µl (approximately 1.25 × 10⁶) beads resuspended in bead activation buffer. The beads are activated with 10 µl of freshly made 50 mg/ml EDC and S-NHS each (in bead activation buffer of 100mM MES buffer of pH 6) for 20-30 min at room temperature with 30 RPM shaking on rotospin in dark. Beads washed twice with phosphate buffered saline (PBS) and bead pellet resuspended in water.
2. The polysaccharide to be coupled is activated by mixing with freshly prepared 200 mg/ml of EDC and the mixture added on to activated beads. The bead-polysaccharide-EDC mixture incubated for 2-3 hours in dark at room temperature at 30 RPM on rotospin for coupling and then washed thrice with PBS by centrifugation at 16500 x g for 5 min.
3. To mask any open active group (that may cause non-specific binding during assay) the beads were blocked with 250 mM of glucose and glycine each for 30-45 min. at room temperature with 30 RPM on rotospin in dark. Beads are again washed with PBS and stored in storage buffer equivalent to initial bead volume.
4. A different approach of using polystyrene plate and magnetic washer was followed while using magnetic beads for coupling with below changes.
   - Beads were taken in round bottom 96-well plate. Instead of centrifugation, magnetic ELISA washer was used for washing with 3 cycles (300 µl buffer/well; PBS before coupling reaction & PBS supplemented with 0.2% BSA, 0.1% tween 20 and 0.05% sodium azide after coupling reaction) with 1 min interval in between two wash cycles to let the bead settle down/hold up by magnet at the bottom.
   - Bead activation, coupling and blocking of bead Incubations were done on orbital shaker.

### Advantages of Modified Amine Coupling:

The type 6B, 9V and 19A SIIPL polysaccharide beads coupled by previously available methods had lower and inconsistent efficiency, limiting the assay sensitivity as compared to other serotypes with higher sensitivity. This leads to difficulty in optimizing curve with similar standard range for all polysaccharides of PCV10 and to avoid this, polysaccharides procured from ATCC were used for coupling for BBCIA.

The advantage of modified amine coupling method is that it can produce high efficiency SIIPL polysaccharide coupled beads for above mentioned serotypes which indirectly helped to increase the sensitivity of the assay due to increased number of standard points in linear range. These beads also facilitated easy optimization of antibody dilutions (bead qualification) which was a prerequisite for using these beads in BBCIA for antigen content estimation.

The modified amine coupling method used for these serotypes in three different coupling lots were found to be qualified successfully indicating consistency in bead coupling and their qualification for BBCIA.

In addition to above advantages, the modified amine coupling method has several other advantages over the previous methods as mentioned below.
- The need of lower amount of polysaccharide for coupling (mostly up to 25 µg)
- The method is less laborious and less time consuming
- Reduced time / number of experiments for qualification of beads and optimization of antibody dilution
- When using polystyrene tubes for coupling reaction, the beads use to bind to the inner walls of the tube. These bound beads use to get discarded while collecting supernatant for the discard resulting in low bead count. To reduce the bead loss. the assay was developed in a way that polystyrene 96-well plate used for coupling of polysaccharides with magnetic beads and magnetic ELISA washer for bead washing steps.

The type 6B, 9V and 19A polysaccharide beads coupled by modified amine coupling method were used in combination with beads of other polysaccharides coupled by one of the previous methods and found to be suitable for BBCIA. Three different bead lots for all three polysaccharides were qualified for BBCIA and the recovery of antigen content in final lot was found to be within set specification of 70-130% of labelled content.

These polysaccharides were also coupled at 60 times higher scale of coupling with similar results as for three lots of small-scale coupling and the coupling scale can be further increased.

### (b) Comparative Mouse Immunogenicity Data for Applicant's Developmental PCV21 formulation Ex-121; Ex-119; Ex-131 and commercially available comparators (Prevenar13 and Synflorix)

**Study Objective:** The objective of this study was to evaluate potential of SIIPL's developmental PCV21 formulation Ex-121, Ex-119, Ex-131 to generate a strong immune response and to compare immunogenicity with commercial comparators (Prevenar13 and Synflorix). The total IgG titers were evaluated by multiplexed bead-based assay (Luminex 200).

**Study Details:** SIIPL's PCV-21 developmental formulations Ex-121, Ex-119 and Ex-131 along with comparators (Prevnar 13 and Synflorix) were injected subcutaneously in mice (Harlan mice) at dilution of 1/100^{th}. Mice were injected at Day 0, Day 14, and Day 28 and were bled at Day 42 for endpoint titter estimation. The sera were separated from blood collected on day 42 (Terminal bleeding) to check for total IgG titers by bead-based multiplex assay.

**Method used for IgG titer estimation:** The total IgG titers were determined using multiplex bead-based assay using Luminex 200 platform. The titers were calculated by determining the highest dilution of sera sample having MFI ≥ 100 (cutoff value).

The details of the formulations used in this study is described in Table-15.

| **Table-15: Formulation Details** | |
|---|---|
| **Formulation** | **Details** |
| **PCV21 Ex-121** | Each serotype at 2.2 µg/dose, except, type 6B at 4.4µg/dose; All serotypes as CRM197 conjugated; Aluminium phosphate gel at 187.5 µg/dose |
| **PCV21 Ex-119** | Each serotype at 2.2 µg/dose, except, type 6B at 4.4µg/dose; All serotypes as CRM197 conjugates except Type4-DT, Type 6A-TT, 9V-TT, 19A-TT and Type22F-TT; Aluminium phosphate gel at 187.5 µg/dose |
| **PCV21 Ex-131** | Each serotype at 2.2 µg/dose, except, type 6B at 4.4µg/dose; All serotypes as CRM197 conjugates except Type4-DT, Type3, 6A, 9V, 15B, 19A, 22F-TT; Aluminium phosphate gel at 187.5 µg/dose |

The titers of day 42 are outlined in Table-16 below for Applicant's developmental formulation and compared with Prevnar-13 and Synflorix.

| **Table-16: Mouse Immunogenicity Data** | | | | | | | | | | | |
|---|---|---|---|---|---|---|---|---|---|---|---|
| **Serotype Batch No.** | **Dilution Fold** | **1** | **5** | **6A** | **6B** | **7F** | **9V** | **14** | **19A** | **19F** | **23F** |
| **Ex-121** | **1:100** | 6400 | 3200 | 1600 | 3200 | 3200 | 3200 | 25600 | 12800 | 6400 | 3200 |
| **Ex-119** | **1:100** | 12800 | 6400 | 3200 | 12800 | 6400 | 51200 | 51200 | 51200 | 12800 | 6400 |
| **Ex-131** | **1:100** | 25600 | 25600 | 12800 | 25600 | 12800 | 51200 | 102400 | 204800 | 51200 | 12800 |
| **Prevnar 13** | **1:100** | 12800 | 6400 | 3200 | 3200 | 25600 | 3200 | 51200 | 102400 | 12800 | 1600 |
| **Synflorix** | **1:100** | 6400 | 1600 | NA | 1600 | 6400 | 12800 | 51200 | NA | 1600 | 800 |
| NA (Not applicable): Serotypes absent in formulation | | | | | | | | | | | |

| **Table-16 continued: Mouse Immunogenicity Data** | | | | | | | | | | | | |
|---|---|---|---|---|---|---|---|---|---|---|---|---|
| **Serotype Batch No.** | **Dilution Fold** | **2** | **3** | **4** | **8** | **10A** | **11A** | **12F** | **15B** | **18C** | **22F** | **33F** |
| **Ex-121** | **1:100** | 400 | 1600 | 3200 | 1600 | 6400 | 51200 | 3200 | 3200 | 800 | 12800 | 3200 |
| **Ex-119** | **1:100** | 800 | 3200 | 6400 | 3200 | 12800 | 12800 | 6400 | 6400 | 3200 | 51200 | 6400 |
| **Ex-131** | **1:100** | 3200 | 102400 | 25600 | 102400 | 25600 | 102400 | 12800 | 25600 | 6400 | 204800 | 25600 |
| **Prevnar 13** | **1:100** | NA | 6400 | 12800 | NA | | | | | 200 | NA | |
| **Synflorix** | **1:100** | | NA | 102400 | | | | | | 51200 | | |
| NA (Not applicable): Serotypes absent in formulation | | | | | | | | | | | | |

### Overall Inference for the comparison:

Effect of conjugation with TT- Total IgG titers increased significantly for serotype 3, 6A, 9V and 19A wherein TT conjugates are used in Ex-119; Ex-131 as compared to titers obtained for CRM conjugates in Ex-121.

Additionally, cross reactivity for similar serotypes (i.e. 6A-6B and 19A-19F) is also observed with for Ex-131 as compared to Ex-121.

Immunogenicity studies of vaccine compositions devoid of pneumococcal polysaccharide derived from serotype 3 are ongoing and similar results are expected.

An antibody reacts with antigens other than the antigen that stimulated it. This phenomenon is called cross-reaction or cross-reactivity. Cross-reaction occurs due to the shared epitope on an antigen or conformational similarity of epitopes. In the present study it was seen from the data that there was an increase in the immune response (due to change in carrier protein) of 6A and 19A, immune response towards 6B and 19F also increased possibly due to cross-reactivity.

### TECHNICAL ADVANCEMENTS

The vaccine composition and the method of preparing the vaccine composition of the present disclosure described herein above has several technical advantages including, but not limited to, the realization of:
- vaccine compositions that are devoid of aggregation; show long-term stability across wide temperature ranges
- improved immune response for serotypes 6A, 9V, 19A when TT is used as carrier protein as compared to when CRM197 is used as the carrier protein
- optimum molecular weight of sized polysaccharide and conjugate to ensure high immune response
- high polysaccharide recovery and structural integrity maintained by utilizing high pressure homogenization (as against chemical sizing) for polysaccharide fragmentation
- Stable formulation (low free polysaccharide, low free protein content)
- provide protection against the most prevalent pneumococcal serotypes
- use of higher aluminium salt adjuvant allows improved adsorption for the pneumococcal polysaccharides
- use of higher polysorbate 20 avoids formation of aggregates/ floccules
- enhanced immune response for serotype 6B due to cross-reactivity from 6A-TT
- use of CDAP activation of polysaccharides resulting in faster conjugation reaction along with better preservation of the polysaccharide epitope
- use of multiple carrier proteins avoids immune suppression as a result of using single type of carrier protein

The foregoing description of the specific embodiments fully reveals the general nature of the embodiments herein that others can, by applying current knowledge, readily modify and/or adapt for various applications such specific embodiments without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation. Therefore, while the embodiments herein have been described in terms of preferred embodiments, those skilled in the art will recognize that the embodiments herein can be practiced with modification within the spirit and scope of the embodiments as described herein.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

The use of the expression "at least" or "at least one" suggests the use of one or more elements or ingredients or quantities, as the use may be in the embodiment of the disclosure to achieve one or more of the desired object or results.

Any discussion of documents, acts, materials, devices, articles or the like that has been included in this specification is solely for the purpose of providing a context for the disclosure. It is not to be taken as an admission that any or all of these matters form a part of the prior art base or were common general knowledge in the field relevant to the disclosure as it existed anywhere before the priority date of this application.

The numerical values mentioned for the various physical parameters, dimensions or quantities are only approximations and it is envisaged that the values ten percent higher/lower than the numerical values assigned to the parameters, dimensions or quantities fall within the scope of the disclosure, unless there is a statement in the specification specific to the contrary.

While considerable emphasis has been placed herein on the components and component parts of the preferred embodiments, it will be appreciated that many embodiments can be made and that many changes can be made in the preferred embodiments without departing from the principles of the disclosure. These and other changes in the preferred embodiment as well as other embodiments of the disclosure will be apparent to those skilled in the art from the disclosure herein, whereby it is to be distinctly understood that the foregoing descriptive matter is to be interpreted merely as illustrative of the disclosure and not as a limitation.

## Claims

1. A method of preparing a stable and immunogenic multivalent pneumococcal polysaccharide-protein conjugate vaccine composition, the method comprising the following steps:
**i.** individually conjugating pneumococcal polysaccharide derived from *Streptococcus pneumoniae* serotypes selected from the group comprising of 1, 2, 3, 4, 5, 6, 6A, 6B, 6C, 6D, 6E, 6F, 6G, 6H, 7A, 7B, 7C, 7D, 7F, 8, 9A, 9L, 9F, 9N, 9V, 10A, 10B, 10C, 10D, 10F, 11, 11A, 11B, 11C, 11D, 11E, 11F, 12A, 12B, 12F, 13, 13F, 14, 15A, 15B, 15C, 15F, 16, 16A, 16F, 17A, 17F, 18, 18A, 18B, 18C, 18F, 19A, 19B, 19C, 19F, 20, 20A, 20B, 21, 22A, 22F, 23A, 23B, 23F, 24A, 24B, 24F, 25A, 25F, 27, 28A, 28F, 29, 31, 32A, 32F, 33A, 33B, 33C, 33D, 33E, 33F, 34, 35A, 35B, 35C, 35D, 35F, 36, 37, 38, 39, 40, 41A, 41F, 42, 43, 44, 45, 46, 47A, 47F, and 48 to a carrier-protein in a ratio between 1: 0.5 and 1: 1.5, and at a pH in range of 8.0 to 10 to obtain polysaccharide-carrier protein conjugates, wherein the pneumococcal polysaccharides are conjugated to carrier protein using cyanylation reagent, and the vaccine composition comprises at least three distinct carrier proteins;
**ii.** adsorbing a first set of pneumococcal polysaccharide-protein conjugates comprising polysaccharides derived from *Streptococcus pneumoniae* serotypes selected from the group consisting of 4, 6A, 9V, 15B, 22F and 23F obtained in step (i) on an aluminium salt adjuvant in presence of a salt, histidine and succinic acid at a temperature in the range of 18 °C to 30 °C, pH in the range of 5.0 to 7.0, for a time period of 18 hours to 30 hours;
**iii.** adsorbing a second set of pneumococcal polysaccharide-carrier protein conjugates comprising polysaccharides derived from *S. pneumoniae* serotypes selected from the group comprising 1, 2, 3, 4, 5, 6, 6A, 6B, 6C, 6D, 6E, 6F, 6G, 6H, 7A, 7B, 7C, 7D, 7F, 8, 9A, 9L, 9F, 9N, 9V, 10A, 10B, 10C, 10D, 10F, 11, 11A, 11B, 11C, 11D, 11E, 11F, 12A, 12B, 12F, 13, 13F, 14, 15A, 15B, 15C, 15F, 16, 16A, 16F, 17A, 17F, 18, 18A, 18B, 18C, 18F, 19A, 19B, 19C, 19F, 20, 20A, 20B, 21, 22A, 22F, 23A, 23B, 23F, 24A, 24B, 24F, 25A, 25F, 27, 28A, 28F, 29, 31, 32A, 32F, 33A, 33B, 33C, 33D, 33E, 33F, 34, 35A, 35B, 35C, 35D, 35F, 36, 37, 38, 39, 40, 41A, 41F, 42, 43, 44, 45, 46, 47A, 47F, and 48 obtained in step (i) on an aluminium salt adjuvant in presence of a salt, histidine, and succinic acid at a temperature in the range of 18 °C to 30 °C, pH in the range of 5.0 to 7.0, for a time period of 12 hours to 24 hours; and
**iv.** blending the first set obtained in step (ii) with the second set obtained in step (iii) in presence of a surfactant in the range of 80 µg to 150 µg per 0.5 ml dose of the composition with the help of a Rushton turbine flat blade impeller for a time period of 50 hours to 80 hours, at a temperature in the range of 4 °C to 12 °C;
wherein the composition comprises i) total concentration of aluminium salt adjuvant in the range of 20-375 µg of Al³⁺ per 0.5 ml dose of the composition, ii) each polysaccharide-carrier protein has an adsorption percentage of at least 30 %, iii) preserves the desired physicochemical and immunogenic characteristics of the polysaccharide -carrier protein conjugate, and iv) the surfactant in the range of 80 µg to 150 µg per 0.5 ml dose of the composition results in minimal aggregate and particle formation, and improved flow properties of the vaccine composition.

2. The method as claimed in claim 1, wherein the second set of pneumococcal polysaccharide-carrier protein conjugates comprises *S. pneumoniae* polysaccharides derived from serotypes selected from the group consisting of:
a. 1, 2, 3, 5, 6B, 7F, 8, 10A, 11A, 12F, 14, 18C, 19A, 19F and 33F;
b. 1, 2, 5, 6B, 7F, 8, 10A, 11A, 12F, 14, 18C, 19A, 19F and 33F;
c. 1, 2, 3, 5, 6B, 7F, 8, 10A, 11A, 12F, 14, 18C, 19A, 19F, 24F, and 33F; or
d. 1, 2, 5, 6B, 7F, 8, 10A, 11A, 12F, 14, 18C, 19A, 19F, 24F, and 33F.

3. The method as claimed in claim 1, wherein the aluminium salt adjuvant in step (ii) and step (iii) is selected from the group comprising aluminium hydroxide, aluminium phosphate, aluminum hydroxyphosphate, and potassium aluminum sulfate and mixtures thereof; and wherein the total concentration of aluminium salt adjuvant is in the range of 170 µg to 200 µg of Al³⁺ per 0.5 ml dose of the vaccine composition.

4. The method as claimed in claim 1, wherein the at least 3 distinct carrier-proteins are selected from the group consisting of CRM₁₉₇, diphtheria toxoid (DT), tetanus toxoid (TT), *Neisseria meningitidis* outer membrane complex, fragment C of tetanus toxoid, recombinant full-length tetanus toxin with eight individual amino acid mutations (8MTT), pertussis toxoid, protein D of *H. influenzae*, *E. coli* LT, *E. coli* ST, exotoxin A from *Pseudomonas aeruginosa,* outer membrane complex c (OMPC), porins, fHbp, Por A, Por B transferrin binding proteins, pneumolysin, pneumococcal surface protein A (PspA), pneumococcal surface adhesin A (PsaA), diphtheria toxin fragment B (DTFB), PhtA, PhtB, PhtE, pneumococcal PhtD, pneumococcal surface proteins BVH-3 and BVH-11, M. catarrhalis uspA, protective antigen (PA) of Bacillus anthracis and detoxified edema factor (EF) and lethal factor (LF) of Bacillus anthracis, ovalbumin, keyhole limpet hemocyanin (KLH), CSa peptidase group A or group B *Streptococcus,* human serum albumin, bovine serum albumin (BSA), purified protein derivative of tuberculin (PPD), Cholera toxin subunit B, synthetic peptides, heat shock proteins, pertussis proteins, cytokines, lymphokines, hormones, growth factors, artificial proteins comprising multiple human CD4+ T cell epitopes from various pathogen-derived antigens such as N 19, iron-uptake proteins, toxin A or B from *C*. *difficile* and *S*. *agalactiae* proteins and any equivalents thereof conjugated to at least one pneumococcal polysaccharide

5. The method as claimed in claim 1, wherein at least one pneumococcal polysaccharide derived from serotypes selected from the group comprising 3, 6A, 9V, 15B, 19A, and 22F is conjugated to tetanus toxoid as the carrier protein, at least one pneumococcal polysaccharide derived from serotypes 4 and 24F is conjugated to diphtheria toxoid as the carrier protein and at least one pneumococcal polysaccharide derived from serotype selected from the group comprising 1, 2, 3, 4, 5, 6, 6A, 6B, 6C, 6D, 6E, 6F, 6G, 6H, 7A, 7B, 7C,7D,7F, 8, 9A, 9L, 9F, 9N, 9V, 10A, 10B, 10C, 10D, 10F, 11, 11A, 11B, 11C, 11D, 11E, 11F, 12A, 12B, 12F, 13, 13F, 14, 15A, 15B, 15C, 15F, 16, 16A, 16F, 17A, 17F, 18, 18A, 18B, 18C, 18F, 19A, 19B, 19C, 19F, 20, 20A, 20B, 21, 22A, 22F, 23A, 23B, 23F, 24A, 24B, 24F, 25A, 25F, 27, 28A, 28F, 29, 31, 32A, 32F, 33A, 33B, 33C, 33D, 33E, 33F, 34, 35A, 35B, 35C, 35D, 35F, 36, 37, 38, 39, 40, 41A, 41F, 42, 43, 44, 45, 46, 47A, 47F, and 48 is conjugated to CRM197 as the carrier protein.

6. The method as claimed in claim 1, wherein the pneumococcal polysaccharides are conjugated to carrier protein using a cyanylation reaction and the cyanylation agent is selected from the group consisting of 1-cyano-4-dimethylaminopyridinium tetrafluoroborate (CDAP), 1-cyano-4-pyrrolidinopyridinium tetrafluorborate (CPPT), 1-cyano-imidazole (1-CI), 1-cyanobenzotriazole (1-CBT), 2-cyanopyridazine-3(2H)one (2-CPO), functional derivatives, and modifications thereof.

7. The method as claimed in claim 6, wherein the pneumococcal polysaccharides are conjugated to carrier protein using a cyanylation reaction comprising reacting the pneumococcal polysaccharides with 1-cyano-4-dimethylaminopyridinium tetrafluoroborate (CDAP) mixed in a ratio of between 1:0.3 to 1:2 by weight of pneumococcal polysaccharides: CDAP at temperature in range of 22 °C to 25 °C for a time period of 4 minutes to 10 minutes, and contacting the CDAP activated polysaccharide with carrier protein mixed in a ratio of between 1: 0.5 and 1: 1.5 by weight of CDAP activated pneumococcal polysaccharides: carrier protein, reaction carried out at a pH in range of 8.0-10 for a time period of 3 hours to 5 hours followed by quenching with glycine to obtain polysaccharide-carrier protein conjugates.

8. The method as claimed in claim 1, wherein prior to conjugation the pneumococcal polysaccharide or carrier protein is derivatized with a hetero or homo-bifunctional linker selected from the group consisting of hydrazine, carbohydrazide, hydrazine chloride, a dihydrazide, ε-aminohexanoic acid, chlorohexanol dimethyl acetal, D-glucuronolactone, cystamine and p-nitrophenylethyl amine, hexanediamine, ethylenediamine, 1,6-diaminooxyhexane or β-propinamido, nitrophenyl ethylamine, haloalkyl halide, 6-amino caproic acid, and combinations thereof using a carbodiimide, reductive amination or cyanylation reaction.

9. The method as claimed in claim 1, wherein prior to conjugation step the pneumococcal polysaccharides except polysaccharide derived from serotype 6A are subjected to sizing using one or more of thermal treatment, sonic treatment, mechanical means, chemical hydrolysis, endolytic enzyme treatment, physical shear, Gaulin-homogenizer, and sonication; and post sizing the molecular weight of the pneumococcal polysaccharides except polysaccharide derived from serotype 6A is in the range of 50 kDa to 600 kDa (SEC-HPLC), preferably in the range of 100 kDa to 200 kDa (SEC-HPLC); and wherein the molecular weight of the pneumococcal polysaccharide derived from serotype 6A is in the range of 400 kDa to 900 kDa (SEC-HPLC), preferably in the range of 700 kDa to 800 kDa (SEC-HPLC).

10. The method as claimed in claim 1, wherein the molecular weight of the sized polysaccharide derived from serotype 4 is in the range of 130 kDa to 170 kDa (SEC-HPLC), preferably in the range of 130 kDa to 165 kDa (SEC-HPLC); the molecular weight of the sized polysaccharide derived from serotype 24F is in the range of 100 kDa to 200 kDa (SEC-HPLC), preferably in the range of 120 kDa to 170 kDa (SEC-HPLC); the molecular weight of the sized polysaccharide derived from serotype 6B is in the range of 100 kDa to 200 kDa (SEC-HPLC), preferably in the range of 110 kDa to 175 kDa (SEC-HPLC); the molecular weight of the sized polysaccharide derived from serotype 9V is in the range of 120 kDa to 160 kDa (SEC-HPLC), preferably in the range of 130 kDa to 150 kDa (SEC-HPLC); the molecular weight of the sized polysaccharide derived from serotype 15B is in the range of 120 kDa to 160 kDa (SEC-HPLC), preferably in the range of 130 kDa to 150 kDa (SEC-HPLC); the molecular weight of the sized polysaccharide derived from serotype 19A is in the range of 120 kDa to 160 kDa (SEC-HPLC), preferably in the range of 130 kDa to 150 kDa (SEC-HPLC); the molecular weight of the sized polysaccharide derived from serotype 22F is in the range of 100 kDa to 150 kDa (SEC-HPLC), preferably in the range of 120 kDa to 140 kDa (SEC-HPLC) or combination thereof.

11. The method as claimed in claim 1, wherein
the salt in step (ii) and step (iii) is selected from the group comprising magnesium chloride, potassium chloride, sodium chloride and combinations thereof, and the concentration of the salt is in the range of 2 mg to 10 mg per 0.5 ml dose of the vaccine composition;
histidine in step (ii) and step (iii) is in the range of 0.1 mg to 10 mg per 0.5 ml dose of the vaccine composition; succinic acid in step (ii) and step (iii) is in the range of 0.1 mg to 10 mg per 0.5 ml dose of the vaccine composition; and
the surfactant in step (iv) is selected from the group comprising polysorbate, polymer glycol, sorbitan ester, and combinations thereof; and the concentration of surfactant is in the range of 80 µg to 120 µg per 0.5 ml dose of the vaccine composition.

12. The method as claimed in claim 1, wherein the surfactant in step (iv) is polysorbate 20 and the concentration of polysorbate 20 is in the range of 90 µg to 120 µg per 0.5 ml dose of the vaccine composition.

13. The method as claimed in claim 1, wherein the adsorption in step (ii) and step (iii) is carried out in presence of a preservative selected from the group comprising 2-phenoxyethanol, benzethonium chloride (Phemerol), phenol, m-cresol, thiomersal, formaldehyde, methyl paraben, propyl paraben, benzalkonium chloride, benzyl alcohol, chlorobutanol, p-chlor-m-cresol, benzyl alcohol, and combinations thereof and the concentration of preservative is in the range of 1 mg to 8 mg per 0.5 ml dose of the vaccine composition.

14. The method as claimed in claim 13, wherein the preservative is 2-phenoxyethanol having concentration in the range of 2.5 mg to 5 mg per 0.5 ml dose of the vaccine composition.

15. A stable and immunogenic multivalent pneumococcal polysaccharide-protein conjugate vaccine composition prepared by a method as claimed in any one of the preceding claims having polysaccharide derived from *Streptococcus pneumoniae* serotypes selected from the group consisting of 1, 2, 3, 4, 5, 6, 6A, 6B, 6C, 6D, 6E, 6F, 6G, 6H, 7A, 7B, 7C, 7D, 7F, 8, 9A, 9L, 9F, 9N, 9V, 10A, 10B, 10C, 10D, 10F, 11, 11A, 11B, 11C, 11D, 11E, 11F, 12A, 12B, 12F, 13, 13F, 14, 15A, 15B, 15C, 15F, 16, 16A, 16F, 17A, 17F, 18, 18A, 18B, 18C, 18F, 19A, 19B, 19C, 19F, 20, 20A, 20B, 21, 22A, 22F, 23A, 23B, 23F, 24A, 24B, 24F, 25A, 25F, 27, 28A, 28F, 29, 31, 32A, 32F, 33A, 33B, 33C, 33D, 33E, 33F, 34, 35A, 35B, 35C, 35D, 35F, 36, 37, 38, 39, 40, 41A, 41F, 42, 43, 44, 45, 46, 47A, 47F, and 48; wherein the vaccine composition comprises at least 3 distinct carrier proteins selected from the group consisting of CRM₁₉₇, diphtheria toxoid (DT), tetanus toxoid (TT), *Neisseria meningitidis* outer membrane complex, fragment C of tetanus toxoid, recombinant full-length tetanus toxin with eight individual amino acid mutations (8MTT), pertussis toxoid, protein D of *H. influenzae*, *E. coli* LT, *E. coli* ST, exotoxin A from *Pseudomonas aeruginosa,* outer membrane complex c (OMPC), porins, fHbp, Por A, Por B transferrin binding proteins, pneumolysin, pneumococcal surface protein A (PspA), pneumococcal surface adhesin A (PsaA), diphtheria toxin fragment B (DTFB), PhtA, PhtB, PhtE, pneumococcal PhtD, pneumococcal surface proteins BVH-3 and BVH-11, M. catarrhalis uspA, protective antigen (PA) of Bacillus anthracis and detoxified edema factor (EF) and lethal factor (LF) of Bacillus anthracis, ovalbumin, keyhole limpet hemocyanin (KLH),C5a peptidase group A or group B *Streptococcus*, human serum albumin, bovine serum albumin (BSA), purified protein derivative of tuberculin (PPD), Cholera toxin subunit B, synthetic peptides, heat shock proteins, pertussis proteins, cytokines, lymphokines, hormones, growth factors, artificial proteins comprising multiple human CD4+ T cell epitopes from various pathogen-derived antigens such as N 19, iron-uptake proteins, toxin A or B from *C. difficile* and *S*. *agalactiae* proteins and any equivalents thereof,
wherein each carrier protein is conjugated to at least one pneumococcal polysaccharide; and optionally wherein each dose of the composition comprises 1.0 µg to 10 µg of polysaccharide of each pneumococcal serotype.

16. The vaccine composition as claimed in claim 15, wherein the composition is a 15, 16, 17, 18, 19, 20 21, 22, 23, 24, 25, 26, 27, 28, 29, or 30-valent pneumococcal polysaccharide-carrier protein conjugate composition.

17. The vaccine composition as claimed in claim 15, wherein the vaccine composition comprises twenty distinct *Streptococcus pneumoniae* polysaccharide-carrier protein conjugates having polysaccharide derived from serotypes 1, 2, 4, 5, 6A, 6B, 7F, 8, 9V, 10A, 11A, 12F, 14, 15B, 18C, 19A, 19F 22F, 23F and 33F.

18. The vaccine composition as claimed in claim 15, wherein the vaccine composition comprises twenty one distinct *Streptococcus pneumoniae* polysaccharide-carrier protein conjugates having polysaccharide derived from serotypes 1, 2, 4, 5, 6A, 6B, 7F, 8, 9V, 10A, 11A, 12F, 14, 15B, 18C, 19A, 19F 22F, 23F 24F, and 33F.

19. The vaccine composition as claimed in claim 15, wherein the vaccine composition comprises 3 distinct carrier proteins selected from CRM₁₉₇, diphtheria toxoid (DT) and tetanus toxoid (TT), each conjugated to at least one pneumococcal polysaccharide;

20. The vaccine composition as claimed in claim 19, wherein the pneumococcal polysaccharide conjugated to CRM197 is selected from the group comprising 1, 2, 3, 4, 5, 6, 6A, 6B, 6C, 6D, 6E, 6F, 6G, 6H, 7A, 7B, 7C,7D,7F, 8, 9A, 9L, 9F, 9N, 9V, 10A, 10B, 10C, 10D, 10F, 11, 11A, 11B, 11C, 11D, 11E, 11F, 12A, 12B, 12F, 13, 13F, 14, 15A, 15B, 15C, 15F, 16, 16A, 16F, 17A, 17F, 18, 18A, 18B, 18C, 18F, 19A, 19B, 19C, 19F, 20, 20A, 20B, 21, 22A, 22F, 23A, 23B, 23F, 24A, 24B, 24F, 25A, 25F, 27, 28A, 28F, 29, 31, 32A, 32F, 33A, 33B, 33C, 33D, 33E, 33F, 34, 35A, 35B, 35C, 35D, 35F, 36, 37, 38, 39, 40, 41A, 41F, 42, 43, 44, 45, 46, 47A, 47F, and 48;
the pneumococcal polysaccharide conjugated to TT is selected from the group comprising 1, 3, 5, 6A, 9V, 15B, 19A, and 22F; and the pneumococcal polysaccharide conjugated to DT is selected from the group comprising 3, 4, 15B, and 24F.

21. The vaccine composition as claimed in claim 19, wherein at least one pneumococcal polysaccharide derived from serotypes selected from the group comprising 3, 6A, 9V, 15B, 19A, and 22F is conjugated to tetanus toxoid as the carrier protein, at least one pneumococcal polysaccharide derived from serotypes 4 and 24F is conjugated to diphtheria toxoid as the carrier protein and at least one pneumococcal polysaccharide derived from serotype selected from the group comprising 1, 2, 3, 4, 5, 6, 6A, 6B, 6C, 6D, 6E, 6F, 6G, 6H, 7A, 7B, 7C,7D,7F, 8, 9A, 9L, 9F, 9N, 9V, 10A, 10B, 10C, 10D, 10F, 11, 11A, 11B, 11C, 11D, 11E, 11F, 12A, 12B, 12F, 13, 13F, 14, 15A, 15B, 15C, 15F, 16, 16A, 16F, 17A, 17F, 18, 18A, 18B, 18C, 18F, 19A, 19B, 19C, 19F, 20, 20A, 20B, 21, 22A, 22F, 23A, 23B, 23F, 24A, 24B, 24F, 25A, 25F, 27, 28A, 28F, 29, 31, 32A, 32F, 33A, 33B, 33C, 33D, 33E, 33F, 34, 35A, 35B, 35C, 35D, 35F, 36, 37, 38, 39, 40, 41A, 41F, 42, 43, 44, 45, 46, 47A, 47F, and 48 is conjugated to CRM197 as the carrier protein.

22. The vaccine composition as claimed in any one of the preceding claims 15 to 21, wherein the composition is a 20 valent composition and is formulated as a 0.5 ml dose comprising:
a. 20 distinct *S*. *pneumoniae* polysaccharides derived from serotypes 1, 2, 4, 5, 6A, 6B, 7F, 8, 9V, 10A, 11A, 12F, 14, 15B, 18C, 19A, 19F, 22F, 23F, and 33F;
b. aluminium phosphate as adjuvant containing 170 µg to 200 µg of Al³⁺;
c. histidine at a concentration of 1 mg to 2 mg;
d. succinate at a concentration of 1 mg to 2 mg;
e. polysorbate 20 at a concentration of 80 µg to 120 µg;
f. sodium chloride at a concentration of 2 mg to 10 mg; and
g. pH in the range of 5 to 7;
wherein
i. *S*. *pneumoniae* polysaccharide derived from serotype 4 is conjugated to DT as carrier protein; *S. pneumoniae* polysaccharides derived from serotypes 15B, 6A, 9V, 19A, and 22F are conjugated to TT as carrier protein; and *S*. *pneumoniae* polysaccharides derived from serotypes 1, 2, 5, 6B, 7F, 8, 10A, 11A, 12F, 14, 18C, 19F, 23F and 33F are conjugated to CRM197 as the carrier protein;
ii. 2.2 µg to 3.3 µg of each *S. pneumoniae* polysaccharide except for serotype 6B;
iii. 4.4 µg to 6.6 µg *S*. *pneumoniae* polysaccharide from serotype 6B;
iv. 10 µg to 200 µg of CRM197
v. 5 µg to 70 µg of tetanus toxoid; and
vi. 1 µg to 25 µg of diphtheria toxoid.

23. The vaccine composition as claimed in any one of the preceding claims 15 to 21, wherein the composition is a 21 valent composition and is formulated as a 0.5 ml dose comprising:
a. 21 distinct *S*. *pneumoniae* polysaccharides derived from serotypes 1, 2, 4, 5, 6A, 6B, 7F, 8, 9V, 10A, 11A, 12F, 14, 15B, 18C, 19A, 19F, 22F, 23F, 24F and 33F;
b. aluminium phosphate as adjuvant containing 170 µg to 200 µg of Al³⁺;
c. histidine at a concentration of 1 mg to 2 mg;
d. succinate at a concentration of 1 mg to 2mg;
e. polysorbate 20 at a concentration of 80 µg to 120 µg;
f. sodium chloride at a concentration of 2 mg to 10 mg; and
g. pH in the range of 5 to 7;
wherein
i. *S*. *pneumoniae* polysaccharide derived from serotypes 4, and 24F are conjugated to DT as carrier protein; *S. pneumoniae* polysaccharides derived from serotypes 15B, 6A, 9V, 19A, and 22F are conjugated to TT as carrier protein; and *S. pneumoniae* polysaccharides derived from serotypes 1, 2, 5, 6B, 7F, 8, 10A, 11A, 12F, 14, 18C, 19F, 23F and 33F are conjugated to CRM197 as the carrier protein;
ii. 2.2µg to 3.3 µg of each *S. pneumoniae* polysaccharide except for serotype 6B;
iii. 4.4 µg to 6.6 µg *S*. *pneumoniae* polysaccharide from serotype 6B;
iv. 10 µg to 200 µg of CRM197
v. 5 µg to 70 µg of tetanus toxoid; and
vi. 1 µg to 25 µg of diphtheria toxoid.

24. The vaccine composition as claimed in any one of the claims 15 to 23, wherein *S. pneumoniae* polysaccharide derived from serotype 15B is conjugated to a carrier protein selected from DT and CRM197.

25. The vaccine composition as claimed in any one of the claims 15 to 23, wherein the vaccine composition comprises at least one additional *S. pneumoniae* polysaccharide derived from serotypes selected from 9A, 9N, and 9L.

26. The vaccine composition as claimed in any one of the preceding claims, wherein each pneumococcal polysaccharide-carrier protein conjugate has a molecular weight in the range of 1500 kDa to 30000 kDa (SEC-MALS).

27. The vaccine composition as claimed in claim 26, wherein
the molecular weight of 6A-TT conjugate in the vaccine composition is in the range of 7000 kDa to 10000 kDa (SEC-MALS), preferably in the range of 8000 kDa to 9000 kDa (SEC-MALS);
the molecular weight of 4-DT conjugate in the vaccine composition is in the range of 3000 kDa to 10000 kDa (SEC-MALS), preferably in the range of 3000 kDa to 9000 kDa (SEC-MALS);
the molecular weight of 24F-DT conjugate in the vaccine composition is in the range of 1500 kDa to 10000 kDa (SEC-MALS), preferably in the range of 2000 kDa to 6000 kDa (SEC-MALS);
the molecular weight of 6B-CRM197 conjugate in the vaccine composition is in the range of 4000 kDa to 15000 kDa (SEC-MALS), preferably in the range of 5000 kDa to 12000 kDa (SEC-MALS);
the molecular weight of 9V-TT conjugate in the vaccine composition is in the range of 15000 kDa to 30000 kDa (SEC-MALS), preferably in the range of 20000 kDa to 30000 kDa (SEC-MALS);
the molecular weight of 15B-TT conjugate in the vaccine composition is in the range of 6000 kDa to 10000 kDa (SEC-MALS), preferably in the range of 7500 kDa to 9000 kDa (SEC-MALS);
the molecular weight of 19A-TT conjugate in the vaccine composition is in the range of 5000 kDa to 8000 kDa (SEC-MALS), preferably in the range of 6000 kDa to 7000 kDa (SEC-MALS);
the molecular weight of 22F-TT conjugate in the vaccine composition is in the range of 9000 kDa to 18000 kDa (SEC-MALS), preferably in the range of 10000 kDa to 17000 kDa (SEC-MALS) or a combination thereof.

28. The vaccine composition as claimed in any one of the preceding claims, wherein the composition is a multi-dose composition comprising 2-phenoxyethanol in the range of 2.5 mg to 5 mg per 0.5 ml dose of the vaccine composition.

29. The vaccine composition as claimed in any one of the preceding claims, wherein the composition is stable at 2-8 °C, 25 °C and 40 °C; and optionally the pneumococcal polysaccharide-carrier protein conjugates have free polysaccharide less than 15 %, and free protein less than 10 %.

30. The vaccine composition as claimed in any one of the preceding claims, wherein the pneumococcal polysaccharides derived from serotypes 6A, 9V and 19A show enhanced IgG titers when conjugated to tetanus toxoid (TT) as carrier protein as compared to when pneumococcal polysaccharides derived from serotypes 6A, 9V and 19A are conjugated to CRM197 as the carrier protein; and optionally wherein there is at least 2-fold increase in total IgG titer and functional IgG titer when serotypes 6A, 9V, 19A, and 15B are conjugated to TT as carrier protein as compared to when serotypes 6A, 9V, 19A, and 15B are conjugated to CRM197 as carrier protein.
